# EUROPEAN PATENT APPLICATION

(11) **EP 4 183 804 A1**
(43) Date of publication of application: **24.05.2023**
(21) Application number: 22176926.8
(22) Date of filing: 08.03.2019
(51) Int. Cl.: C07K 16/28, G01N 33/564, A61P 17/06, A61K 39/00

(54) **USE OF IL-36R ANTIBODIES FOR TREATMENT OF INFLAMMATORY BOWEL DISEASE**

(30) Priority: 14.03.2018 US 201862642641 P; 11.09.2018 US 201862729511 P; 10.10.2018 US 201862743778 P
(62) Divisional of application: 19714899.2
(71) Applicant: Boehringer Ingelheim International GmbH, 55216 Ingelheim am Rhein (DE)
(72) Inventor: BOECHER, Wulf Otto, 55216 INGELHEIM AM RHEIN (DE); LAMAR, Janine, 55216 INGELHEIM AM RHEIN (DE); PADULA, Steven John, 55216 INGELHEIM AM RHEIN (DE)
(74) Representative: Simon, Elke Anna Maria

(57) **Abstract**

The present invention relates to the treatment of inflammatory bowel disease (IBD) with anti-IL36R antibodies.

## Description

### RELATED APPLICATIONS

This application claims priority to U.S. Provisional Patent Application Nos. 62/642,641 (filed March 14, 2018), 62/729,511 (filed September 11, 2018) and 62/743,778 (filed October 10, 2018), the entire content of each of which is hereby incorporated herein by reference as though fully set forth herein.

### SEQUENCE LISTING

The instant application contains a Sequence Listing which has been submitted in ASCII format via EFS-Web and is hereby incorporated by reference in its entirety. Said ASCII copy, created on March 5, 2019, is named 09-0681-US-4-2019-03-08-SL.txt and is 146,055 bytes in size.

### TECHNICAL FIELD OF THE INVENTION

The present invention relates to the treatment of Inflammatory Bowel Disease with anti-IL36R antibodies. The present invention further relates to the treatment of moderate to severe inflammatory bowel disease (IBD, including ulcerative colitis and Crohn's disease) with anti-IL36R antibodies. The present invention further relates to the treatment of moderate to severe active inflammatory bowel disease (IBD, including ulcerative colitis and Crohn's disease) with anti-IL36R antibodies in patients who have failed a previous therapy.

### BACKGROUND

Inflammatory bowel disease (IBD) is an umbrella term used to describe disorders that involve chronic inflammation of the digestive tract. Types of IBD include ulcerative colitis and Crohn's disease. Ulcerative Colitis (UC), for example, is a chronic inflammatory bowel disease (IBD) characterized by the key symptoms of chronic diarrhea, bloody stools and abdominal pain. It has an estimated incidence of 24.3 and 19.2 cases per 100,000 persons per year in Europe and the USA, respectively, resulting in a continuously rising prevalence. UC is characterized clinically by abdominal pain, fever, and blood or mucosa-containing diarrhea, and pathologically by inflammatory lesions in the gastrointestinal mucosa. Inflammatory lesions characteristically occur distal to the terminal ileum, and by confinement of lesions to the mucosa and submucosa without transmural inflammation. UC typically follows a relapsing and remitting course, and is associated with substantial acute and long-term morbidity and increased mortality. The mainstays of drug therapy for UC are: orally administered aminosalicylates, glucocorticoids, oral immunosuppressive agents azathioprine and 6-mercaptopurine, and TNF antagonists. In patients with mild UC, 5-ASAs are safe and effective for induction and maintenance treatment. Glucocorticoids, immunosuppressives, TNF antagonists, and more recently vedolizumab, are reserved for patients with moderate to severe disease, in whom the primary goals of drug therapy are to induce and subsequently to maintain remission from signs and symptoms of active disease.

Biologic treatment of moderate/severe UC is associated with approximately one third of patients each failing with primary or secondary non-response. In addition, treatment may be limited due to safety and tolerability issues. Therefore, despite of therapeutic progress, there remains a significant unmet medical need for new treatment options with an improved safety and efficacy profile compared to the current therapeutic standard.

### SUMMARY OF THE INVENTION

The present invention addresses the above need by providing biotherapeutics, in particular antibodies, which bind to IL-36R as a first- second-, third- or subsequent-line therapy for treating inflammatory bowel disease including ulcerative colitis and/or Crohn's disease.

In a first aspect, the present invention relates to a method of treating inflammatory bowel disease in a mammal, said method including administering to the mammal a therapeutically effective amount of an anti-IL-36R antibody.

In a second aspect, the present invention relates to a method of treating a mild to severe inflammatory bowel disease in a mammal, said method including administering to the mammal a therapeutically effective amount of an anti-IL-36R antibody.

In a third aspect, the present invention relates to a method of treating ulcerative colitis in a mammal, said method including administering to the mammal a therapeutically effective amount of an anti-IL-36R antibody.

In a fourth aspect, the present invention relates to a method of treating Crohn's disease in a mammal, said method including administering to the mammal a therapeutically effective amount of an anti-IL-36R antibody. In another related aspect, the present invention relates to a method of treating fistulizing Crohn's disease in a mammal, said method including administering to the mammal a therapeutically effective amount of an anti-IL-36R antibody.

In a fifth aspect, the present invention relates to a method of treating inflammatory bowel disease in a mammal, said method including administering to the mammal a therapeutically effective amount of an anti-IL-36R antibody, wherein the anti-IL-36R antibody is administered in one or more induction dose and/or optionally one or more maintenance dose.

In a sixth aspect, the present invention relates to a method of treating moderate to severe active inflammatory bowel disease, e.g., ulcerative colitis or Crohn's Disease, in an adult patient who has failed a previous therapy, said method including administering to the patient a therapeutically effective amount of an anti-IL-36R antibody.

In a seventh aspect, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12 and has failed a previous therapy, said method including administering to said each patient a therapeutically effective amount of an anti-IL-36R antibody.

In an eighth aspect, the present invention relates to a method for reducing or inhibiting symptoms of moderate to severe active inflammatory bowel disease, e.g., ulcerative colitis or Crohn's Disease, in adult patients, wherein each of said patients has a total MCS (Mayo Clinical Score) of 6 to 12 and has failed a previous therapy, including administering to said each patient a therapeutically effective amount of an anti-IL-36R antibody.

In an embodiment relating to aspects sixth-eighth, each of said patients has a total MCS (Mayo Clinical Score) of 6 to 12 and mESS (modified Endoscopic Subscore) ≥ 2 and has failed a previous therapy.

In an embodiment relating to any of the above aspects, the inflammatory bowel disease is ulcerative colitis or Crohn's disease.

In an embodiment relating to aspects sixth-eighth, the previous therapy includes a biologics therapy or a small-molecule therapy or both. In a related embodiment, the biologics therapy includes a treatment with a TNF antagonist. In a related embodiment, the biologics therapy includes one or more of infliximab, golimumab, adalimumab or vedolizumab. In another related embodiment, the small-molecule therapy includes a treatment with tofacitinib.

In an embodiment relating to any of the above aspects, the anti-IL-36R is administered by a route of administration comprising intravenous, subcutaneous, intraperitoneal or intranasal.

In an embodiment relating to aspects sixth-eighth, at least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In an embodiment relating to aspects sixth-eighth, at least 10%, 20%, 30%, 40%, 50%, 60%, 70% or 80% of the patients achieve mucosal healing (defined as mESS≤1) at week 12 of the treatment.

In an embodiment relating to aspects sixth-eighth, at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

In an embodiment relating to any of the above aspects, the anti-IL-36R antibody includes: a) a light chain variable region comprising the amino acid sequence of SEQ ID NO: 26 (L-CDR1); the amino acid sequence of SEQ ID NO: 35, 102, 103, 104, 105 106 or 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

In an embodiment relating to any of the above aspects, the anti-IL-36R antibody includes:
I. a) a light chain variable region comprising the amino acid sequence of SEQ ID NO: 26 (L-CDR1); the amino acid sequence of SEQ ID NO: 102 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
II. a) a light chain variable region comprising the amino acid sequence of SEQ ID NO: 26 (L-CDR1); the amino acid sequence of SEQ ID NO: 103 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
III. a) a light chain variable region comprising the amino acid sequence of SEQ ID NO: 26 (L-CDR1); the amino acid sequence of SEQ ID NO: 104 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
IV. a) a light chain variable region comprising the amino acid sequence of SEQ ID NO: 26 (L-CDR1); the amino acid sequence of SEQ ID NO: 105 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
V. a) a light chain variable region comprising the amino acid sequence of SEQ ID NO: 26 (L-CDR1); the amino acid sequence of SEQ ID NO: 106 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
VI. a) a light chain variable region comprising the amino acid sequence of SEQ ID NO: 26 (L-CDR1); the amino acid sequence of SEQ ID NO: 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

In an embodiment relating to any of the above aspects, the anti-IL-36R antibody includes:
(i) a light chain variable region comprising the amino acid sequence of SEQ ID NO: 77; and a heavy chain variable region comprising the amino acid sequence of SEQ ID NO: 87;or
(ii) a light chain variable region comprising the amino acid sequence of SEQ ID NO: 77; and a heavy chain variable region comprising the amino acid sequence of SEQ ID NO: 88; or
(iii) a light chain variable region comprising the amino acid sequence of SEQ ID NO: 77; and a heavy chain variable region comprising the amino acid sequence of SEQ ID NO: 89; or
(iv) a light chain variable region comprising the amino acid sequence of SEQ ID NO: 80; and a heavy chain variable region comprising the amino acid sequence of SEQ ID NO: 87;or
(v) a light chain variable region comprising the amino acid sequence of SEQ ID NO: 80; and a heavy chain variable region comprising the amino acid sequence of SEQ ID NO: 88; or
(vi) a light chain variable region comprising the amino acid sequence of SEQ ID NO: 80; and a heavy chain variable region comprising the amino acid sequence of SEQ ID NO: 89; or
(vii) a light chain variable region comprising the amino acid sequence of SEQ ID NO: 85; and a heavy chain variable region comprising the amino acid sequence of SEQ ID NO: 100; or
(viii) a light chain variable region comprising the amino acid sequence of SEQ ID NO: 85; and a heavy chain variable region comprising the amino acid sequence of SEQ ID NO:101; or
(ix) a light chain variable region comprising the amino acid sequence of SEQ ID NO: 86; and a heavy chain variable region comprising the amino acid sequence of SEQ ID NO: 100; or
(x) a light chain variable region comprising the amino acid sequence of SEQ ID NO: 86; and a heavy chain variable region comprising the amino acid sequence of SEQ ID NO:101.

In an embodiment relating to any of the above aspects, the anti-IL-36R antibody includes:
i. a light chain comprising the amino acid sequence of SEQ ID NO: 115; and a heavy chain comprising the amino acid sequence of SEQ ID NO: 125; or
ii. a light chain comprising the amino acid sequence of SEQ ID NO: 115; and a heavy chain comprising the amino acid sequence of SEQ ID NO: 126; or
iii. a light chain comprising the amino acid sequence of SEQ ID NO: 115; and a heavy chain comprising the amino acid sequence of SEQ ID NO: 127; or
iv. a light chain comprising the amino acid sequence of SEQ ID NO: 118; and a heavy chain comprising the amino acid sequence of SEQ ID NO: 125; or
v. a light chain comprising the amino acid sequence of SEQ ID NO: 118; and a heavy chain comprising the amino acid sequence of SEQ ID NO: 126; or
vi. a light chain comprising the amino acid sequence of SEQ ID NO: 118; and a heavy chain comprising the amino acid sequence of SEQ ID NO: 127; or
vii. a light chain comprising the amino acid sequence of SEQ ID NO: 123; and a heavy chain comprising the amino acid sequence of SEQ ID NO: 138; or
viii. a light chain comprising the amino acid sequence of SEQ ID NO: 123; and a heavy chain comprising the amino acid sequence of SEQ ID NO: 139; or
ix. a light chain comprising the amino acid sequence of SEQ ID NO: 124; and a heavy chain comprising the amino acid sequence of SEQ ID NO: 138.

In an embodiment relating to any of the above aspects, the anti-IL-36R antibody is administered in one or more induction dose and/or optionally one or more maintenance dose. In a related embodiment, the induction dose includes 150 mg, 225 mg, 300 mg, 450 mg, 600 mg, 750, 900 mg or 1,200 mg of said anti-IL-36R antibody. In another related embodiment, 1, 2 or 3 induction dose(s) is/are administered. In another related embodiment, 2 or 3 induction doses are administered at 4 weeks intervals. In another related embodiment, the maintenance dose includes 150 mg, 225 mg or 300 mg of said anti-IL-36R antibody. In another related embodiment, 1, 2 or 3 maintenance dose(s) is/are administered to the patient; wherein a first maintenance dose is administered after the last induction dose. In another related embodiment, the first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered. In another related embodiment, the induction dose is administered intravenously and the maintenance dose is administered intravenously and/or subcutaneously.

In an embodiment relating to any of the above aspects, the method comprising (a) administering to the mammal or the patient a dose of an anti-IL-36R antibody at week 0, at week 4 and at week 8 by intravenous injection or infusion, wherein the doses of the anti-IL-36R antibody comprise 150 mg, 200 mg, 225 mg, 300 mg, 450 mg, 600 mg, 750 mg, 900 mg, or 1,200 mg of the antibody. In one embodiment, the method further comprises (b) administering to the mammal or the patient three doses of the anti-IL-36R antibody, for example at week 14, at week 18 and at week 22 by intravenous injection or infusion, wherein the doses of the anti-IL-36R antibody comprise 600 mg of the antibody. In one embodiment, the method further comprises (c) administering to the mammal or the patient one or more doses of the anti-IL-36R antibody at 8 weeks intervals by subcutaneous injections, for example four doses of the anti-IL-36R antibody at 8 weeks intervals by subcutaneous injections, for example at week 26, at week 34, at week 42 and at week 50, wherein the one or more doses of the anti-IL-36R antibody comprise 150 mg of the antibody.

In an embodiment relating to any of the above aspects, the anti-IL-36R antibody or an antigen binding fragment thereof (disclosed herein) is present in a stable pharmaceutical formulation (as described in co-pending U.S. provisional application No. 62/815,405, filed March 8, 2019, the entire content of which is hereby incorporated herein by reference in its entirety) for administration to a mammal or patient according to any one of the aspects of the present invention.

In one embodiment, the method of treatment according to any of the aspects described herein, includes administering to the mammal or patient a therapeutic amount of a stable pharmaceutical formulation comprising from about 20 mg/mL to about 150 mg/mL of an anti-IL-36R antibody (disclosed herein), about 20 mM to about 80 mM of a pharmaceutically acceptable buffer (e.g., acetate buffer), about 100 mM to about 250 mM of a pharmaceutically acceptable tonicifying agent (e.g., sucrose), about 0 mM to about 80 mM of a pharmaceutically acceptable stabilizing agent (e.g., arginine) or a pharmaceutically acceptable salt thereof, about 0 to about 150 mM of a pharmaceutically acceptable salt (e.g., sodium chloride), and a pharmaceutically acceptable surfactant (e.g., polysorbate 20) in an amount about 0 g/L to about 1.5 g/L, wherein the inflammatory bowel disease in the mammal is treated, or the mild to severe inflammatory bowel disease in the mammal is treated, or the ulcerative colitis in the mammal is treated, or the Crohn's disease in the mammal is treated, or the fistulizing Crohn's disease in the mammal is treated, or the moderate to severe active inflammatory bowel disease in the patient who has failed a previous therapy is treated, or the moderate to severe active inflammatory bowel disease in the patient who has failed a previous therapy is treated, or the signs and symptoms of moderate to severe active inflammatory bowel disease is reduced or inhibited. In a related embodiment, the stable pharmaceutical formulation is an aqueous pharmaceutical formulation. In a related embodiment, the pH of the aqueous pharmaceutical formulation is about 5 to about 7. In a related embodiment, the pharmaceutical formulation for an induction dose is administered to the mammal or patient by intravenous administration. In a related embodiment, the pharmaceutical formulation for a maintenance dose is administered to the mammal or patient by intravenous or subcutaneous administration. In a related embodiment, the pharmaceutical formulation for an induction dose comprises an anti-IL-36R antibody in an amount of about 60 mg/mL. In a related embodiment, the pharmaceutical formulation for a maintenance dose comprises an anti-IL-36R antibody in an amount of about 150 mg/mL.

In an embodiment relating to any of the above aspects, at week 12, the mammal or the patient is evaluated for deep remission, for example defined as reaching clinical remission (CDAI score <150) and endoscopic remission (CDEIS ≤ 4). In one embodiment, for a patient with initial isolated ileitis endoscopic remission is defined by CDEIS ≤ 2.

In an embodiment relating to any of the above aspects, the mammal or the patient maintains clinical remission after the administration of the one or more maintenance doses. In one embodiment, the mammal or the patient maintains a CDAI score of less than 150 after the administration of the one or more maintenance doses. In one embodiment, the mammal or the patient maintains a PRO-2 score equal to or less than 75 after the administration of the one or more maintenance doses. In one embodiment, the mammal or the patient maintains a CDAI score of less than 150 and a PRO-2 score equal to or less than 75 after the administration of the one or more maintenance doses.

In an embodiment relating to any of the above aspects, at week 12, a mammal or a patient is evaluated for Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

In an embodiment relating to any of the above aspects, the mammal or the patient maintains clinical remission after the administration of the one or more maintenance doses. In a related embodiment, the clinical remission is maintained up to 4, 6, 8, 10, 12, 14, 16, 18, or 20 weeks following the administration of the last maintenance dose. In one embodiment, the mammal or the patient maintains an improved Clinical Remission (i.e., mMCS SFS=0 or 1, if drop ≥1 from BL; and RBS=0; and mESS≤1) after the administration of the one or more maintenance doses. In one embodiment, the mammal or the patient maintains an improved Mucosal Healing (i.e., mESS ≤ 1) after the administration of the one or more maintenance doses. In one embodiment, the mammal or the patient maintains maintains an improved Clinical Response (i.e., total MCS reduction ≥3 pts. and ≥30% from BL; AND RBS drop from baseline by ≥1pt., or absolute RBS ≤1 pt) after the administration of the one or more maintenance doses. In one embodiment, the mammal or the patient maintains an improved IBDQ score after the administration of the one or more maintenance doses. In one embodiment, the mammal or the patient maintains an improved combined Mucosal healing and histologic remission (i.e., mESS ≤ 1 and Robarts Histology Index ≤ 6) after the administration of the one or more maintenance doses. In a related embodiment, the improved effects are compared to placebo. In a related embodiment, the improved effects are maintained at higher percentage with an anti-IL-36R antibody of the present invention than with placebo.

In an embodiment relating to any of the above aspects, the percentage of mammals or patients receiving an anti-IL-36R antibody of the present invention shows improved symptoms after 12 weeks of treatment compared with the percentage of mammals or patients on placebo. In a related embodiment, the improved symptoms include improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6). In a related embodiment, the improved symptoms are maintained up to 40, 44, 48 or 52 weeks after the administration of the one or more maintenance doses. In a related embodiment, the improved symptoms are maintained at higher percentage with an anti-IL-36R antibody of the present invention than with placebo.

In an embodiment relating to any of the above aspects, the improved effects are maintained at higher percentage with an anti-IL-36R antibody of the present invention than with placebo. In an embodiment relating to any of the above aspects, the improved effects are maintained for 4 to 12 weeks following the administration of the last maintenance dose.

In an embodiment relating to any of the above aspects, a higher percentage of mammals or patients receiving an anti-IL-36R antibody of the present invention show improved symptoms after 12 weeks of treatment compared with a lesser percentage of mammals or patients on placebo. In a related embodiment, the improved symptoms include improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 20%, 30%, 40%, 50%, 60%, 70% or 80% of the patients achieve mucosal healing (defined as mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

### DETAILED DESCRIPTION OF THE INVENTION

This invention therefore relates to the treatment of inflammatory bowel disease (IBD, including ulcerative colitis and Crohn's disease) with anti-IL36R antibodies. The present invention further relates to the treatment of moderate to severe inflammatory bowel disease (IBD, including ulcerative colitis and Crohn's disease) with anti-IL36R antibodies. The present invention further relates to the treatment of moderate to severe active inflammatory bowel disease (IBD, including ulcerative colitis and Crohn's disease) with anti-IL36R antibodies in patients who have failed a previous therapy.

Without wishing to be bound by this theory it is believed that anti-IL-36R antibodies or antigen-binding fragments thereof bind to human IL-36R and thus interfere with the binding of IL-36 agonists, and in doing so block at least partially the signaling cascade from the IL-36R to inflammatory mediators. The anti-IL36R antibodies of the present invention are disclosed in U.S. Patent No. 9,023,995 or WO2013/074569, the entire content of each of which is incorporated herein by reference.

IL-36R is also known as IL-1 RL2 and IL-1 Rrp2. It has been reported that agonistic IL-36 ligands (α, β, or γ) initiate the signaling cascade by engaging the IL-36 receptor which then forms a heterodimer with the IL-1 receptor accessory protein (IL-1 RAcP). IL-36 antagonist ligands (IL-36RA/IL1 F5, IL-38/ILF10) inhibit the signaling cascade.

### Definitions

The term "about" shall generally mean an acceptable degree of error or variation for the quantity measured given the nature or precision of the measurements. Typical, exemplary degrees of error or variation are within 5% or within 3% or within 1% of a given value or range of values. For example, the expression of "about 100" includes 105 and 95 or 103 and 97 or 101 and 99, and all values in between (e.g., 95.1, 95.2, etc. for range of 95-105; or 97.1, 97.2, etc. for the range of 97-103; 99.1, 99.2, etc. for the range of 99-101). Numerical quantities given herein are approximates unless stated otherwise, meaning that the term "about" can be inferred when not expressly stated.

A "pharmaceutical formulation" or "formulation" refers to the process but also the product of a process in which an active drug or agent is combined with chemical substances to produce a final medicinal or drug product, the final formulation therefore refers to medicinal products such as liquids, powders or compositions. Therefore, in one embodiment, a pharmaceutical formulation is a pharmaceutical composition.

A "pharmaceutical composition" refers in this context to a liquid or powder preparation which is in such form as to permit the biological activity of the active ingredient(s) to be unequivocally effective, and which contains no additional components which are significantly toxic to the subjects to which the composition would be administered. Such compositions are sterile. A "powder" refers to a freeze-dried or lyophilized or a spray-dried pharmaceutical composition for parenteral use. The powder is reconstituted or dissolved typically in water. Lyophilisation is a low temperature dehydration process which involves freezing the product, lowering pressure, then removing the ice by sublimation. Freeze drying results in a high quality product because of the low temperature used in processing. For a well-developed lyophilized formulation, the shape and appearance of the product is maintained over time and the quality of the rehydrated product is excellent. Spray drying is another method of producing a dry powder from a liquid or slurry by rapidly drying with a hot gas and with the goal of achieving a consistent particle size distribution.

As used herein, the terms "induction dose", "maintenance dose" refer to the temporal sequence of administration of the anti-IL-36R antibody. Thus, the "induction dose" is the dose which is administered at the beginning of the treatment regimen (also referred to as the "baseline dose"); it may also be referred to as an "initial dose." The "maintenance dose" is the dose which is administered after the induction dose, which may also be referred to as a "subsequent dose." The term "induction dose" also refers to an initial treatment dose that is administered intravenously. Thus, the term "induction dose" may also be referred to as an "intravenous dose." The induction, maintenance doses may all contain the same amount of anti-IL-36R antibody or an antigen binding fragment thereof, but generally may differ from one another in terms of the amount of the antibody administered, mode of administration or the frequency of administration. In an embodiment, the induction dose is equal or larger than the maintenance dose. An "induction dose" which may be interchangeably referred to as an "initial dose" or "intravenous dose" can be a single dose or, alternatively, a set of doses. The maintenance dose may also be referred to as a "subsequent dose" or simply "subcutaneous dose" if it is administered subcutaneously following the induction dose. The maintenance dose can be a single dose or, alternatively, a set of doses administered subcutaneously or intravenously to the patient or mammal.

In certain embodiments, the amount of the anti-IL-36R antibody contained in the induction/initial/intravenous and maintenance/subsequent/subcutaneous doses varies from one another during the course of treatment. In certain embodiments, the one or more initial/induction/intravenous doses each comprise a first amount of the antibody or antigen-binding fragment thereof and the one or more maintenance/subsequent/subcutaneous doses each comprise a second amount of the antibody or antigen-binding fragment thereof. In some embodiments, the first amount of antibody or fragment thereof is 1.5x, 2x, 2.5x, 3x, 3.5x, 4x, or 5x the second or subsequent amount of the antibody or antigen-binding fragment thereof. In certain embodiments, one or more (e.g., 1, 2, 3, 4, or 5 or more) initial doses are administered at the beginning of the treatment regimen as "loading doses" or "leading doses" followed by subsequent doses that are administered on a less frequent basis (e.g., "maintenance doses"). In one embodiment, the induction dose, the initial dose or the intravenous dose is 150 mg, 225 mg, 300 mg, 450 mg, 600 mg, 750, 900 mg or 1,200 mg of the anti-IL-36R antibody. In one embodiment, the maintenance dose, the subsequent or the subcutaneous dose is about 150 mg, 180 mg, 225 mg, 270 mg or 300 mg. In another embodiment, the maintenance or subsequent dose is administered at least four weeks following the induction or initial dose.

As used herein "buffer" refers to a buffered solution that resists changes in pH by the action of its acid-base conjugate components. The "pH" herein refers to the acidity or basicity of the composition at room temperature. Standard methods to measure the pH of a composition are known to the skilled in the art. Typically, measuring pH consists of calibrating the instrument, placing the electrodes in a well-mixed sample, and then reading the pH directly from the pH meter. The exemplary buffers of the present invention include acetate, citrate, histidine, succinate, phosphate and Tris.

As used herein, the term "tonicifying agent" or "tonicity agent" or "tonicifyer" refers to substances providing an osmotic pressure equivalent to that of serum in the body including salts (e.g. sodium chloride, potassium chloride, magnesium chloride) or sugars (e.g. sucrose, trehalose, sorbitol, magnesium sulfate (MgSO₄), glycerol, mannitol or dextrose). In addition, sugars present in the solution act as a cryoprotectant for the protein which allows the drug substance to be frozen without damage. This permits shipment in the frozen form and long-term storage of the drug substance prior to the filling of drug product. The exemplary tonicifying agents of the present invention include sodium chloride, potassium chloride, magnesium chloride (salts) and/or sucrose, trehalose, sorbitol, magnesium sulfate (MgSO₄), glycerol, mannitol or dextrose (sugars).

As used herein, the term "stabilizer" or "stabilizing agent" refers to substances contributing to the stability of the active ingredient in a pharmaceutical formulation. The exemplary stabilizing agents of the present invention include arginine, histidine, glycine, cysteine, proline, methionine, lysine, or pharmaceutically acceptable salts thereof.

As used herein, the term "surfactant" refers to substances which tend to reduce the surface tension of a liquid in which they are dissolved. The exemplary surfactants of the present invention include poloxamer 188, polysorbate 20, polysorbate 40, polysorbate 60 or polysorbate 80.

In a first aspect, the present invention relates to a method of treating inflammatory bowel disease in a mammal, said method including administering to the mammal a therapeutically effective amount of an anti-IL-36R antibody.

In a second aspect, the present invention relates to a method of treating a mild to severe inflammatory bowel disease in a mammal, said method including administering to the mammal a therapeutically effective amount of an anti-IL-36R antibody.

In a third aspect, the present invention relates to a method of treating ulcerative colitis in a mammal, said method including administering to the mammal a therapeutically effective amount of an anti-IL-36R antibody.

In a fourth aspect, the present invention relates to a method of treating Crohn's disease in a mammal, said method including administering to the mammal a therapeutically effective amount of an anti-IL-36R antibody.

In a fifth aspect, the present invention relates to a method of treating inflammatory bowel disease in a mammal, said method including administering to the mammal a therapeutically effective amount of an anti-IL-36R antibody, wherein the anti-IL-36R antibody is administered in one or more induction dose and/or optionally one or more maintenance dose.

In a sixth aspect, the present invention relates to a method of treating moderate to severe active inflammatory bowel disease, e.g., ulcerative colitis or Crohn's Disease, in an adult patient who has failed a previous therapy, said method including administering to the patient a therapeutically effective amount of an anti-IL-36R antibody.

In a seventh aspect, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12 and has failed a previous therapy, said method including administering to said each patient a therapeutically effective amount of an anti-IL-36R antibody.

In an eighth aspect, the present invention relates to a method for reducing or inhibiting symptoms of moderate to severe active inflammatory bowel disease, e.g., ulcerative colitis or Crohn's Disease, in adult patients, wherein each of said patients has a total MCS (Mayo Clinical Score) of 6 to 12 and has failed a previous therapy, comprising administering to said each patient a therapeutically effective amount of an anti-IL-36R antibody.

In an embodiment relating to aspects sixth-eighth, each of said patients has a total MCS (Mayo Clinical Score) of 6 to 12 and mESS (modified Endoscopic Subscore) ≥ 2 and has failed a previous therapy.

In an embodiment relating to any of the above aspects, the inflammatory bowel disease is ulcerative colitis or Crohn's disease.

In an embodiment relating to aspects sixth-eighth, the previous therapy includes a biologics therapy or a small-molecule therapy or both. In a related embodiment, the biologics therapy includes a treatment with a TNF antagonist. In a related embodiment, the biologics therapy includes a treatment with one or more of infliximab, golimumab, adalimumab or vedolizumab. In another related embodiment, the small-molecule therapy comprises a treatment with tofacitinib.

In an embodiment relating to any of the above aspects, the anti-IL-36R is administered by a route of administration comprising intravenous, subcutaneous, intraperitoneal or intranasal.

In an embodiment relating to aspects sixth-eighth, at least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In an embodiment relating to aspects sixth-eighth, at least 10%, 20%, 30%, 40%, 50%, 60%, 70% or 80% of the patients achieve mucosal healing (defined as mESS≤1) at week 12 of the treatment.

In an embodiment relating to aspects sixth-eighth, at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

In an embodiment relating to any of the above aspects, the anti-IL-36R antibody is administered in one or more induction dose and/or optionally one or more maintenance dose. In a related embodiment, the induction dose includes 150 mg, 225 mg, 300 mg, 450 mg, 600 mg, 750, 900 mg or 1,200 mg of said anti-IL-36R antibody. In another related embodiment, 1, 2 or 3 induction dose(s) is/are administered. In another related embodiment, 2 or 3 induction doses are administered at 4 weeks intervals. In another related embodiment, the maintenance dose includes 150 mg, 225 mg or 300 mg of said anti-IL-36R antibody. In another related embodiment, 1, 2 or 3 maintenance dose(s) is/are administered to the patient; wherein a first maintenance dose is administered after the last induction dose. In another related embodiment, the first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered. In another related embodiment, the induction dose is administered intravenously and the maintenance dose is administered intravenously and/or subcutaneously.

In an embodiment relating to any of the above aspects, the method comprising (a) administering to the mammal or the patient a dose of an anti-IL-36R antibody at week 0, at week 4 and at week 8 by intravenous infusion, wherein the doses of the anti-IL-36R antibody comprise 150 mg, 200 mg, 225 mg, 300 mg, 450 mg, 600 mg, 750 mg, 900 mg, or 1,200 mg of the antibody. In one embodiment, the method further comprises (b) administering to the mammal or the patient three doses of the anti-IL-36R antibody, for example at week 14, at week 18 and at week 22 by intravenous infusion, wherein the doses of the anti-IL-36R antibody comprise 600 mg of the antibody. In one embodiment, the method further comprises (c) administering to the mammal or the patient one or more doses of the anti-IL-36R antibody at 8 weeks intervals by subcutaneous injection, for example four doses of the anti-IL-36R antibody at 8 weeks intervals by subcutaneous injection, for example at week 26, at week 34, at week 42 and at week 50, wherein the one or more doses of the anti-IL-36R antibody comprise 150 mg of the antibody.

In an embodiment relating to any of the above aspects, at week 12, a mammal or a patient is evaluated for deep remission, for example defined as reaching clinical remission (CDAI score <150) and endoscopic remission (CDEIS ≤ 4). In one embodiment, for a patient with initial isolated ileitis endoscopic remission is defined by CDEIS ≤ 2.

In an embodiment relating to any of the above aspects, the mammal or the patient maintains clinical remission after the administration of the one or more maintenance doses. In one embodiment, the mammal or the patient maintains a CDAI score of less than 150 after the administration of the one or more maintenance doses. In one embodiment, the mammal or the patient maintains a PRO-2 score equal to or less than 75 after the administration of the one or more maintenance doses. In one embodiment, the mammal or the patient maintains a CDAI score of less than 150 and a PRO-2 score equal to or less than 75 after the administration of the one or more maintenance doses.

In an embodiment relating to any of the above aspects, at week 12, a mammal or a patient is evaluated for improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

In an embodiment relating to any of the above aspects, the mammal or the patient maintains clinical remission after the administration of the one or more maintenance doses. In one embodiment, the mammal or the patient maintains an improved Clinical Remission (i.e., mMCS SFS=0 or 1, if drop ≥1 from BL; and RBS=0; and mESS≤1) after the administration of the one or more maintenance doses. In one embodiment, the mammal or the patient maintains an improved Mucosal Healing (i.e., mESS ≤ 1) after the administration of the one or more maintenance doses. In one embodiment, the mammal or the patient maintains maintains an improved Clinical Response (i.e., total MCS reduction ≥3 pts. and ≥30% from BL; AND RBS drop from baseline by ≥1pt., or absolute RBS ≤1 pt) after the administration of the one or more maintenance doses. In one embodiment, the mammal or the patient maintains an improved IBDQ score after the administration of the one or more maintenance doses. In one embodiment, the mammal or the patient maintains an improved combined Mucosal healing and histologic remission (i.e., mESS ≤ 1 and Robarts Histology Index ≤ 6) after the administration of the one or more maintenance doses. In a related embodiment, the improved effects are compared to placebo. In a related embodiment, the improved effects are maintained at higher percentage with an anti-IL-36R antibody of the present invention than with placebo.

In an embodiment relating to any of the above aspects, the percentage of mammals or patients receiving an anti-IL-36R antibody of the present invention shows improved symptoms after 12 weeks of treatment compared with the percentage of mammals or patients on placebo. In a related embodiment, the improved symptoms comparise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6). In a related embodiment, the improved symptoms are maintained up to 40, 44, 48 or 52 weeks after the administration of the one or more maintenance doses. In a related embodiment, the improved symptoms are maintained at higher percentage with an anti-IL-36R antibody of the present invention than with placebo.

In an embodiment relating to any of the above aspects, the improved effects are maintained at higher percentage with an anti-IL-36R antibody of the present invention than with placebo.

In an embodiment relating to any of the above aspects, the improved effects (including the remission or improved symptoms) last for 4, 5, 6, 7, 8, 9, 10, 11, 12, 13, 14, 15, 16, 17, 18, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks following the administration of the last maintenance dose of BI655130.

In an embodiment relating to any of the above aspects, a higher percentage of mammals or patients receiving an anti-IL-36R antibody of the present invention show improved symptoms after 12 weeks of treatment compared with a lesser percentage of mammals or patients on placebo. In a related embodiment, the improved symptoms comprise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 20%, 30%, 40%, 50%, 60%, 70% or 80% of the patients achieve mucosal healing (defined as mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

In an embodiment relating to any of the above aspects, the method comprising (a) administering to the patient a dose of an anti-IL-36R antibody at week 0, at week 4 and at week 8 by intravenous infusion, wherein the doses of the anti-IL-36R antibody comprise 150 mg, 200 mg, 225 mg, 300 mg, 450 mg, 600 mg, 750 mg, 900 mg, or 1,200 mg of the antibody. In one embodiment, the method further comprises (b) administering to the patient three doses of the anti-IL-36R antibody, for example at week 14, at week 18 and at week 22 by intravenous infusion, wherein the doses of the anti-IL-36R antibody comprise 600 mg of the antibody. In one embodiment, the method further comprises (c) administering to the patient one or more doses of the anti-IL-36R antibody at 8 weeks intervals by subcutaneous injection, for example four doses of the anti-IL-36R antibody at 8 weeks intervals by subcutaneous injection, for example at week 26, at week 34, at week 42 and at week 50, wherein the one or more doses of the anti-IL-36R antibody comprise 150 mg of the antibody.

In an embodiment relating to any of the above aspects, at week 12, a patient is evaluated for deep remission, for example defined as reaching clinical remission (CDAI score <150) and endoscopic remission (CDEIS ≤ 4). In one embodiment, for a patient with initial isolated ileitis endoscopic remission is defined by CDEIS ≤ 2.

In an embodiment relating to any of the above aspects, the method comprising (a) administering to a patient a dose of an anti-IL-36R antibody at week 0, at week 4 and at week 8 by intravenous infusion, wherein the doses of the anti-IL-36R antibody comprise 225 mg or 600 mg of the antibody. In one embodiment, the method further comprises (b) administering to the patient one or more doses of the anti-IL-36R antibody at 8 weeks intervals by subcutaneous injection, for example four doses of the anti-IL-36R antibody at 8 weeks intervals by subcutaneous injection, for example at week 26, at week 34, at week 42 and at week 50, wherein the one or more doses of the anti-IL-36R antibody comprise 150 mg of the antibody.

In an embodiment relating to any of the above aspects, the method comprising administering to a patient 150 mg of the anti-IL-36R antibody at 8 weeks intervals by subcutaneous injection.

In an embodiment relating to any of the above aspects, the method comprising administering at least one induction dose of the anti-IL-36R antibody to the patient, wherein said induction dose comprises 225 to 1,200 mg of the anti-IL-36R antibody, for example 300 to 1,200 mg of the anti-IL-36R antibody. In another embodiment, the induction dose comprises 225 mg, 300 mg, 450 mg, 600 mg, 750 mg, 900 mg or 1,200 mg of the anti-IL-36R antibody. In one embodiment, 1, 2 or 3 induction doses are administered to the patient. In one embodiment, 2 or 3 inductions doses are administered, for example at 4 weeks intervals. In one embodiment, the induction dose(s) is administered by intravenous infusion.

In an embodiment relating to any of the above aspects, the induction dose comprises 225 mg of the anti-IL-36R antibody and three inductions doses are administered to the patient at 4 weeks intervals.

In an embodiment relating to any of the above aspects, the induction dose comprises 300 mg of the anti-IL-36R antibody and three inductions doses are administered to the patient at 4 weeks intervals.

In an embodiment relating to any of the above aspects, the induction dose comprises 450 mg of the anti-IL-36R antibody and three inductions doses are administered to the patient at 4 weeks intervals.

In an embodiment relating to any of the above aspects, the induction dose comprises 600 mg of the anti-IL-36R antibody and three inductions doses are administered to the patient at 4 weeks intervals.

In an embodiment relating to any of the above aspects, the induction dose comprises 750 mg of the anti-IL-36R antibody and three inductions doses are administered to the patient at 4 weeks intervals.

In an embodiment relating to any of the above aspects, the induction dose comprises 900 mg of the anti-IL-36R antibody and three inductions doses are administered to the patient at 4 weeks intervals.

In an embodiment relating to any of the above aspects, the induction dosecomprise 1,200 mg of the anti-IL-36R antibody and three inductions doses are administered to the patient at 4 weeks intervals.

In an embodiment relating to any of the above aspects, at least one additional induction dose of the anti-IL-36R antibody is administered to the patient after the last induction dose above. In one embodiment, an additional induction dose comprises 225 to 1,200 mg of the anti-IL-36R antibody, for example 300 to 1,200 mg of the anti-IL-36R antibody. In one embodiment, an additional induction dose comprises 225 mg, 300 mg, 450 mg, 600 mg, 750 mg, 900 mg or 1,200 mg of the anti-IL-36R antibody. In one embodiment, 1, 2 or 3 additional induction doses are administered to the patient. In one embodiment, 2 or 3 additional inductions doses are administered, for example at 4 weeks intervals. In one embodiment, the additional induction dose(s) is administered by intravenous infusion.

In an embodiment relating to any of the above aspects, the patient has a CDAI score of 220-450 before the administration of the first induction dose.

In an embodiment relating to any of the above aspects, the patient achieves clinical remission after the administration of the one or more induction doses. In one embodiment, the patient achieves a CDAI score of less than 150 after the administration of the one or more induction doses. In one embodiment, the patient achieves a PRO-2 score equal to or less than 75 after the administration of the one or more induction doses. In one embodiment, the patient achieves a CDAI score of less than 150 and a PRO-2 score equal to or less than 75 after the administration of the one or more induction doses.

In an embodiment relating to any of the above aspects, the present invention further provides a method for inducing clinical remission of ulcerative colitis or Crohn's Disease in a patient comprising administering to the patient an anti-IL-36R antibody as described above or herein.

In an embodiment relating to any of the above aspects, the present invention further provides a method for inducing clinical response to ulcerative colitis or Crohn's Disease in a patient comprising administering to the patient an anti-IL-36R antibody as described above or herein.

In an embodiment relating to any of the above aspects, the method further comprises administering a first maintenance dose of the anti-IL-36R antibody to the patient after the last induction dose is administered and administering at least one additional maintenance dose to the patient 4 to 12 weeks after said first maintenance dose is administered. In one embodiment, the first maintenance dose is administered 2 to 8 weeks, for example 4 to 6 weeks, for example 2 weeks, 4 weeks, 6 weeks or 8 weeks, after the last induction dose is administered. In one embodiment, the at least one additional maintenance dose is administered to the patient 4, 6, 8 or 12 weeks after the first maintenance dose is administered.

In an embodiment relating to any of the above aspects, the first maintenance dose comprises 150 to 300 mg of the anti-IL-36R antibody. In one embodiment, the first maintenance dose comprises 150 mg, 225 mg or 300 mg of the anti-IL-36R antibody. In one embodiment, the first maintenance dose comprises 180 mg or 270 mg of the anti-IL-36R antibody.

In an embodiment relating to any of the above aspects, the at least one additional maintenance dose comprises 150 to 300 mg of the anti-IL-36R antibody. In one embodiment, the at least one additional maintenance dose comprises 150 mg, 225 mg or 300 mg of the anti-IL-36R antibody. In one embodiment, the at least one additional maintenance dose comprises 180 mg or 270 mg of the anti-IL-36R antibody.

In one embodiment, the first maintenance dose and the at least one additional maintenance dose comprise 150 to 300 mg of the anti-IL-36R antibody. In one embodiment, the first maintenance dose and the at least one additional maintenance dose comprise 150 mg, 225 mg or 300 mg of said anti-IL-36R antibody. In one embodiment, the first maintenance dose and the at least one additional maintenance dose comprise 180 mg or 270 mg of said anti-IL-36R antibody.

In one embodiment, a maintenance dose is administered by subcutaneous injection.

In an embodiment relating to any of the above aspects, the maintenance doses comprise 150 mg of the anti-IL-36R antibody and are administered to the patient at 4 weeks intervals. In one embodiment, the maintenance doses comprise 150 mg of the anti-IL-36R antibody and are administered to the patient at 8 weeks intervals. In one embodiment, the maintenance doses comprise 225 mg of the anti-IL-36R antibody and are administered to the patient at 8 weeks intervals. In one embodiment, the maintenance doses comprise 225 mg of the anti-IL-36R antibody and are administered to the patient at 12 weeks intervals. In one embodiment, the maintenance doses comprise 300 mg of the anti-IL-36R antibody and are administered to the patient at 8 weeks intervals. In one embodiment, the maintenance doses comprise 300 mg of the anti-IL-36R antibody and are administered to the patient at 12 weeks intervals.

In an embodiment relating to any of the above aspects, the patient maintains clinical remission after the administration of the one or more maintenance doses. In one embodiment, the patient maintains a CDAI score of less than 150 after the administration of the one or more maintenance doses. In one embodiment, the patient maintains a PRO-2 score equal to or less than 75 after the administration of the one or more maintenance doses. In one embodiment, the patient maintains a CDAI score of less than 150 and a PRO-2 score equal to or less than 75 after the administration of the one or more maintenance doses.

In an embodiment relating to any of the above aspects, at least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In an embodiment relating to any of the above aspects, at least 10%, 20%, 30%, 40%, 50%, 60%, 70% or 80% of the patients achieve mucosal healing (defined as mESS≤1) at week 12 of the treatment.

In an embodiment relating to any of the above aspects, at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

Representative examples of doses and dose regimens according to the present invention are disclosed in Table 1.

**Table 1: doses and dose regimens**

| Induction dose (mg) | Frequency of induction doses | Maintenance dose (mg) | Frequency of maintenance doses |
|---|---|---|---|
| 225 | Every 4 weeks | 150 | Every 4 weeks |
| 225 | Every 4 weeks | 150 | Every 8 weeks |
| 225 | Every 4 weeks | 180 | Every 4 weeks |
| 225 | Every 4 weeks | 180 | Every 8 weeks |
| 225 | Every 4 weeks | 225 | Every 8 weeks |
| 225 | Every 4 weeks | 225 | Every 12 weeks |
| 225 | Every 4 weeks | 270 | Every 8 weeks |
| 225 | Every 4 weeks | 270 | Every 12 weeks |
| 225 | Every 4 weeks | 300 | Every 8 weeks |
| 225 | Every 4 weeks | 300 | Every 12 weeks |
| 300 | Every 4 weeks | 150 | Every 4 weeks |
| 300 | Every 4 weeks | 150 | Every 8 weeks |
| 300 | Every 4 weeks | 180 | Every 4 weeks |
| 300 | Every 4 weeks | 180 | Every 8 weeks |
| 300 | Every 4 weeks | 225 | Every 8 weeks |
| 300 | Every 4 weeks | 225 | Every 12 weeks |
| 300 | Every 4 weeks | 270 | Every 8 weeks |
| 300 | Every 4 weeks | 270 | Every 12 weeks |
| 300 | Every 4 weeks | 300 | Every 8 weeks |
| 300 | Every 4 weeks | 300 | Every 12 weeks |
| 450 | Every 4 weeks | 150 | Every 4 weeks |
| 450 | Every 4 weeks | 150 | Every 8 weeks |
| 450 | Every 4 weeks | 180 | Every 4 weeks |
| 450 | Every 4 weeks | 180 | Every 8 weeks |
| 450 | Every 4 weeks | 225 | Every 8 weeks |
| 450 | Every 4 weeks | 225 | Every 12 weeks |
| 450 | Every 4 weeks | 270 | Every 8 weeks |
| 450 | Every 4 weeks | 270 | Every 12 weeks |
| 450 | Every 4 weeks | 300 | Every 8 weeks |
| 450 | Every 4 weeks | 300 | Every 12 weeks |
| 600 | Every 4 weeks | 150 | Every 4 weeks |
| 600 | Every 4 weeks | 150 | Every 8 weeks |
| 600 | Every 4 weeks | 180 | Every 4 weeks |
| 600 | Every 4 weeks | 180 | Every 8 weeks |
| 600 | Every 4 weeks | 225 | Every 8 weeks |
| 600 | Every 4 weeks | 225 | Every 12 weeks |
| 600 | Every 4 weeks | 270 | Every 8 weeks |
| 600 | Every 4 weeks | 270 | Every 12 weeks |
| 600 | Every 4 weeks | 300 | Every 8 weeks |
| 600 | Every 4 weeks | 300 | Every 12 weeks |
| 750 | Every 4 weeks | 150 | Every 4 weeks |
| 750 | Every 4 weeks | 150 | Every 8 weeks |
| 750 | Every 4 weeks | 180 | Every 4 weeks |
| 750 | Every 4 weeks | 180 | Every 8 weeks |
| 750 | Every 4 weeks | 225 | Every 8 weeks |
| 750 | Every 4 weeks | 225 | Every 12 weeks |
| 750 | Every 4 weeks | 270 | Every 8 weeks |
| 750 | Every 4 weeks | 270 | Every 12 weeks |
| 750 | Every 4 weeks | 300 | Every 8 weeks |
| 750 | Every 4 weeks | 300 | Every 12 weeks |
| 900 | Every 4 weeks | 150 | Every 4 weeks |
| 900 | Every 4 weeks | 150 | Every 8 weeks |
| 900 | Every 4 weeks | 180 | Every 4 weeks |
| 900 | Every 4 weeks | 180 | Every 8 weeks |
| 900 | Every 4 weeks | 225 | Every 8 weeks |
| 900 | Every 4 weeks | 225 | Every 12 weeks |
| 900 | Every 4 weeks | 270 | Every 8 weeks |
| 900 | Every 4 weeks | 270 | Every 12 weeks |
| 900 | Every 4 weeks | 300 | Every 8 weeks |
| 900 | Every 4 weeks | 300 | Every 12 weeks |
| 1,200 | Every 4 weeks | 150 | Every 4 weeks |
| 1,200 | Every 4 weeks | 150 | Every 8 weeks |
| 1,200 | Every 4 weeks | 180 | Every 4 weeks |
| 1,200 | Every 4 weeks | 180 | Every 8 weeks |
| 1,200 | Every 4 weeks | 225 | Every 8 weeks |
| 1,200 | Every 4 weeks | 225 | Every 12 weeks |
| 1,200 | Every 4 weeks | 270 | Every 8 weeks |
| 1,200 | Every 4 weeks | 270 | Every 12 weeks |
| 1,200 | Every 4 weeks | 300 | Every 8 weeks |
| 1,200 | Every 4 weeks | 300 | Every 12 weeks |

In one embodiment, 1, 2 or 3 induction dose(s) is/are administered to the patient in a dose regimen described in Table 1.

Additional representative examples of doses and dose regimens according to the present invention are described below. In these examples, the first maintenance dose is administered is administered to the patient 4 weeks after the last induction dose. It is also contemplated in the present invention to administer the first maintenance dose 2 weeks, 6 weeks or 8 weeks after the last induction dose.

For example, in the context of the present invention, inductions doses are administered to the patient at week 0, week 4 and week 8, followed by a first maintenance dose at week 12, a second maintenance dose at week 16, a third maintenance dose at week 20 and so on at dosing intervals of 4 weeks between the maintenance doses. In one embodiment, the inductions doses comprise 225 mg, 300 mg, 450 mg, 600 mg, 750 mg, 900 mg or 1,200 mg of the anti-IL-36R antibody. In one embodiment, the maintenance doses comprise 150 mg of the anti-IL-36R antibody.

For example, in the context of the present invention, inductions doses are administered to the patient at week 0, week 4 and week 8, followed by a first maintenance dose at week 12, a second maintenance dose at week 20, a third maintenance dose at week 28 and so on at dosing intervals of 8 weeks between the maintenance doses. In one embodiment, the inductions doses comprise 200 mg, 450 mg, 600 mg, 900 mg or 1,200 mg of the anti-IL-36R antibody. In one embodiment, the maintenance doses comprise 150 mg, 225 mg or 300 mg of the anti-IL-36R antibody.

For example, in the context of the present invention, inductions doses are administered to the patient at week 0, week 4 and week 8, followed by a first maintenance dose at week 12, a second maintenance dose at week 24, a third maintenance dose at week 36 and so on at dosing intervals of 12 weeks between the maintenance doses. In one embodiment, the inductions doses comprise 225 mg, 300 mg, 450 mg, 600 mg, 750 mg, 900 mg or 1,200 mg of the anti-IL-36R antibody. In one embodiment, the maintenance doses comprise 225 mg or 300 mg of the anti-IL-36R antibody.

In one embodiment, the anti-IL-36R antibody or an antigen binding fragment thereof (disclosed herein) is present in a pharmaceutical formulation (as described in co-pending U.S. provisional application No. 62/815,405, filed March 8, 2019, the entire content of which is hereby incorporated herein by reference in its entirety) suitable for administration to a mammal or patient according to any one of the aspects described herein.

In another embodiment, the formulation comprises a therapeutic amount of an anti-IL-36R antibody (disclosed herein) and
i) a pharmaceutically acceptable buffer; or
ii) a pharmaceutically acceptable tonicifying agent; or
iii) a pharmaceutically acceptable stabilizing agent; or
iv) a pharmaceutically acceptable salt; or
v) a pharmaceutically acceptable surfactant; or
vi) a pharmaceutically acceptable buffer and a pharmaceutically acceptable tonicifying agent; or
vii) a pharmaceutically acceptable buffer, a pharmaceutically acceptable tonicifying agent and a pharmaceutically acceptable stabilizing agent; or
viii) a pharmaceutically acceptable buffer, a pharmaceutically acceptable tonicifying agent, a pharmaceutically acceptable stabilizing agent and a pharmaceutically acceptable salt; or
ix) a pharmaceutically acceptable buffer, a pharmaceutically acceptable tonicifying agent, a pharmaceutically acceptable stabilizing agent, a pharmaceutically acceptable salt and a pharmaceutically acceptable surfactant;
each in pharmaceutically acceptable quantities and at a pharmaceutically acceptable pH.

In another embodiment, the anti-IL-36R antibody or antigen binding fragment thereof is present in the formulation at a concentration of about 15 mg/mL, about 20 mg/mL, about 25 mg/mL, about 30 mg/mL, about 60 mg/mL, about 75 mg/mL, about 80 mg/mL, about 100 mg/mL or about 150 mg/mL. In another related embodiment, the pharmaceutically acceptable buffer is present in the formulation at a concentration within the range from about 20 mM to about 80 mM, or at a concentration of about 20 mM, about 25 mM, about 35 mM, about 40 mM, about 45 mM, about 50 mM, about 60 mM. In another related embodiment, the pharmaceutically acceptable tonicifying agent is present in the formulation at a concentration within the range from about 100 mM to about 250 mM, or at a concentration of about 100 mM, about 120 mM, about 150 mM, about 180 mM, about 200 mM. In another related embodiment, the pharmaceutically acceptable stabilizing agent is present in the formulation at a concentration within the range from about 0 mM to about 80 mM, or at a concentration of about 25 mM or about 50 mM. In another related embodiment, the pharmaceutically acceptable salt is present in the formulation at a concentration of within the range from about 0 to about 150 mM, or at a concentration of about 3 mM, 5 mM, 10 mM, 25 mM or 50 mM. In another related embodiment, the pharmaceutically acceptable surfactant is present in the formulation at a concentration within the range from about 0 g/L to about 1.5 g/L, or at a concentration of about 0.1 g/L, 0.2 g/L, 0.4 g/L, 0.5 g/L or 1 g/L. In an embodiment related to the first aspect, the formulation is characterized by a pH within the range from about 5 to about 8. In another related embodiment, the pH is about 5, about 5.5, about 6, about 6.5, about 7, about 7.5 or about 8.

In another embodiment, the buffer comprises histidine, phosphate, succinate, citrate, acetate or TRIS; the tonicifying agent is one or more sugar and/or polyol including sucrose, trehalose, sorbitol, magnesium sulfate (MgSO4), glycerol, mannitol or dextrose; the stabilizer comprises an amino acid including arginine, histidine, glycine, cysteine, proline, methionine, lysine, aspartate, glutamate or pharmaceutically acceptable salts thereof; the salt comprises sodium chloride (NaCl), magnesium chloride (MgCl2), potassium chloride (KCI), lithium chloride (LiCl), calcium chloride (CaCl2), boric acid salts or zinc chloride (ZnCl2); and the surfactant comprises poloxamer 188, polysorbate 20, polysorbate 40, polysorbate 60 or polysorbate 80.

In one embodiment, the method of treatment according to any of the aspects described herein, includes administering to the mammal or patient a therapeutic amount of a stable pharmaceutical formulation comprising from about 20 mg/mL to about 150 mg/mL of an anti-IL-36R antibody, about 20 mM to about 80 mM of a pharmaceutically acceptable buffer (e.g., acetate buffer), about 100 mM to about 250 mM of a pharmaceutically acceptable tonicifying agent (e.g., sucrose), about 0 mM to about 80 mM of a pharmaceutically acceptable stabilizing agent (e.g., arginine) or a pharmaceutically acceptable salt thereof, about 0 to about 150 mM of a pharmaceutically acceptable salt (e.g., sodium chloride), and a pharmaceutically acceptable surfactant (e.g., polysorbate 20) in an amount about 0 g/L to about 1.5 g/L, wherein the inflammatory bowel disease in the mammal is treated, or the mild to severe inflammatory bowel disease in the mammal is treated, or the ulcerative colitis in the mammal is treated, or the Crohn's disease in the mammal is treated, or the fistulizing Crohn's disease in the mammal is treated, or the moderate to severe active inflammatory bowel disease in the patient who has failed a previous therapy is treated, or the moderate to severe active inflammatory bowel disease in the patient who has failed a previous therapy is treated, or the signs and symptoms of moderate to severe active inflammatory bowel disease is reduced or inhibited. In a related embodiment, the stable pharmaceutical formulation is an aqueous pharmaceutical formulation. In a related embodiment, the pH of the aqueous pharmaceutical formulation is about 5 to about 7. In a related embodiment, the pharmaceutical formulation for an induction dose is administered to the mammal or patient by intravenous administration. In a related embodiment, the pharmaceutical formulation for a maintenance dose is administered to the mammal or patient by intravenous or subcutaneous administration. In a related embodiment, the pharmaceutical formulation for an induction dose comprises an anti-IL-36R antibody in an amount of about 60 mg/mL. In a related embodiment, the pharmaceutical formulation for a maintenance dose comprises an anti-IL-36R antibody in an amount of about 150 mg/mL. In a related embodiment, the anti-IL-36R antibody comprising: (i) a light chain including an amino acid sequence set forth as SEQ ID NO:118 and a heavy chain including an amino acid sequence set forth as SEQ ID NO:125; or (ii) a light chain including an amino acid sequence set forth as SEQ ID NO:118 and a heavy chain including an amino acid sequence set forth as SEQ ID NO:126; or (iii) a light chain including an amino acid sequence set forth as SEQ ID NO:118 and a heavy chain including an amino acid sequence set forth as SEQ ID NO:127. In a related embodiment, the anti-IL-36R antibody comprising: a light chain variable region comprising the amino acid sequence of SEQ ID NO: 77; and a heavy chain variable region comprising the amino acid sequence of SEQ ID NO: 87; or a light chain variable region comprising the amino acid sequence of SEQ ID NO: 77; and a heavy chain variable region comprising the amino acid sequence of SEQ ID NO: 88; or a light chain variable region comprising the amino acid sequence of SEQ ID NO: 77; and a heavy chain variable region comprising the amino acid sequence of SEQ ID NO: 89; or a light chain variable region comprising the amino acid sequence of SEQ ID NO: 80; and a heavy chain variable region comprising the amino acid sequence of SEQ ID NO: 87;or a light chain variable region comprising the amino acid sequence of SEQ ID NO: 80; and a heavy chain variable region comprising the amino acid sequence of SEQ ID NO: 88; or a light chain variable region comprising the amino acid sequence of SEQ ID NO: 80; and a heavy chain variable region comprising the amino acid sequence of SEQ ID NO: 89.

In one embodiment, the method of treatment according to any of the preceding aspects, comprises administering to the mammal or patient a therapeutic amount of a stable pharmaceutical formulation selected from the group consisting of consisting of:
I. formulation including about 20 mg/mL to about 150 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0;
II. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
III. formulation including about 20 mg/mL to about 150 mg/mL of the anti-IL-36R antibody, about 45 mM acetate, about 180 mM sucrose, about 25 mM Glycine, about 0.4 g/L Polysorbate 80, with a pH of about 5.5;
IV. formulation including about 20 mg/mL to about 150 mg/mL of the anti-IL-36R antibody, about 25 mM citrate, about 150 mM trehalose, about 25 mM methionine, about 0.2 g/L Polysorbate 20, with a pH of about 6.0;
V. formulation including about 20 mg/mL to about 150 mg/mL of the anti-IL-36R antibody, about 25 mM histidine, about 180 mM sucrose, about 20 mM mannitol, about 0.2 g/L Polysorbate 20, with a pH of about 6.5;
VI. formulation including about 20 mg/mL to about 150 mg/mL of the anti-IL-36R antibody, about 25 mM citrate, about 200 mM sucrose, about 0.4 g/L Polysorbate 80, with a pH of about 6.5;
VII. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
VIII. formulation including about 20 mg/mL to about 150 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
IX. formulation including about 20 mg/mL to about 150 mg/mL of the anti-IL-36R antibody, about 25 mM acetate, about 100 mM mannitol, about 50 mM NaCl, about 0.2 g/L Polysorbate 20, with a pH of about 5.5;
X. formulation including about 20 mg/mL to about 150 mg/mL of the anti-IL-36R antibody, about 20 mM succinate, about 220 mM sucrose, about 0.1 g/L Polysorbate 80, with a pH of about 6.0; and
XI. formulation including about 20 mg/mL to about 150 mg/mL of the anti-IL-36R antibody, about 25 mM citrate, about 0.4 g/L Polysorbate 20, with a pH of about 6.5,

Wherein the inflammatory bowel disease in the mammal is treated, or the mild to severe inflammatory bowel disease in the mammal is treated, or the ulcerative colitis in the mammal is treated, or the Crohn's disease in the mammal is treated, or the fistulizing Crohn's disease in the mammal is treated, or the moderate too severe active inflammatory bowel disease in the patient who has failed a previous therapy is treated, or the moderate to severe active inflammatory bowel disease in the patient who has failed a previous therapy is treated, or the signs and symptoms of moderate to severe active inflammatory bowel disease is reduced or inhibited. In a related embodiment, the stable pharmaceutical formulation is an aqueous pharmaceutical formulation. In a related embodiment, the pharmaceutical formulation for an induction dose is administered to the mammal or patient by intravenous administration. In a related embodiment, the pharmaceutical formulation for a maintenance dose is administered to the mammal or patient by intravenous or subcutaneous administration. In a related embodiment, the pharmaceutical formulation for an induction dose comprises an anti-IL-36R antibody in an amount of about 60 mg/mL. In a related embodiment, the pharmaceutical formulation for a maintenance dose comprises an anti-IL-36R antibody in an amount of about 150 mg/mL. In a related embodiment, the anti-IL-36R antibody comprising: (i) a light chain including an amino acid sequence set forth as SEQ ID NO:118 and a heavy chain including an amino acid sequence set forth as SEQ ID NO:125; or (ii) a light chain including an amino acid sequence set forth as SEQ ID NO:118 and a heavy chain including an amino acid sequence set forth as SEQ ID NO:126; or (iii) a light chain including an amino acid sequence set forth as SEQ ID NO:118 and a heavy chain including an amino acid sequence set forth as SEQ ID NO:127. In a related embodiment, the anti-IL-36R antibody comprising: a light chain variable region comprising the amino acid sequence of SEQ ID NO: 77; and a heavy chain variable region comprising the amino acid sequence of SEQ ID NO: 87; or a light chain variable region comprising the amino acid sequence of SEQ ID NO: 77; and a heavy chain variable region comprising the amino acid sequence of SEQ ID NO: 88; or a light chain variable region comprising the amino acid sequence of SEQ ID NO: 77; and a heavy chain variable region comprising the amino acid sequence of SEQ ID NO: 89; or a light chain variable region comprising the amino acid sequence of SEQ ID NO: 80; and a heavy chain variable region comprising the amino acid sequence of SEQ ID NO: 87;or a light chain variable region comprising the amino acid sequence of SEQ ID NO: 80; and a heavy chain variable region comprising the amino acid sequence of SEQ ID NO: 88; or a light chain variable region comprising the amino acid sequence of SEQ ID NO: 80; and a heavy chain variable region comprising the amino acid sequence of SEQ ID NO: 89.

In one embodiment, the method of treatment according to any of the preceding aspects, comprises administering to the mammal or patient a therapeutic amount of a stable pharmaceutical formulation selected from the group consisting of consisting of:
I. formulation including about 20 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0;
II. formulation including about 60 mg/mL of the anti-IL-36R antibody, about 45 mM acetate, about 150 mM sucrose, about 25 mM L-Arginine, about 0.4 g/L Polysorbate 20, with a pH of about 5.5;
III. formulation including about 20 mg/mL of the anti-IL-36R antibody, about 45 mM acetate, about 180 mM sucrose, about 25 mM Glycine, about 0.4 g/L Polysorbate 80, with a pH of about 5.5;
IV. formulation including about 150 mg/mL of the anti-IL-36R antibody, about 25 mM citrate, about 150 mM trehalose, about 25 mM methionine, about 0.2 g/L Polysorbate 20, with a pH of about 6.0;
V. formulation including about 150 mg/mL of the anti-IL-36R antibody, about 25 mM histidine, about 180 mM sucrose, about 20 mM mannitol, about 0.2 g/L Polysorbate 20, with a pH of about 6.5;
VI. formulation including about 20 mg/mL of the anti-IL-36R antibody, about 25 mM citrate, about 200 mM sucrose, about 0.4 g/L Polysorbate 80, with a pH of about 6.5;
VII. formulation including about 150 mg/mL of the anti-IL-36R antibody, about 45 mM acetate, about 150 mM sucrose, about 25 mM L-Arginine, about 0.4 g/L Polysorbate 20, with a pH of about 5.5;
VIII. formulation including about 15 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
IX. formulation including about 80 mg/mL of the anti-IL-36R antibody, about 25 mM acetate, about 100 mM mannitol, about 50 mM NaCl, about 0.2 g/L Polysorbate 20, with a pH of about 5.5;
X. formulation including about 100 mg/mL of the anti-IL-36R antibody, about 20 mM succinate, about 220 mM sucrose, about 0.1 g/L Polysorbate 80, with a pH of about 6.0; and
XI. formulation including about 60 mg/mL of the anti-IL-36R antibody, about 25 mM citrate, about 0.4 g/L Polysorbate 20, with a pH of about 6.5,

Wherein the inflammatory bowel disease in the mammal is treated, or the mild to severe inflammatory bowel disease in the mammal is treated, or the ulcerative colitis in the mammal is treated, or the Crohn's disease in the mammal is treated, or the fistulizing Crohn's disease in the mammal is treated, or the moderate too severe active inflammatory bowel disease in the patient who has failed a previous therapy is treated, or the moderate to severe active inflammatory bowel disease in the patient who has failed a previous therapy is treated, or the signs and symptoms of moderate to severe active inflammatory bowel disease is reduced or inhibited. In a related embodiment, the stable pharmaceutical formulation is an aqueous pharmaceutical formulation. In a related embodiment, the pharmaceutical formulation for an induction dose is administered to the mammal or patient by intravenous administration. In a related embodiment, the pharmaceutical formulation for a maintenance dose is administered to the mammal or patient by intravenous or subcutaneous administration. In a related embodiment, the pharmaceutical formulation for an induction dose comprises an anti-IL-36R antibody in an amount of about 60 mg/mL. In a related embodiment, the pharmaceutical formulation for a maintenance dose comprises an anti-IL-36R antibody in an amount of about 150 mg/mL. In a related embodiment, the anti-IL-36R antibody comprising: (i) a light chain including an amino acid sequence set forth as SEQ ID NO:118 and a heavy chain including an amino acid sequence set forth as SEQ ID NO:125; or (ii) a light chain including an amino acid sequence set forth as SEQ ID NO:118 and a heavy chain including an amino acid sequence set forth as SEQ ID NO:126; or (iii) a light chain including an amino acid sequence set forth as SEQ ID NO:118 and a heavy chain including an amino acid sequence set forth as SEQ ID NO:127. In a related embodiment, the anti-IL-36R antibody comprising: a light chain variable region comprising the amino acid sequence of SEQ ID NO: 77; and a heavy chain variable region comprising the amino acid sequence of SEQ ID NO: 87; or a light chain variable region comprising the amino acid sequence of SEQ ID NO: 77; and a heavy chain variable region comprising the amino acid sequence of SEQ ID NO: 88; or a light chain variable region comprising the amino acid sequence of SEQ ID NO: 77; and a heavy chain variable region comprising the amino acid sequence of SEQ ID NO: 89; or a light chain variable region comprising the amino acid sequence of SEQ ID NO: 80; and a heavy chain variable region comprising the amino acid sequence of SEQ ID NO: 87;or a light chain variable region comprising the amino acid sequence of SEQ ID NO: 80; and a heavy chain variable region comprising the amino acid sequence of SEQ ID NO: 88; or a light chain variable region comprising the amino acid sequence of SEQ ID NO: 80; and a heavy chain variable region comprising the amino acid sequence of SEQ ID NO: 89.

In one embodiment, in a method of the present invention, a patient is evaluated for deep remission, for example defined as reaching clinical remission (CDAI score <150) and endoscopic remission (CDEIS ≤ 4). In one embodiment, for a patient with initial isolated ileitis endoscopic remission is defined by CDEIS ≤ 2. In one embodiment, the PRO response of the patient is assessed, for example defined by either a PRO-2 score of <8 or a reduction from baseline of at least 8 points (PRO-2: Patient reported outcome-2).

In an embodiment relating to any of the above aspects, at week 12, a mammal or a patient is evaluated for improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

In an embodiment relating to any of the above aspects, the mammal or the patient maintains clinical remission after the administration of the one or more maintenance doses. In one embodiment, the mammal or the patient maintains an improved Clinical Remission (i.e., mMCS SFS=0 or 1, if drop ≥1 from BL; and RBS=0; and mESS≤1) after the administration of the one or more maintenance doses. In one embodiment, the mammal or the patient maintains an improved Mucosal Healing (i.e., mESS ≤ 1) after the administration of the one or more maintenance doses. In one embodiment, the mammal or the patient maintains an improved Clinical Response (i.e., total MCS reduction ≥3 pts. and ≥30% from BL; AND RBS drop from baseline by ≥1pt., or absolute RBS ≤1 pt) after the administration of the one or more maintenance doses. In one embodiment, the mammal or the patient maintains an improved IBDQ score after the administration of the one or more maintenance doses. In one embodiment, the mammal or the patient maintains an improved combined Mucosal healing and histologic remission (i.e., mESS ≤ 1 and Robarts Histology Index ≤ 6) after the administration of the one or more maintenance doses. In a related embodiment, the improved effects are compared to placebo. In a related embodiment, the improved effects are maintained at higher percentage with an anti-IL-36R antibody of the present invention than with placebo. In a related embodiment, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the mammals or patients maintain improved effects with an anti-IL-36R antibody of the present invention than with placebo.

In an embodiment relating to any of the above aspects, the percentage of mammals or patients receiving an anti-IL-36R antibody of the present invention shows improved symptoms after 12 weeks of treatment compared with the percentage of mammals or patients on placebo. In a related embodiment, the improved symptoms comparise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6). In a related embodiment, the improved symptoms are maintained up to 40, 44, 48 or 52 weeks after the administration of the one or more maintenance doses. In a related embodiment, the improved symptoms are maintained at higher percentage with an anti-IL-36R antibody of the present invention than with placebo. In a related embodiment, the improved effects are maintained at higher percentage with an anti-IL-36R antibody of the present invention than with placebo. In a related embodiment, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the mammals or patients show improved symptoms after 12 weeks of treatment compared with the percentage of mammals or patients on placebo.

In an embodiment relating to any of the above aspects, a higher percentage of mammals or patients receiving an anti-IL-36R antibody of the present invention show improved symptoms after 12 weeks of treatment compared with a lesser percentage of mammals or patients on placebo. In a related embodiment, the improved symptoms comprise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6). In a related embodiment, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the mammals or patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In one embodiment, in a method of the present invention, a patient is evaluated for clinical remission, clinical response, endoscopic remission, endoscopic response or mucosal healing, for example as defined herein.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve mucosal healing (defined as mESS≤1) at week 12 of the treatment.

In one embodiment, the present invention relates to a method of treating moderate to severe active inflammatory bowel disease, e.g., ulcerative colitis or Crohn's Disease, in adult patients, wherein each of said patients has a total MCS (Mayo Clinical Score) of 6 to 12, and/or has an mESS (modified Endoscopic Subscore) ≥ 2, and has failed a previous therapy, said method including administering to said each patient a therapeutically effective amount of an anti-IL-36R antibody; wherein at least at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90%of the patients achieve combined mucosal healing and histologic remission (defined as mESS≤1 and Robarts Histology Index≤6) at week 12 of the treatment.

In an embodiment relating to any of the above aspects, the anti-IL-36R antibody includes: a) a light chain variable region comprising the amino acid sequence of SEQ ID NO: 26 (L-CDR1); the amino acid sequence of SEQ ID NO: 35, 102, 103, 104, 105 106 or 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

In an embodiment relating to any of the above aspects, the anti-IL-36R antibody includes:
I. a) a light chain variable region comprising the amino acid sequence of SEQ ID NO: 26 (L-CDR1); the amino acid sequence of SEQ ID NO: 102 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
II. a) a light chain variable region comprising the amino acid sequence of SEQ ID NO: 26 (L-CDR1); the amino acid sequence of SEQ ID NO: 103 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
III. a) a light chain variable region comprising the amino acid sequence of SEQ ID NO: 26 (L-CDR1); the amino acid sequence of SEQ ID NO: 104 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
IV. a) a light chain variable region comprising the amino acid sequence of SEQ ID NO: 26 (L-CDR1); the amino acid sequence of SEQ ID NO: 105 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
V. a) a light chain variable region comprising the amino acid sequence of SEQ ID NO: 26 (L-CDR1); the amino acid sequence of SEQ ID NO: 106 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
VI. a) a light chain variable region comprising the amino acid sequence of SEQ ID NO: 26 (L-CDR1); the amino acid sequence of SEQ ID NO: 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

In an embodiment relating to any of the above aspects, the anti-IL-36R antibody includes:
(i) a light chain variable region comprising the amino acid sequence of SEQ ID NO: 77; and a heavy chain variable region comprising the amino acid sequence of SEQ ID NO: 87;or
(ii) a light chain variable region comprising the amino acid sequence of SEQ ID NO: 77; and a heavy chain variable region comprising the amino acid sequence of SEQ ID NO: 88; or
(iii) a light chain variable region comprising the amino acid sequence of SEQ ID NO: 77; and a heavy chain variable region comprising the amino acid sequence of SEQ ID NO: 89; or
(iv) a light chain variable region comprising the amino acid sequence of SEQ ID NO: 80; and a heavy chain variable region comprising the amino acid sequence of SEQ ID NO: 87;or
(v) a light chain variable region comprising the amino acid sequence of SEQ ID NO: 80; and a heavy chain variable region comprising the amino acid sequence of SEQ ID NO: 88; or
(vi) a light chain variable region comprising the amino acid sequence of SEQ ID NO: 80; and a heavy chain variable region comprising the amino acid sequence of SEQ ID NO: 89; or
(vii) a light chain variable region comprising the amino acid sequence of SEQ ID NO: 85; and a heavy chain variable region comprising the amino acid sequence of SEQ ID NO: 100; or
(viii) a light chain variable region comprising the amino acid sequence of SEQ ID NO: 85; and a heavy chain variable region comprising the amino acid sequence of SEQ ID NO:101; or
(ix) a light chain variable region comprising the amino acid sequence of SEQ ID NO: 86; and a heavy chain variable region comprising the amino acid sequence of SEQ ID NO: 100; or
(x) a light chain variable region comprising the amino acid sequence of SEQ ID NO: 86; and a heavy chain variable region comprising the amino acid sequence of SEQ ID NO:101.

In an embodiment relating to any of the above aspects, the anti-IL-36R antibody includes:
i. a light chain comprising the amino acid sequence of SEQ ID NO: 115; and a heavy chain comprising the amino acid sequence of SEQ ID NO: 125; or
ii. a light chain comprising the amino acid sequence of SEQ ID NO: 115; and a heavy chain comprising the amino acid sequence of SEQ ID NO: 126; or
iii. a light chain comprising the amino acid sequence of SEQ ID NO: 115; and a heavy chain comprising the amino acid sequence of SEQ ID NO: 127; or
iv. a light chain comprising the amino acid sequence of SEQ ID NO: 118; and a heavy chain comprising the amino acid sequence of SEQ ID NO: 125; or
v. a light chain comprising the amino acid sequence of SEQ ID NO: 118; and a heavy chain comprising the amino acid sequence of SEQ ID NO: 126; or
vi. a light chain comprising the amino acid sequence of SEQ ID NO: 118; and a heavy chain comprising the amino acid sequence of SEQ ID NO: 127; or
vii. a light chain comprising the amino acid sequence of SEQ ID NO: 123; and a heavy chain comprising the amino acid sequence of SEQ ID NO: 138; or
viii. a light chain comprising the amino acid sequence of SEQ ID NO: 123; and a heavy chain comprising the amino acid sequence of SEQ ID NO: 139; or
ix. a light chain comprising the amino acid sequence of SEQ ID NO: 124; and a heavy chain comprising the amino acid sequence of SEQ ID NO: 138.

In an embodiment relating to any of the above aspects, the anti-IL36R antibody is BI 655130. In one embodiment, the anti-IL36R antibody is disclosed in U.S. Patent No. 9,023,995 or WO2013/074569. In an embodiment relating to any of the above aspects, the improved effects (including the remission or improved symptoms) last for 4, 5, 6, 7, 8, 9, 10, 11, 12, 13, 14, 15, 16, 17, 18, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks following the administration of the last maintenance dose of BI655130.

The epitopes are most commonly proteins, short oligopeptides, oligopeptide mimics (i.e., organic compounds that mimic antibody binding properties of the IL-36R antigen), or combinations thereof. The minimum size of a peptide or polypeptide epitope for an antibody is thought to be about four to five amino acids. Peptide or polypeptide epitopes contain for example at least seven amino acids or for example at least nine amino acids or for example between about 15 to about 20 amino acids. Since an antibody can recognize an antigenic peptide or polypeptide in its tertiary form, the amino acids comprising an epitope need not be contiguous, and in some cases, may not even be on the same peptide chain. Epitopes may be determined by various techniques known in the art, such as X-ray crystallography, Hydrogen/Deuterium Exchange Mass Spectrometry (HXMS), site-directed mutagenesis, alanine scanning mutagenesis, and peptide screening methods.

The generalized structure of antibodies or immunoglobulin is well known to those of skill in the art. These molecules are heterotetrameric glycoproteins, typically of about 150,000 daltons, composed of two identical light (L) chains and two identical heavy (H) chains and are typically referred to as full length antibodies. Each light chain is covalently linked to a heavy chain by one disulfide bond to form a heterodimer, and the heterotrameric molecule is formed through a covalent disulfide linkage between the two identical heavy chains of the heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (V_{H}), followed by three or four constant domains (C_{H1}, C_{H2}, C_{H3}, and C_{H4}), as well as a hinge region between C_{H1} and C_{H2}. Each light chain has two domains, an amino-terminal variable domain (V_{L}) and a carboxy-terminal constant domain (C_{L}). The V_{L} domain associates non-covalently with the V_{H} domain, whereas the C_{L} domain is commonly covalently linked to the C_{H1} domain via a disulfide bond. Particular amino acid residues are believed to form an interface between the light and heavy chain variable domains (Chothia et al., 1985, J. Mol. Biol. 186:651-663). Variable domains are also referred herein as variable regions.

Certain domains within the variable domains differ extensively between different antibodies i.e., are "hypervariable." These hypervariable domains contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, Md., whereas HVLs (also referred herein as CDRs) are structurally defined according to the three-dimensional structure of the variable domain, as described by Chothia and Lesk, 1987, J. Mol. Biol. 196: 901-917. These two methods result in slightly different identifications of a CDR. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain. The exact residue numbers that encompass a particular CDR will vary depending on the sequence and size of the CDR. Those skilled in the art can routinely determine which residues comprise a particular CDR given the variable region amino acid sequence of the antibody. The CDR1, CDR2, CDR3 of the heavy and light chains therefore define the unique and functional properties specific for a given antibody.

The three CDRs within each of the heavy and light chains are separated by framework regions (FR), which contain sequences that tend to be less variable. From the amino terminus to the carboxy terminus of the heavy and light chain variable domains, the FRs and CDRs are arranged in the order: FR1, CDR1, FR2, CDR2, FR3, CDR3, and FR4. The largely β-sheet configuration of the FRs brings the CDRs within each of the chains into close proximity to each other as well as to the CDRs from the other chain. The resulting conformation contributes to the antigen binding site (see Kabat et al., 1991, NIH Publ. No. 91-3242, Vol. I, pages 647-669), although not all CDR residues are necessarily directly involved in antigen binding.

FR residues and Ig constant domains are not directly involved in antigen binding, but contribute to antigen binding and/or mediate antibody effector function. Some FR residues are thought to have a significant effect on antigen binding in at least three ways: by noncovalently binding directly to an epitope, by interacting with one or more CDR residues, and by affecting the interface between the heavy and light chains. The constant domains are not directly involved in antigen binding but mediate various Ig effector functions, such as participation of the antibody in antibody dependent cellular cytotoxicity (ADCC), complement dependent cytotoxicity (CDC) and antibody dependent cellular phagocytosis (ADCP).

The light chains of vertebrate immunoglobulins are assigned to one of two clearly distinct classes, kappa (κ) and lambda (λ), based on the amino acid sequence of the constant domain. By comparison, the heavy chains of mammalian immunoglobulins are assigned to one of five major classes, according to the sequence of the constant domains: IgA, IgD, IgE, IgG, and IgM. IgG and IgA are further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of the classes of native immunoglobulins are well known.

The terms, "antibody", "anti-IL-36R antibody", "humanized anti-IL-36R antibody", "humanized anti-IL-36R epitope antibody", and "variant humanized anti-IL-36R epitope antibody" specifically encompass monoclonal antibodies (including full length monoclonal antibodies), polyclonal antibodies, multispecific antibodies (e.g., bispecific antibodies), and antibody fragments such as variable domains and other portions of antibodies that exhibit a desired biological activity, e.g., IL-36R binding.The term "monoclonal antibody" (mAb) refers to an antibody that is highly specific, being directed against a single antigenic determinant, an "epitope". Therefore, the modifier "monoclonal" is indicative of antibodies directed to the identical epitope and is not to be construed as requiring production of the antibody by any particular method. It should be understood that monoclonal antibodies can be made by any technique or methodology known in the art; including e.g., the hybridoma method ( Kohler et al., 1975, Nature 256:495), or recombinant DNA methods known in the art (see, e.g., U.S. Pat. No. 4,816,567), or methods of isolation of monoclonal recombinantly produced using phage antibody libraries, using techniques described in Clackson et al., 1991, Nature 352: 624-628, and Marks et al., 1991, J. Mol. Biol. 222: 581-597.

The term "monomer" refers to a homogenous form of an antibody. For example, for a full-length antibody, monomer means a monomeric antibody having two identical heavy chains and two identical light chains.

Chimeric antibodies consist of the heavy and light chain variable regions of an antibody from one species (e.g., a non-human mammal such as a mouse) and the heavy and light chain constant regions of another species (e.g., human) antibody and can be obtained by linking the DNA sequences encoding the variable regions of the antibody from the first species (e.g., mouse) to the DNA sequences for the constant regions of the antibody from the second (e.g. human) species and transforming a host with an expression vector containing the linked sequences to allow it to produce a chimeric antibody. Alternatively, the chimeric antibody also could be one in which one or more regions or domains of the heavy and/or light chain is identical with, homologous to, or a variant of the corresponding sequence in a monoclonal antibody from another immunoglobulin class or isotype, or from a consensus or germline sequence. Chimeric antibodies can include fragments of such antibodies, provided that the antibody fragment exhibits the desired biological activity of its parent antibody, for example binding to the same epitope (see, e.g., U.S. Pat. No. 4,816,567; and Morrison et al., 1984, Proc. Natl. Acad. Sci. USA 81: 6851-6855).

The terms, "antibody fragment", "anti-IL-36R antibody fragment", "anti-IL-36R epitope antibody fragment", "humanized anti-IL-36R antibody fragment", "humanized anti-IL-36R epitope antibody fragment", "variant humanized anti-IL-36R epitope antibody fragment" refer to a portion of a full length anti-IL-36R antibody, in which a variable region or a functional capability is retained, for example, specific IL-36R epitope binding. Examples of antibody fragments include, but are not limited to, a Fab, Fab', F(ab')₂, Fd, Fv, scFv and scFv-Fc fragment, a diabody, a linear antibody, a single-chain antibody, a minibody, a diabody formed from antibody fragments, and multispecific antibodies formed from antibody fragments.

Full length antibodies can be treated with enzymes such as papain or pepsin to generate useful antibody fragments. Papain digestion is used to produces two identical antigen-binding antibody fragments called "Fab" fragments, each with a single antigen-binding site, and a residual "Fc" fragment. The Fab fragment also contains the constant domain of the light chain and the C_{H1} domain of the heavy chain. Pepsin treatment yields a F(ab')₂ fragment that has two antigen-binding sites and is still capable of crosslinking antigen.

Fab' fragments differ from Fab fragments by the presence of additional residues including one or more cysteines from the antibody hinge region at the C-terminus of the C_{H1} domain. F(ab')₂ antibody fragments are pairs of Fab' fragments linked by cysteine residues in the hinge region. Other chemical couplings of antibody fragments are also known.

"Fv" fragment contains a complete antigen-recognition and binding site consisting of a dimer of one heavy and one light chain variable domain in tight, noncovalent association. In this configuration, the three CDRs of each variable domain interact to define an antigen-biding site on the surface of the V_{H}-V_{L} dimer. Collectively, the six CDRs confer antigen-binding specificity to the antibody.

A "single-chain Fv" or "scFv" antibody fragment is a single chain Fv variant comprising the V_{H} and V_{L} domains of an antibody where the domains are present in a single polypeptide chain. The single chain Fv is capable of recognizing and binding antigen. The scFv polypeptide may optionally also contain a polypeptide linker positioned between the V_{H} and V_{L} domains in order to facilitate formation of a desired three-dimensional structure for antigen binding by the scFv (see, e.g., Pluckthun, 1994, In The Pharmacology of monoclonal Antibodies, Vol. 113, Rosenburg and Moore eds., Springer-Verlag, New York, pp. 269-315).

A "diabody" refers to small antibody fragments with two antigen-binding sites, which fragments comprise a heavy chain variable domain (V.sub.H) connected to a light chain variable domain (V.sub.L) in the same polypeptide chain (V.sub.H-V.sub.L or V.sub.L-V.sub.H). Diabodies are described more fully in, e.g., Holliger et al. (1993) Proc. Natl. Acad. Sci. USA 90: 6444-6448.

Other recognized antibody fragments include those that comprise a pair of tandem Fd segments (V_{H}-C_{H1}-V_{H}-C_{H1}) to form a pair of antigen binding regions. These "linear antibodies" can be bispecific or monospecific as described in, for example, Zapata et al. 1995, Protein Eng. 8(10):1057-1062.

A "humanized antibody" or a "humanized antibody fragment" is a specific type of chimeric antibody which includes an immunoglobulin amino acid sequence variant, or fragment thereof, which is capable of binding to a predetermined antigen and which, comprises one or more FRs having substantially the amino acid sequence of a human immunoglobulin and one or more CDRs having substantially the amino acid sequence of a non-human immunoglobulin. This non-human amino acid sequence often referred to as an "import" sequence is typically taken from an "import" antibody domain, particularly a variable domain. In general, a humanized antibody includes at least the CDRs or HVLs of a non-human antibody, inserted between the FRs of a human heavy or light chain variable domain. The present invention describes specific humanized anti-IL-36R antibodies which contain CDRs derived from the mouse monoclonal antibodies or humanized CDRs inserted between the FRs of human germline sequence heavy and light chain variable domains. It will be understood that certain mouse FR residues may be important to the function of the humanized antibodies and therefore certain of the human germline sequence heavy and light chain variable domains residues are modified to be the same as those of the corresponding mouse sequence.

In another aspect, a humanized anti-IL-36R antibody comprises substantially all of at least one, and typically two, variable domains (such as contained, for example, in Fab, Fab', F(ab')2, Fabc, and Fv fragments) in which all, or substantially all, of the CDRs correspond to those of a non-human immunoglobulin, and specifically herein, all of the CDRs are mouse or humanized sequences as detailed herein below and all, or substantially all, of the FRs are those of a human immunoglobulin consensus or germline sequence. In another aspect, a humanized anti- IL-36R antibody also includes at least a portion of an immunoglobulin Fc region, typically that of a human immunoglobulin. Ordinarily, the antibody will contain both the light chain as well as at least the variable domain of a heavy chain. The antibody also may include one or more of the C_{H1}, hinge, C_{H2}, C_{H3}, and/or C_{H4} regions of the heavy chain, as appropriate.

A humanized anti-IL-36r antibody can be selected from any class of immunoglobulins, including IgM, IgG, IgD, IgA and IgE, and any isotype, including IgG₁, IgG₂, IgG₃, IgG₄, IgA₁ and IgA₂. For example, the constant domain can be a complement fixing constant domain where it is desired that the humanized antibody exhibit cytotoxic activity, and the isotype is typically IgG₁. Where such cytotoxic activity is not desirable, the constant domain may be of another isotype, e.g., IgG₂. An alternative humanized anti-IL-36R antibody can comprise sequences from more than one immunoglobulin class or isotype, and selecting particular constant domains to optimize desired effector functions is within the ordinary skill in the art. In specific embodiments, the present invention provides antibodies that are IgG1 antibodies and more particularly, are IgG1 antibodies in which there is a knock-out of effector functions.

The FRs and CDRs, or HVLs, of a humanized anti-IL-36R antibody need not correspond precisely to the parental sequences. For example, one or more residues in the import CDR, or HVL, or the consensus or germline FR sequence may be altered (e.g., mutagenized) by substitution, insertion or deletion such that the resulting amino acid residue is no longer identical to the original residue in the corresponding position in either parental sequence but the antibody nevertheless retains the function of binding to IL-36R. Such alteration typically will not be extensive and will be conservative alterations. Usually, at least 75% of the humanized antibody residues will correspond to those of the parental consensus or germline FR and import CDR sequences, more often at least 90%, and most frequently greater than 95%, or greater than 98% or greater than 99%.

Immunoglobulin residues that affect the interface between heavy and light chain variable regions ("the V_{L}-V_{H} interface") are those that affect the proximity or orientation of the two chains with respect to one another. Certain residues that may be involved in interchain interactions include V_{L} residues 34, 36, 38, 44, 46, 87, 89, 91, 96, and 98 and V_{H} residues 35, 37, 39, 45, 47, 91, 93, 95, 100, and 103 (utilizing the numbering system set forth in Kabat et al., Sequences of Proteins of Immunological Interest (National Institutes of Health, Bethesda, Md., 1987)). U.S. Pat. No. 6,407,213 also discusses that residues such as V_{L} residues 43 and 85, and V_{H} residues 43 and 60 also may be involved in this interaction. While these residues are indicated for human IgG only, they are applicable across species. Important antibody residues that are reasonably expected to be involved in interchain interactions are selected for substitution into the consensus sequence.

The terms "consensus sequence" and "consensus antibody" refer to an amino acid sequence which comprises the most frequently occurring amino acid residue at each location in all immunoglobulins of any particular class, isotype, or subunit structure, e.g., a human immunoglobulin variable domain. The consensus sequence may be based on immunoglobulins of a particular species or of many species. A "consensus" sequence, structure, or antibody is understood to encompass a consensus human sequence as described in certain embodiments, and to refer to an amino acid sequence which comprises the most frequently occurring amino acid residues at each location in all human immunoglobulins of any particular class, isotype, or subunit structure. Thus, the consensus sequence contains an amino acid sequence having at each position an amino acid that is present in one or more known immunoglobulins, but which may not exactly duplicate the entire amino acid sequence of any single immunoglobulin. The variable region consensus sequence is not obtained from any naturally produced antibody or immunoglobulin. Kabat et al., 1991, Sequences of Proteins of Immunological Interest, 5th Ed. Public Health Service, National Institutes of Health, Bethesda, Md., and variants thereof.

Human germline sequences are found naturally in the human population. A combination of those germline genes generates antibody diversity. Germline antibody sequences for the light chain of the antibody come from conserved human germline kappa or lambda v-genes and j-genes. Similarly the heavy chain sequences come from germline v-, d- and j-genes (LeFranc, M-P, and LeFranc, G, "The Immunoglobulin Facts Book" Academic Press, 2001).

As used herein, "variant", "anti- IL-36R variant", "humanized anti- IL-36R variant", or "variant humanized anti- IL-36R" each refers to a humanized anti-IL-36R antibody having at least a light chain variable murine CDR. Variants include those having one or more amino acid changes in one or both light chain or heavy chain variable domains, provided that the amino acid change does not substantially impair binding of the antibody to IL-36R.

An "isolated" antibody is one that has been identified and separated and/or recovered from a component of its natural environment. Contaminant components of the antibody's natural environment are those materials that may interfere with diagnostic or therapeutic uses of the antibody, and can be enzymes, hormones, or other proteinaceous or nonproteinaceous solutes. In one aspect, the antibody will be purified to at least greater than 95% isolation by weight of antibody.

An isolated antibody includes an antibody in situ within recombinant cells in which it is produced, since at least one component of the antibody's natural environment will not be present. Ordinarily however, an isolated antibody will be prepared by at least one purification step in which the recombinant cellular material is removed.

The term "antibody performance" refers to factors that contribute to antibody recognition of antigen or the effectiveness of an antibody in vivo. Changes in the amino acid sequence of an antibody can affect antibody properties such as folding, and can influence physical factors such as initial rate of antibody binding to antigen (kₐ), dissociation constant of the antibody from antigen (k_{d}), affinity constant of the antibody for the antigen (Kd), conformation of the antibody, protein stability, and half life of the antibody.

The term "epitope tagged" when used herein, refers to an anti-IL-36R antibody fused to an "epitope tag". An "epitope tag" is a polypeptide having a sufficient number of amino acids to provide an epitope for antibody production, yet is designed such that it does not interfere with the desired activity of the humanized anti-IL-36R antibody. The epitope tag is usually sufficiently unique such that an antibody raised against the epitope tag does not substantially cross-react with other epitopes. Suitable tag polypeptides generally contain at least 6 amino acid residues and usually contain about 8 to 50 amino acid residues, or about 9 to 30 residues. Examples of epitope tags and the antibody that binds the epitope include the flu HA tag polypeptide and its antibody 12CA5 (Field et al., 1988 Mol. Cell. Biol. 8: 2159-2165; c-myc tag and 8F9, 3C7, 6E10, G4, B7 and 9E10 antibodies thereto (Evan et al., 1985, Mol. Cell. Biol. 5(12):3610-3616; and Herpes simplex virus glycoprotein D (gD) tag and its antibody (Paborsky et al. 1990, Protein Engineering 3(6): 547-553). In certain embodiments, the epitope tag is a "salvage receptor binding epitope". As used herein, the term "salvage receptor binding epitope" refers to an epitope of the Fc region of an IgG molecule (such as IgG₁, IgG₂, IgG₃, or IgG₄) that is responsible for increasing the in vivo serum half-life of the IgG molecule.

In some embodiments, the antibodies of the present invention may be conjugated to a cytotoxic agent. This is any substance that inhibits or prevents the function of cells and/or causes destruction of cells. The term is intended to include radioactive isotopes (such as I¹³¹, I¹²⁵, Y⁹⁰, and Re¹⁸⁶), chemotherapeutic agents, and toxins such as enzymatically active toxins of bacterial, fungal, plant, or animal origin, and fragments thereof. Such cytotoxic agents can be coupled to the humanized antibodies of the present invention using standard procedures, and used, for example, to treat a patient indicated for therapy with the antibody.

A "chemotherapeutic agent" is a chemical compound useful in the treatment of cancer. There are numerous examples of chemotherapeutic agents that could be conjugated with the therapeutic antibodies of the present invention. Examples of such chemotherapeutic agents include alkylating agents such a thiotepa and cyclosphosphamide; alkyl sulfonates such as busulfan, improsulfan, and piposulfan; aziridines such as benzodopa, carboquone, meturedopa, and uredopa; ethylenimines and methylamelamines including altretamine, triethylenemelamine, trietylenephosphoramide, triethylenethiophosphoramide, and trimethylolomelamine; acetogenins (especially bullatacin and bullatacinone); camptothecin (including the synthetic analogue topotecan); bryostatin; callystatin; CC-1065 (including its adozelesin, carzelesin, and bizelesin synthetic analogues); cryptophycines (particularly cryptophycin 1 and cryptophycin 8); dolastatin, auristatins, (including analogues monomethyl-auristatin E and monomethyl-auristatin F); duocarmycin (including the synthetic analogues, KW-2189 and CBI-TMI); eleutherobin; pancratistatin; sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine; trofosfamide, uracil mustard; nitrosureas such as carmustine, chlorozotocin, fotemustine, lomustine, nimustine, ranimustine; antibiotics such as the enediyne antibiotics (e.g., calicheamicin, especially calichemicin gamma1I and calicheamicin phil1, see for example, Agnew, Chem. Intl. Ed. Engl., 33:183-186; dynemicin, including dynemicin A; bisphosphonates, such as clodronate; esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (Adriamycin^{™}) (including morpholino-doxorubicin, cyanomorpholino-doxorubicin, 2-pyrrolino-doxorubicin, and deoxydoxorubicin), epirubucin, esorubicin, idarubicin, marcellomycin, mitomycins such as mitomycin C, mycophenolic acid, nogalamycin, olivomycins, peplomycin, potfiromycin, puromycine, quelamycin, rodorubicin, streptonigrin, streptozocin, tubercidin, ubenimex, zinostatin, zorubicin; anti-metabolites such a methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adranals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; democolcine; diaziquone; elfomithine; elliptinium acetate; an epothilone; etoglucid; gallium nitrate; hydroxyurea; lentinan; lonidamine; maytansinoids such as maytansine and ansamitocins; mitoguazone, mitoxantrone; mopidamol; nitracrine; pentostatin; phenamet; pirarubicin; losoxantrone; podophyllinic acid; 2-ethylhydrazide; procarbazine; PSK^{®}; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitabronitol; mitolactol; pipobroman; gacytosine; arabinoside ("Ara-C"); cyclophosphamide; thiotepa; taxoids, e.g., paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine (Gemzar^{™}); 6-thioguanine; mercaptopurine; methotrexate; platinum analogs such as cisplatin and carboplatin; vinblastine; platinum; etoposide (VP-16); ifosfamide; mitoxantrone; vincristine; vinorelbine Navelbine^{™}); novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids, or derivatives of any of the above. Also included in this definition are anti-hormonal agents that act to regulate or inhibit hormone action on tumors such as anti-estrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}); aromatase inhibitors that inhibit the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}); and anti-androgens such as flutamide, nilutamide, bicalutamide, leuprolide, and goserelin; and pharmaceutically acceptable salts, acids, or derivatives of any of the above. Any one or more of these agents may be conjugated to the humanized antibodies of the present invention to provide a useful therapeutic agent for the treatment of various disorders.

The antibodies also may be conjugated to prodrugs. A "prodrug" is a precursor or derivative form of a pharmaceutically active substance that is less cytotoxic to tumor cells compared to the parent drug and is capable of being enzymatically activated or converted into the more active form. See, for example, Wilman, 1986, "Prodrugs in Cancer Chemotherapy", In Biochemical Society Transactions, 14, pp. 375-382, 615th Meeting Belfast and Stella et al., 1985, "Prodrugs: A Chemical Approach to Targeted Drug Delivery, In: "Directed Drug Delivery, Borchardt et al., (ed.), pp. 247-267, Humana Press. Useful prodrugs include, but are not limited to, phosphate-containing prodrugs, thiophosphate-containing prodrugs, sulfate-containing prodrugs peptide-containing prodrugs, D-amino acid-modified prodrugs, glycosylated prodrugs, β-lactam-containing prodrugs, optionally substituted phenoxyacetamide-containing prodrugs, and optionally substituted phenylacetamide-containing prodrugs, 5-fluorocytosine and other 5-fluorouridine prodrugs that can be converted into the more active cytotoxic free drug. Examples of cytotoxic drugs that can be derivatized into a prodrug form include, but are not limited to, those chemotherapeutic agents described above.

For diagnostic as well as therapeutic monitoring purposes, the antibodies of the invention also may be conjugated to a label, either a label alone or a label and an additional second agent (prodrug, chemotherapeutic agent and the like). A label, as distinguished from the other second agents refers to an agent that is a detectable compound or composition and it may be conjugated directly or indirectly to a humanized antibody of the present invention. The label may itself be detectable (e.g., radioisotope labels or fluorescent labels) or, in the case of an enzymatic label, may catalyze chemical alteration of a substrate compound or composition that is detectable. Labeled humanized anti-IL-36R antibody can be prepared and used in various applications including in vitro and in vivo diagnostics.

The antibodies of the present invention may be formulated as part of a liposomal preparation in order to affect delivery thereof in vivo. A "liposome" is a small vesicle composed of various types of lipids, phospholipids, and/or surfactant. Liposomes are useful for delivery to a mammal of a compound or formulation, such as a humanized anti-IL-36R antibody disclosed herein, optionally, coupled to or in combination with one or more pharmaceutically active agents and/or labels. The components of the liposome are commonly arranged in a bilayer formation, similar to the lipid arrangement of biological membranes.

Certain aspects of the present invention related to isolated nucleic acids that encode one or more domains of the humanized antibodies of the present invention. An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is distinguished from the nucleic acid molecule as it exists in natural cells.

In various aspects of the present invention one or more domains of the humanized antibodies will be recombinantly expressed. Such recombinant expression may employ one or more control sequences, i.e., polynucleotide sequences necessary for expression of an operably linked coding sequence in a particular host organism. The control sequences suitable for use in prokaryotic cells include, for example, promoter, operator, and ribosome binding site sequences. Eukaryotic control sequences include, but are not limited to, promoters, polyadenylation signals, and enhancers. These control sequences can be utilized for expression and production of humanized anti-IL-36R antibody in prokaryotic and eukaryotic host cells.

A nucleic acid sequence is "operably linked" when it is placed into a functional relationship with another nucleic acid sequence. For example, a nucleic acid presequence or secretory leader is operably linked to a nucleic acid encoding a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers are optionally contiguous. Linking can be accomplished by ligation at convenient restriction sites. If such sites do not exist, synthetic oligonucleotide adaptors or linkers can be used.

As used herein, the expressions "cell", "cell line", and "cell culture" are used interchangeably and all such designations include the progeny thereof. Thus, "transformants" and "transformed cells" include the primary subject cell and cultures derived therefrom without regard for the number of transfers.

The term "mammal" for purposes of treatment refers to any animal classified as a mammal, including humans, domesticated and farm animals, and zoo, sports, or pet animals, such as dogs, horses, cats, cows, and the like. Preferably, the mammal is human or a patient.

An IL-36R-associated disorder includes diseases and disorders of the immune system, such as autoimmune disorders and inflammatory disorders. Such conditions include, but are not limited to, rheumatoid arthritis (RA), systemic lupus erythematosus (SLE), scleroderma, Sjogren's syndrome, multiple sclerosis, psoriasis, psoriatic arthritis, inflammatory bowel disease (e.g., ulcerative colitis and Crohn's disease), pulmonary inflammation, asthma, idiopathic thrombocytopenic purara (ITP) epithelial inflammatory disorders, fibrosis and ankylosing spondylitis.

The term "intravenous infusion" refers to introduction of an agent into the vein of an animal or human patient over a period of time greater than approximately 15 minutes, generally between approximately 30 to 90 minutes.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is less than approximately 15 minutes; in another aspect, less than 5 minutes, and in still another aspect, less than 60 seconds. In yet even another aspect, administration is within a pocket between the skin and underlying tissue, where the pocket may be created by pinching or drawing the skin up and away from underlying tissue.

The term "therapeutically effective amount" is used to refer to an amount of an active agent that relieves or ameliorates one or more of the symptoms of the disorder being treated. In another aspect, the therapeutically effective amount refers to a target serum concentration that has been shown to be effective in, for example, slowing disease progression. Efficacy can be measured in conventional ways, depending on the condition to be treated.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" as used herein. Moreover, as long as the compositions of the invention either alone or in combination with another therapeutic agent alleviate or ameliorate at least one symptom of a disorder being treated as compared to that symptom in the absence of use of the humanized anti-IL-36R antibody composition, the result should be considered an effective treatment of the underlying disorder regardless of whether all the symptoms of the disorder are alleviated or not.

The term "package insert" is used to refer to instructions customarily included in commercial packages of therapeutic products, that contain information about the indications, usage, administration, contraindications and/or warnings concerning the use of such therapeutic products.

### Antibodies

In one aspect, described and disclosed herein are anti-IL-36R antibodies, in particular humanized anti-IL-36R antibodies, and compositions and articles of manufacture comprising one or more anti-IL-36R antibody, in particular one or more humanized anti-IL-36R antibody of the present invention. Also described are binding agents that include an antigen-binding fragment of an anti-IL-36 antibody, in particular a humanized anti-IL-36R antibody.

Variable regions and CDRs of representative antibodies of the present invention are disclosed below:

### Anti-IL-36R Mouse Antibody Sequences

Variable regions and CDRs of representative mouse lead antibodies of the present invention (mouse leads) are shown below:
**Light Chain Variable Region (VK) Amino Acid Sequences**

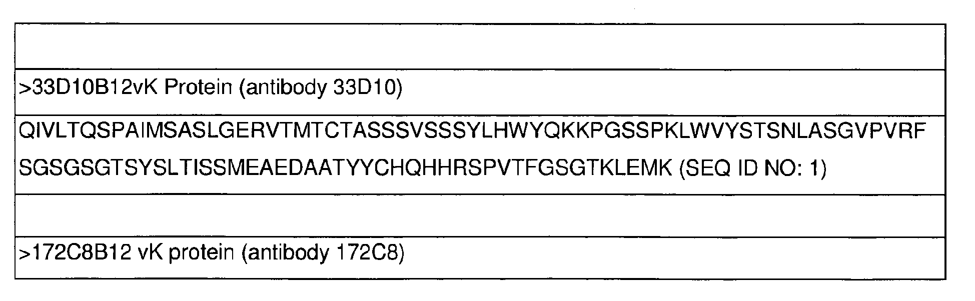

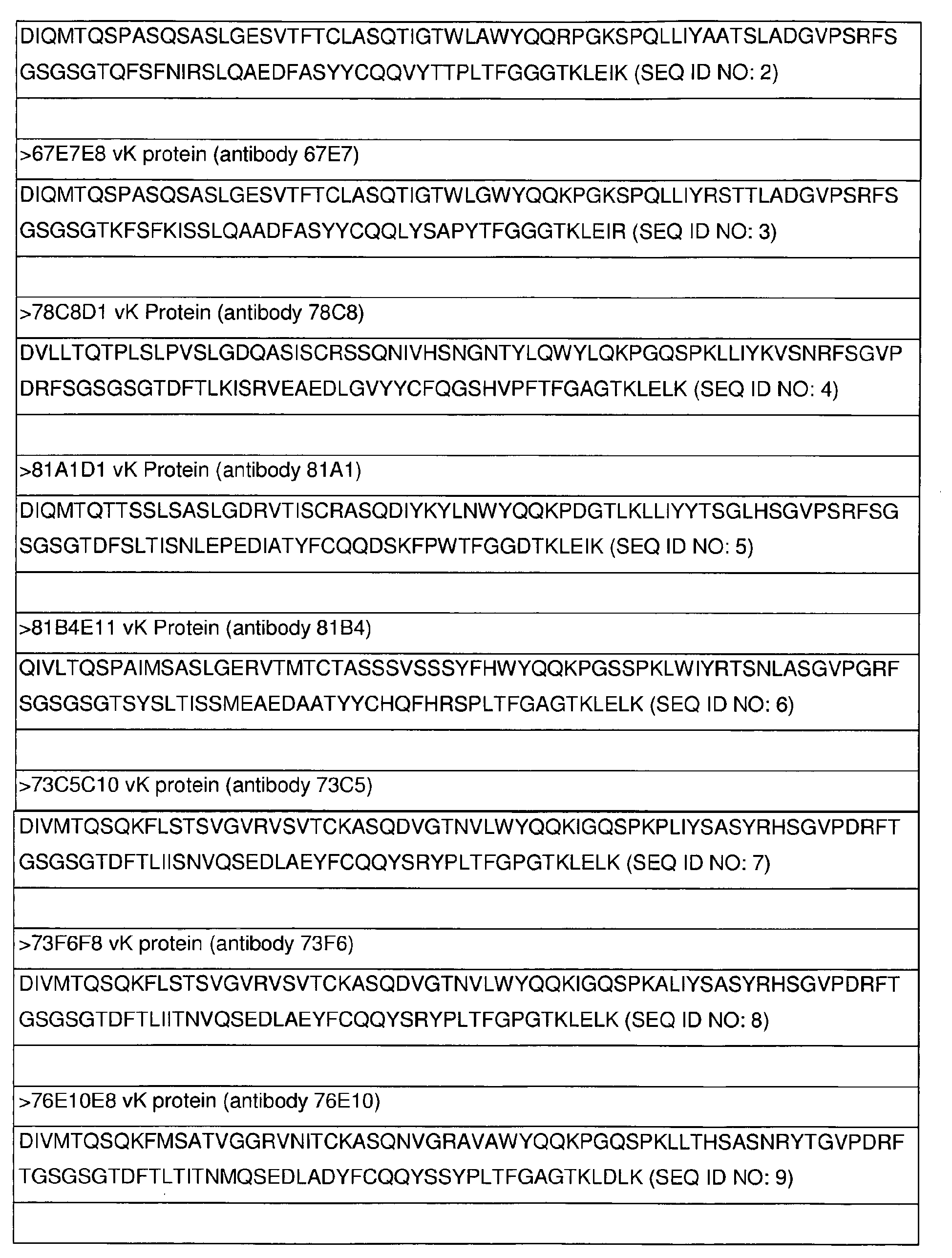

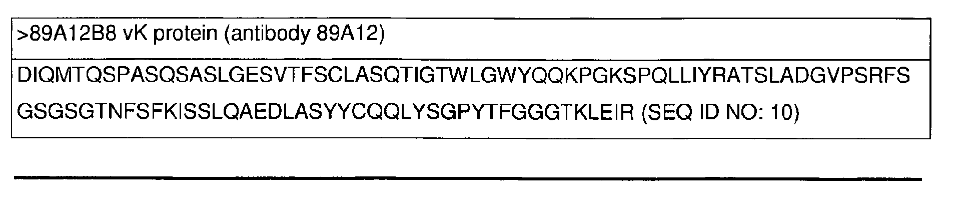

**Heavy Chain Variable Region (VH) Amino Acid Sequences**

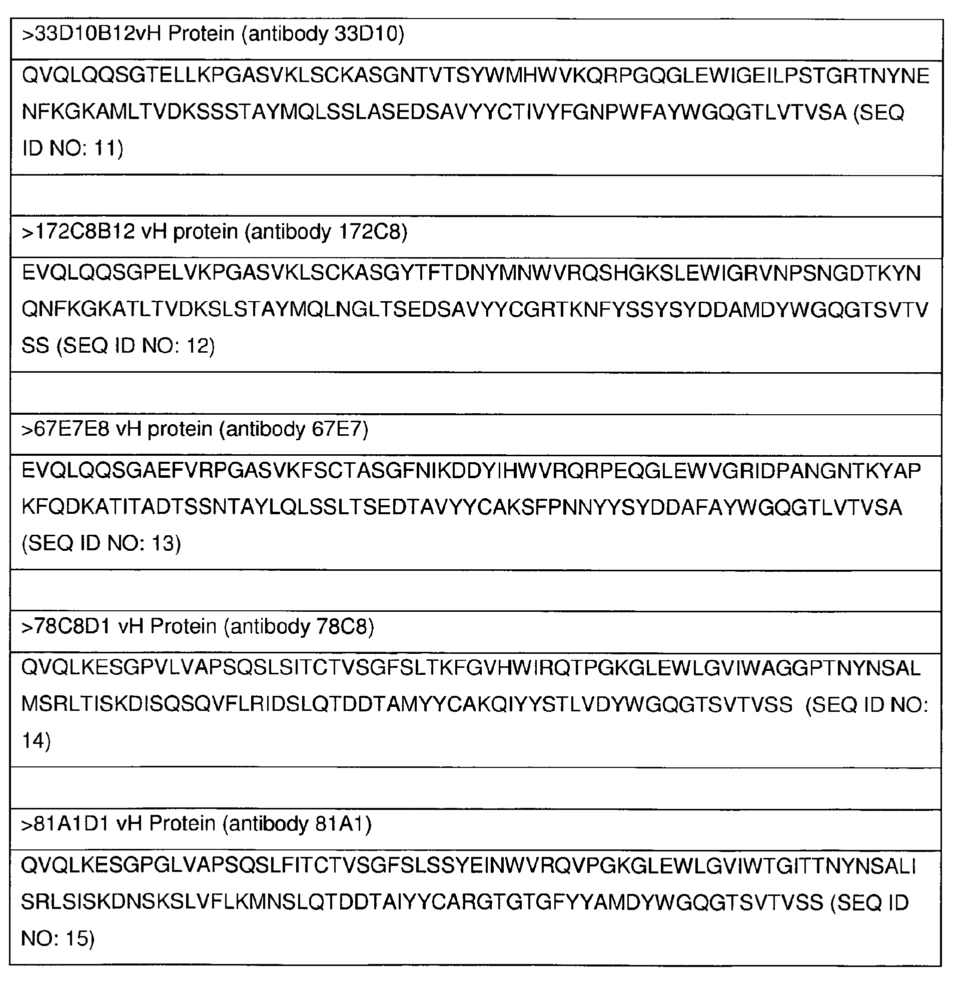

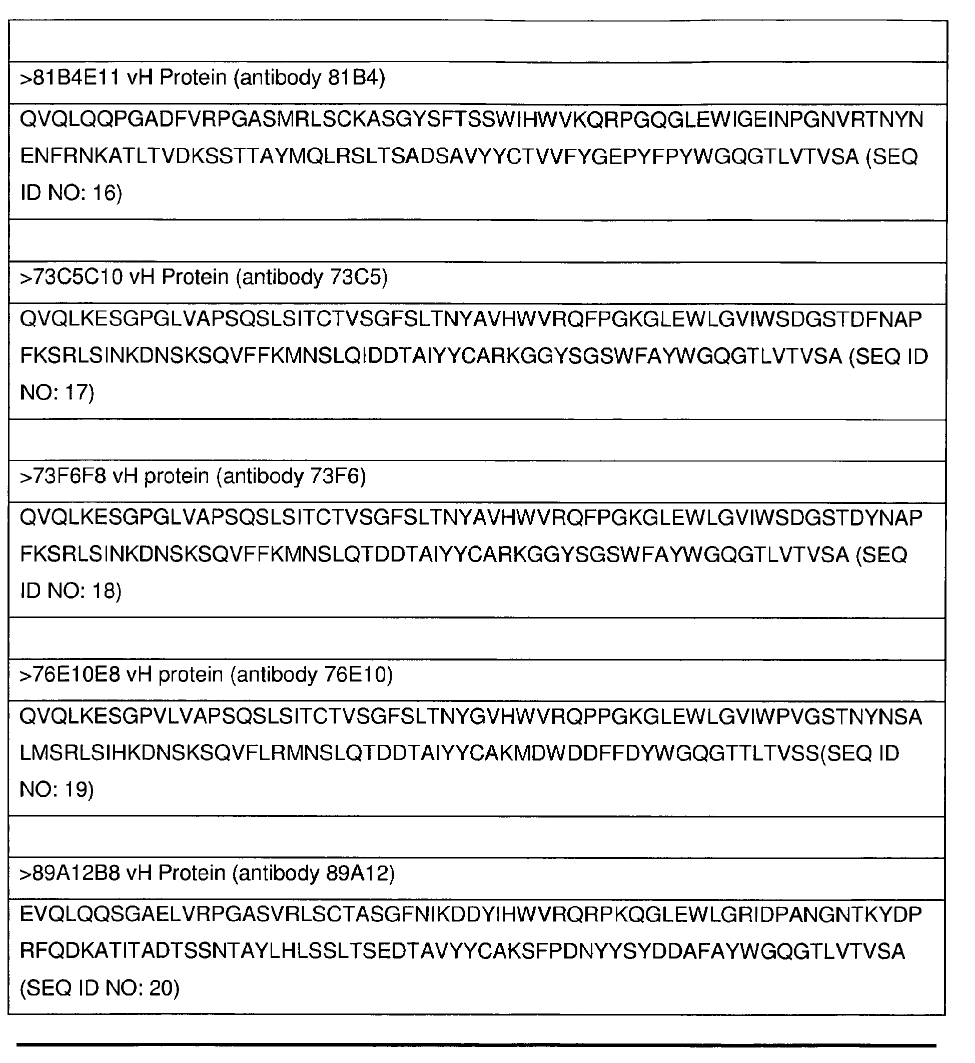

**Light chain CDR-1 (L-CDR1) Amino Acid Sequences**
>33D10G1 L-CDR1
   TASSSVSSSYLH (SEQ ID NO: 21)
>172C8B12 L-CDR1
   LASQTIGTWLA (SEQ ID NO: 22)
>67E7E8 L-CDR1
   LASQTIGTWLG (SEQ ID NO: 23)
>78C8D1 L-CDR1
   RSSQNIVHSNGNTYLQ (SEQ ID NO: 24)
>81A1D1 L-CDR1
   RASQDIYKYLN (SEQ ID NO: 25)
>81 B4E11 L-CDR1
   TASSSVSSSYFH (SEQ ID NO: 26)
>73C5C10 L-CDR1
   KASQDVGTNVL (SEQ ID NO: 27)
>73F6F8 L-CDR1
   KASQDVGTNVL (SEQ ID NO: 27)
>76E10E8 L-CDR1
   KASQNVGRAVA (SEQ ID NO: 28)
>89A12B8 L-CDR1
   LASQTIGTWLG (SEQ ID NO: 29)
**Light chain CDR-2 (L-CDR2) Amino Acid Sequences**
>33D10B12 L-CDR2
   STSNLAS (SEQ ID NO: 30)
>172C8B12 L-CDR2
   AATSLAD (SEQ ID NO: 31)
>67E7E8 L-CDR2
   RSTTLAD (SEQ ID NO: 32)
>78C8D1 L-CDR2
   KVSNRFS (SEQ ID NO: 33)
>81A1D1 L-CDR2
   YTSGLHS (SEQ ID NO: 34)
>81B4E11 L-CDR2
   RTSNLAS (SEQ ID NO: 35)
>73C5C10 L-CDR2
   SASYRHS (SEQ ID NO: 36)
>73F6F8 L-CDR2
   SASYRHS (SEQ ID NO: 36)
>76E10E8 L-CDR2
   SASNRYT (SEQ ID NO: 37)
>89A12B8 L-CDR2
   RATSLAD (SEQ ID NO: 38)
**Light chain CDR-3 (L-CDR3) Amino Acid Sequences**
>33D10B12 L-CDR3
   HQHHRSPVT (SEQ ID NO: 39)
>172C8B12 L-CDR3
   QQVYTTPLT (SEQ ID NO: 40)
>67E7E8 L-CDR3
   QQLYSAPYT (SEQ ID NO: 41)
>78C8D1 L-CDR3
   FQGSHVPFT (SEQ ID NO: 42)
>81A1D1 L-CDR3
   QQDSKFPWT (SEQ ID NO: 43)
>81 B4E11 L-CDR3
   HQFHRSPLT (SEQ ID NO: 44)
>73C5C10 L-CDR3
   QQYSRYPLT (SEQ ID NO: 45)
>73F6F8 L-CDR3
   QQYSRYPLT (SEQ ID NO: 45)
>76E10E8 L-CDR3
   QQYSSYPLT (SEQ ID NO: 46)
>89A12B8 L-CDR3
   QQLYSGPYT (SEQ ID NO: 47)
**Heavy chain CDR-1 (H-CDR1) Amino Acid Sequences**
>33D10B12 H-CDR1
   GNTVTSYWMH (SEQ ID NO: 48)
>172C8B12 H-CDR1
   GYTFTDNYMN (SEQ ID NO: 49)
>67E7E8 H-CDR1
   GFNIKDDYIH (SEQ ID NO: 50)
>78C8D1 H-CDR1
   GFSLTKFGVH (SEQ ID NO: 51)
>81A1D1 H-CDR1
   GFSLSSYEIN (SEQ ID NO: 52)
>81B4E11 H-CDR1
   GYSFTSSWIH (SEQ ID NO: 53)
>73C5C10 H-CDR1
   GFSLTNYAVH (SEQ ID NO: 54)
>73F6F8 H-CDR1
   GFSLTNYAVH (SEQ ID NO: 54)
>76E10E8 H-CDR1
   GFSLTNYGVH (SEQ ID NO: 55)
>89A12B8 H-CDR1
   GFNIKDDYIH (SEQ ID NO: 56)
**Heavy chain CDR-2 (H-CDR2) Amino Acid Sequences**
>33D10B12 H-CDR2
   EILPSTGRTNYNENFKG (SEQ ID NO: 57)
>172C8B12 H-CDR2
   RVNPSNGDTKYNQNFKG (SEQ ID NO: 58)
>67E7E8 H-CDR2
   RIDPANGNTKYAPKFQD (SEQ ID NO: 59)
>78C8D1 H-CDR2
   VIWAGGPTNYNSALMS (SEQ ID NO: 60)
>81A1D1 H-CDR2
   VIWTGITTNYNSALIS (SEQ ID NO: 61)
>81B4E11 H-CDR2
   EINPGNVRTNYNENF (SEQ ID NO: 62)
>73C5C10 H-CDR2
   VIWSDGSTDFNAPFKS (SEQ ID NO: 63)
>73F6F8 H-CDR2
   VIWSDGSTDYNAPFKS (SEQ ID NO: 64)
>76E10E8 H-CDR2
   VIWPVGSTNYNSALMS (SEQ ID NO: 65)
>89A12B8 H-CDR2
   RIDPANGNTKYDPRFQD (SEQ ID NO: 66)
**Heavy chain CDR-3 (H-CDR3) Amino Acid Sequences**
>33D10B12 H-CDR3
   VYFGNPWFAY (SEQ ID NO: 67)
>172C8B12 H-CDR3
   TKNFYSSYSYDDAMDY (SEQ ID NO: 68)
>67E7E8 H-CDR3
   SFPNNYYSYDDAFAY (SEQ ID NO: 69)
>78C8D1 H-CDR3
   QIYYSTLVDY (SEQ ID NO: 70)
>81A1D1 H-CDR3
   GTGTGFYYAMDY (SEQ ID NO: 71)
>81B4E11 H-CDR3
   VFYGEPYFPY (SEQ ID NO: 72)
>73C5C10 H-CDR3
   KGGYSGSWFAY (SEQ ID NO: 73)
>73F6F8 H-CDR3
   KGGYSGSWFAY (SEQ ID NO: 73)
>76E10E8 H-CDR3
   MDWDDFFDY (SEQ ID NO: 74)
>89A12B8 H-CDR3
   SFPDNYYSYDDAFAY (SEQ ID NO: 75)

### Anti-IL-36R Mouse CDR Sequences

A summary of the CDR sequences of the lead mouse antibodies is shown below:

| Antibody | H-CDR Sequences | L-CDR Sequences |
|---|---|---|
| 33D10 | GNTVTSYWMH (H-CDR1) SEQ ID No: 48 | TASSSVSSSYLH (L-CDR1) SEQ ID No: 21 |
| | EILPSTGRTNYNENFKG (H-CDR2) SEQ ID No: 57 | STSNLAS (L-CDR2) SEQ ID No: 30 |
| | VYFGNPWFAY (H-CDR3) SEQ ID No: 67 | HQHHRSPVT (L-CDR3) SEQ ID No: 39 |
| 172C8 | GYTFTDNYMN (H-CDR1) SEQ ID No: 49 | LASQTIGTWLA (L-CDR1) SEQ ID No: 22 |
| | RVNPSNGDTKYNQNFKG (H-CDR2) SEQ ID No: 58 | AATSLAD (L-CDR2) SEQ ID No: 31 |
| | TKNFYSSYSYDDAMDY (H-CDR3) SEQ ID No: 68 | QQVYTTPLT (L-CDR3) SEQ ID No: 40 |
| 67E7 | GFNIKDDYIH (H-CDR1) SEQ ID No: 50 | LASQTIGTWLG (L-CDR1) SEQ ID No: 23 |
| | RIDPANGNTKYAPKFQD (H-CDR2) SEQ ID No: 59 | RSTTLAD (L-CDR2) SEQ ID No: 32 |
| | SFPNNYYSYDDAFAY (H-CDR3) SEQ ID No: 69 | QQLYSAPYT (L-CDR3) |
| 78C8 | GFSLTKFGVH (H-CDR1) SEQ ID No: 51 | RSSQNIVHSNGNTYLQ (L-CDR1) SEQ ID No: 24 |
| | VIWAGGPTNYNSALMS (H-CDR2) SEQ ID No: 60 | KVSNRFS (L-CDR2) SEQ ID No: 33 |
| | QIYYSTLVDY (H-CDR3) SEQ ID No: 70 | FQGSHVPFT (L-CDR3) SEQ ID No: 42 |
| 81A1 | GFSLSSYEIN (H-CDR1) SEQ ID No: 52 | RASQDIYKYLN (L-CDR1) SEQ ID No: 25 |
| | VIWTGITTNYNSALIS (H-CDR2) SEQ ID No: 61 | YTSGLHS (L-CDR2) SEQ ID No: 34 |
| | | QQDSKFPWT (L-CDR3) SEQ ID No: 43 |
| | GTGTGFYYAMDY (H-CDR3) SEQ ID No: 71 | |
| 81B4 | GYSFTSSWIH (H-CDR1) SEQ ID No: 53 | TASSSVSSSYFH (L-CDR1) SEQ ID No: 26 |
| | EINPGNVRTNYNENF (H-CDR2) SEQ ID No: 62 | RTSNLAS (L-CDR2) SEQ ID No: 35 |
| | | HQFHRSPLT (L-CDR3) SEQ ID No: 44 |
| | VFYGEPYFPY (H-CDR3) SEQ ID No: 72 | |
| 73C5 | GFSLTNYAVH (H-CDR1) SEQ ID No: 54 | KASQDVGTNVL (L-CDR1) SEQ ID No: 27 |
| | VIWSDGSTDFNAPFKS (H-CDR2) SEQ ID No: 63 | SASYRHS (L-CDR2) SEQ ID No: 36 |
| | KGGYSGSW FAY (H-CDR3) SEQ ID No: 73 | QQYSRYPLT (L-CDR3) SEQ ID No: 45 |
| 73F6 | GFSLTNYAVH (H-CDR1) SEQ ID No: 54 | KASQDVGTNVL (L-CDR1) SEQ ID No:27 |
| | VIWSDGSTDYNAPFKS (H-CDR2) SEQ ID No: 64 | SASYRHS (L-CDR2) SEQ ID No: 36 |
| | KGGYSGSWFAY (H-CDR3) SEQ ID No: 73 | QQYSRYPLT (L-CDR3) SEQ ID No: 45 |
| 76E10 | GFSLTNYGVH (H-CDR1) SEQ ID No: 55 | KASQNVGRAVA (L-CDR1) SEQ ID No: 28 |
| | VIWPVGSTNYNSALMS (H-CDR2) SEQ ID No: 65 | SASNRYT (L-CDR2) SEQ ID No: 37 |
| | MDWDDFFDY (H-CDR3) SEQ ID No: 74 | QQYSSYPLT (L-CDR3) SEQ ID No: 46 |
| 89A12 | GFNIKDDYIH (H-CDR1) SEQ ID No: 56 | LASQTIGTWLG (L-CDR1) SEQ ID No: 29 |
| | RIDPANGNTKYDPRFQD (H-CDR2) SEQ ID No: 66 | RATSLAD (L-CDR2) SEQ ID No: 38 |
| | | QQLYSGPYT (L-CDR3) |
| | SFPDNYYSYDDAFAY (H-CDR3) SEQ ID No: 75 | SEQ ID No: 47 |

### Anti-IL-36R Humanized Antibody Sequences

Human framework sequences were selected for the mouse leads based on the framework homology, CDR structure, conserved canonical residues, conserved interface packing residues and other parameters to produce humanized variable regions (see Example 5).

Representative humanized variable regions derived from antibodies 81B4 and 73C5 are shown below.

### Light Chain Variable Region (VK) Amino Acid Sequences

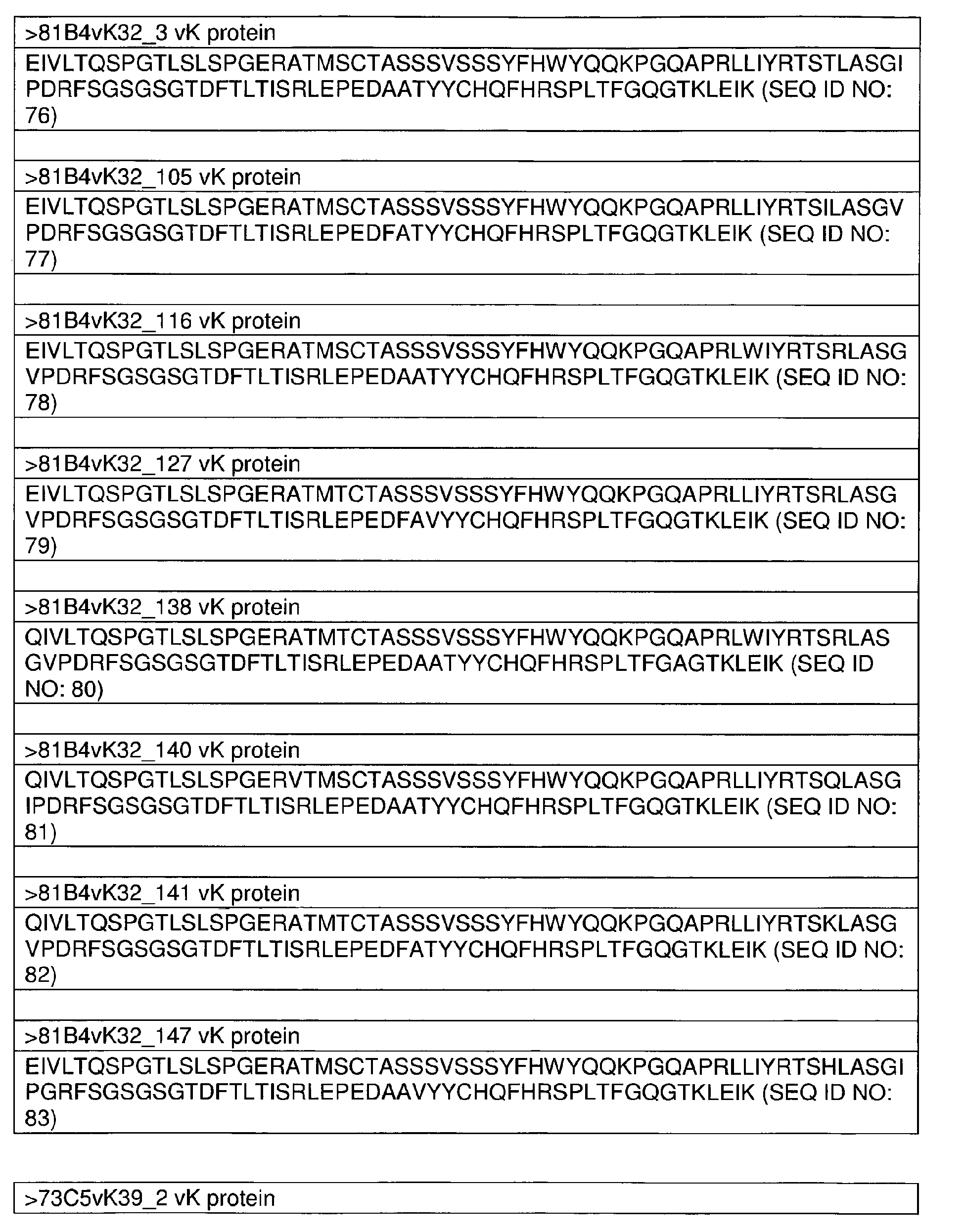

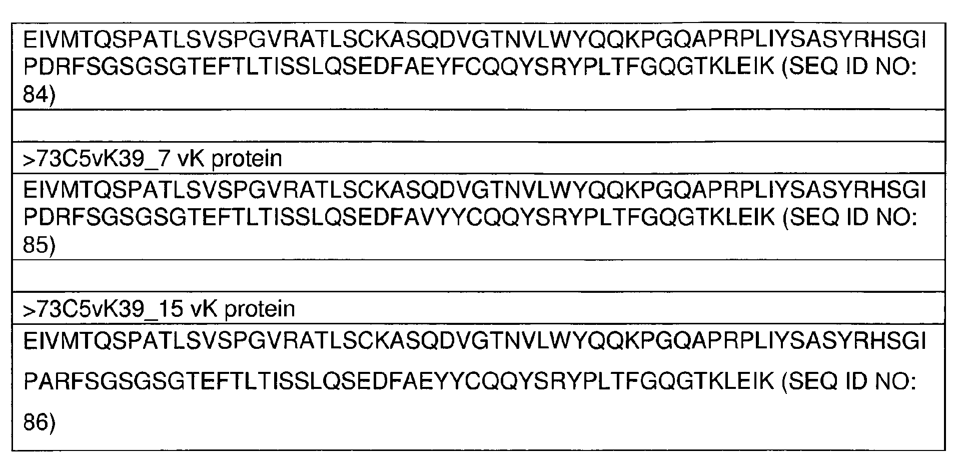

### Heavy Chain Variable Region (VH) Amino Acid Sequences

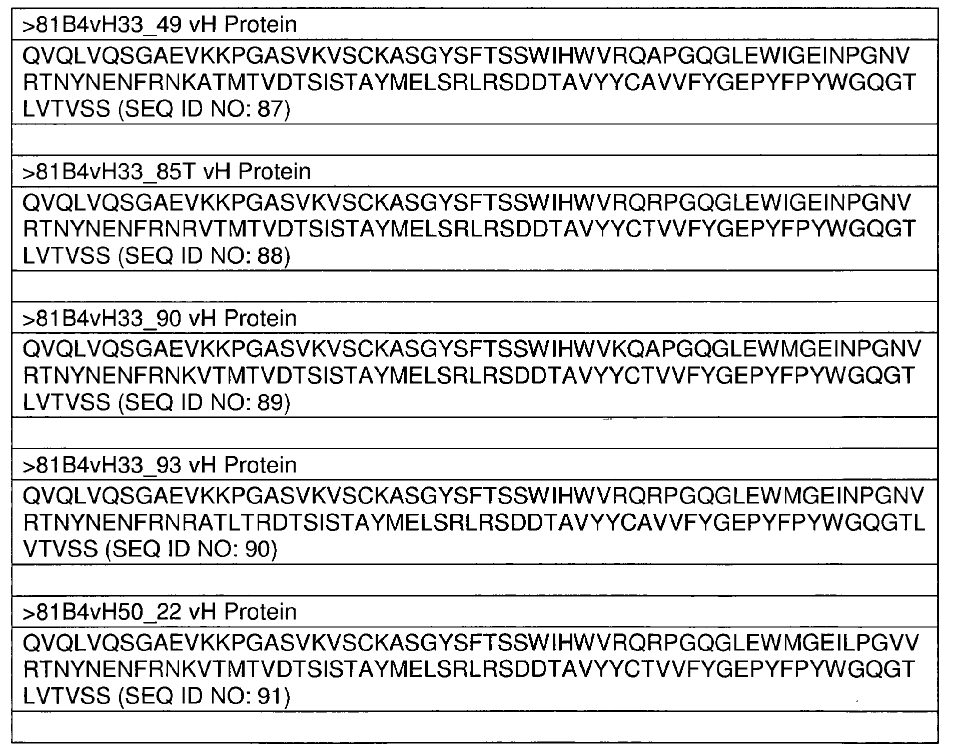

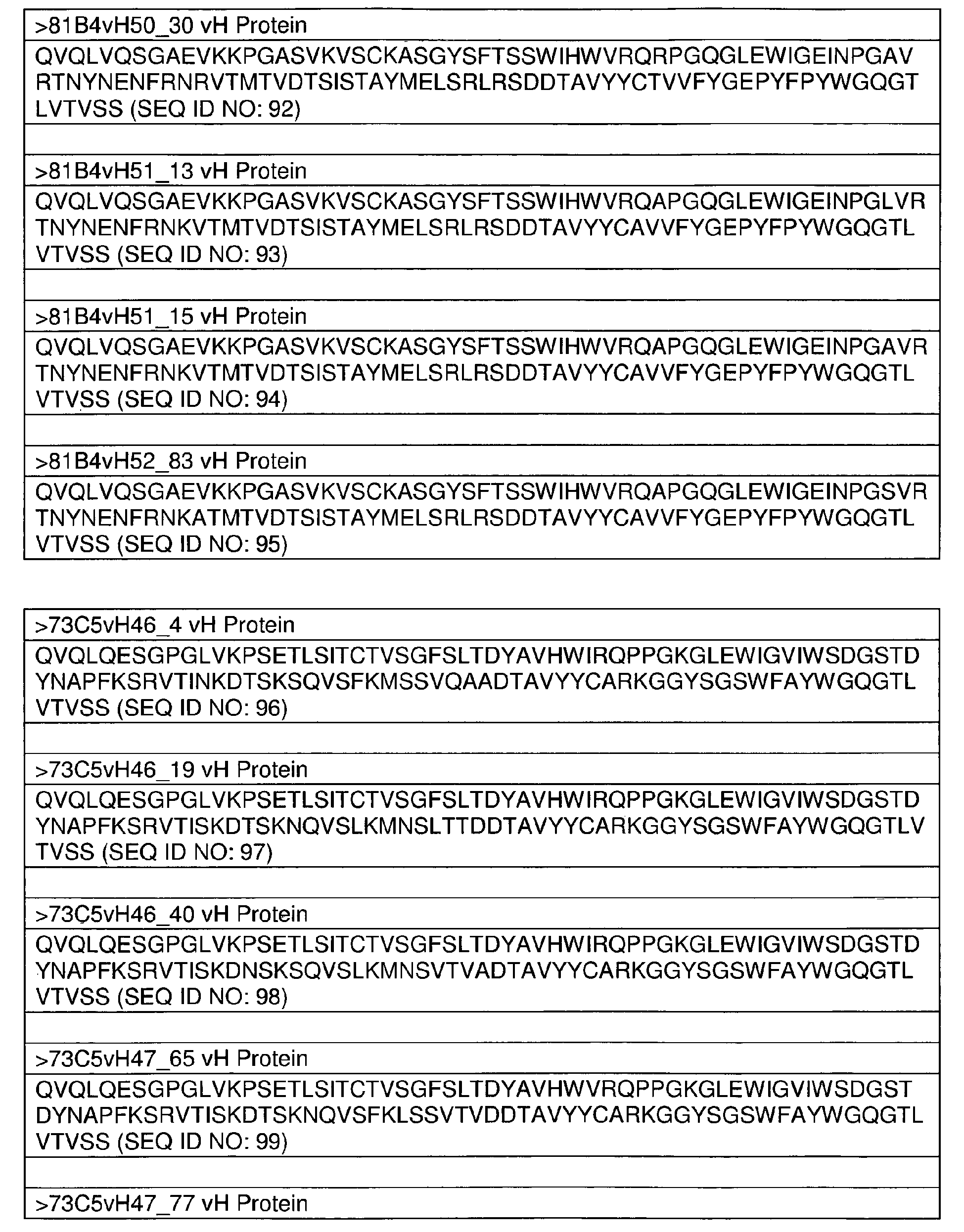

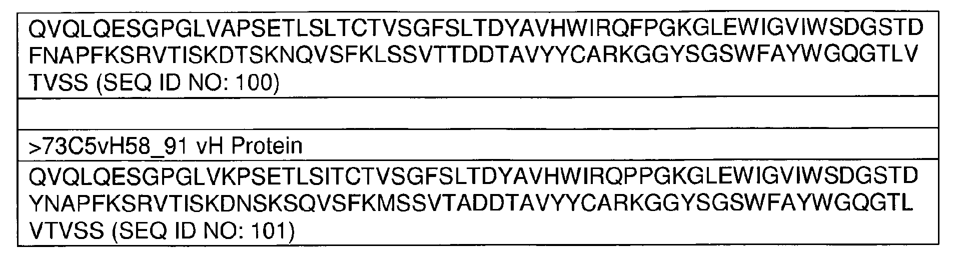

The CDR sequences from the humanized variable regions derived from antibodies 81B4 and 73C5 shown above are depicted below.
L-CDR1 Amino Acid Sequences
   >81B4vK32_3 L-CDR1
      TASSSVSSSYFH (SEQ ID NO: 26)
   >81B4vK32_105 L-CDR1
      TASSSVSSSYFH (SEQ ID NO: 26)
   >81B4vK32_116 L-CDR1
      TASSSVSSSYFH (SEQ ID NO: 26)
   >81B4vK32_127 L-CDR1
      TASSSVSSSYFH (SEQ ID NO: 26)
   >81B4vK32_138 L-CDR1
      TASSSVSSSYFH (SEQ ID NO: 26)
   >81 B4vK32_140 L-CDR1
      TASSSVSSSYFH (SEQ ID NO: 26)
   >81B4vK32_141 L-CDR1
      TASSSVSSSYFH (SEQ ID NO: 26)
   >81B4vK32_147 L-CDR1
      TASSSVSSSYFH (SEQ ID NO: 26)
   >73C5vK39_2 L-CDR1
      KASQDVGTNVL (SEQ ID NO: 27)
   >73C5vK39_7 L-CDR1
      KASQDVGTNVL (SEQ ID NO: 27)
   >73C5vK39_15 L-CDR1
      KASQDVGTNVL (SEQ ID NO: 27)
L-CDR2 Amino Acid Sequences
   >81B4vK32_3 L-CDR2 (SEQ ID 102)
      RTSTLAS
   >81B4vK32_105 L-CDR2 (SEQ ID 103)
      RTSILAS
   >81B4vK32_116 L-CDR2 (SEQ ID 104)
      RTSRLAS
   >81B4vK32_127 L-CDR2 (SEQ ID 104)
      RTSRLAS
   >81B4vK32_138 L-CDR2 (SEQ ID 104)
      RTSRLAS
   >81B4vK32_140 L-CDR2 (SEQ ID 105)
      RTSQLAS
   >81B4vK32_141 L-CDR2 (SEQ ID 106)
      RTSKLAS
   >81B4vK32_147 L-CDR2 (SEQ ID 140)
      RTSHLAS
   >73C5vK39_2 L-CDR2
      SASYRHS (SEQ ID NO: 36)
   >73C5vK39_7 L-CDR2
      SASYRHS (SEQ ID NO: 36)
   >73C5vK39_15 L-CDR2
      SASYRHS (SEQ ID NO: 36)
L-CDR3 Amino Acid Sequences
   >81B4vK32_3 L-CDR3
      HQFHRSPLT (SEQ ID NO: 44)
   >81B4vK32_105 L-CDR3
      HQFHRSPLT (SEQ ID NO: 44)
   >81B4vK32_116 L-CDR3
      HQFHRSPLT (SEQ ID NO: 44)
   >81B4vK32_127 L-CDR3
      HQFHRSPLT (SEQ ID NO: 44)
   >81B4vK32_138 L-CDR3
      HQFHRSPLT (SEQ ID NO: 44)
   >81B4vK32_140 L-CDR3
      HQFHRSPLT (SEQ ID NO: 44)
   >81B4vK32_141 L-CDR3
      HQFHRSPLT (SEQ ID NO: 44)
   >81B4vK32_147 L-CDR3
      HQFHRSPLT (SEQ ID NO: 44)
   >73C5vK39_2 L-CDR3
      QQYSRYPLT (SEQ ID NO: 45)
   >73C5vK39_7 L-CDR3
      QQYSRYPLT (SEQ ID NO: 45)
   >73C5vK39_15 L-CDR3
      QQYSRYPLT (SEQ ID NO: 45)
H-CDR1 Amino Acid Sequences
   >81B4vH33_49 H-CDR1
      GYSFTSSWIH (SEQ ID NO: 53)
   >81B4vH33_85T H-CDR1
      GYSFTSSWIH (SEQ ID NO: 53)
   >81B4vH33_90 H-CDR1
      GYSFTSSWIH (SEQ ID NO: 53)
   >81B4vH33_93 H-CDR1
      GYSFTSSWIH (SEQ ID NO: 53)
   >81B4vH50_22 H-CDR1
      GYSFTSSWIH (SEQ ID NO: 53)
   >81B4vH50_30 H-CDR1
      GYSFTSSWIH (SEQ ID NO: 53)
   >81B4vH51_13 H-CDR1
      GYSFTSSWIH (SEQ ID NO: 53)
   >81B4vH51_15 H-CDR1
      GYSFTSSWIH (SEQ ID NO: 53)
   >81B4vH52_83 H-CDR1
      GYSFTSSWIH (SEQ ID NO: 53)
   >73C5vH46_4 H-CDR1
      GFSLTDYAVH (SEQ ID NO: 107)
   >73C5vH46_19 H-CDR1
      GFSLTDYAVH (SEQ ID NO: 107)
   >73C5vH46_40 H-CDR1
      GFSLTDYAVH (SEQ ID NO: 107)
   >73C5vH47_65 H-CDR1
      GFSLTDYAVH (SEQ ID NO: 107)
   >73C5vH47_77 H-CDR1
      GFSLTDYAVH (SEQ ID NO: 107)
   >73C5vH58_91 H-CDR1
      GFSLTDYAVH (SEQ ID NO: 107)
H-CDR2 Amino Acid Sequences
   >81B4vH33_49 H-CDR2
      EINPGNVRTNYNENF (SEQ ID NO: 62)
   >81B4vH33_85T H-CDR2
      EINPGNVRTNYNENF (SEQ ID NO: 62)
   >81B4vH33_90 H-CDR2
      EINPGNVRTNYNENF (SEQ ID NO: 62)
   >81B4vH33_93 H-CDR2
      EINPGNVRTNYNENF (SEQ ID NO: 62)
   >81B4vH50_22 H-CDR2
      EILPGVVRTNYNENF (SEQ ID NO: 108)
   >81B4vH50_30 H-CDR2
      EINPGAVRTNYNENF (SEQ ID NO: 109)
   >81B4vH51_13 H-CDR2
      EINPGLVRTNYNENF (SEQ ID NO: 110)
   >81B4vH51_15 H-CDR2
      EINPGAVRTNYNENF (SEQ ID NO: 109)
   >81B4vH52_83 H-CDR2
      EINPGSVRTNYNENF (SEQ ID NO: 111)
   >73C5vH46_4 H-CDR2
      VIWSDGSTDYNAPFKS (SEQ ID NO: 64)
   >73C5vH46_19 H-CDR2
      VIWSDGSTDYNAPFKS (SEQ ID NO: 64)
   >73C5vH46_40 H-CDR2
      VIWSDGSTDYNAPFKS (SEQ ID NO: 64)
   >73C5vH47_65 H-CDR2
      VIWSDGSTDYNAPFKS (SEQ ID NO: 64)
   >73C5vH47_77 H-CDR2
      VIWSDGSTDFNAPFKS (SEQ ID NO: 63)
   >73C5vH58_91 H-CDR2
      VIWSDGSTDYNAPFKS (SEQ ID NO: 64)
H-CDR3 Amino Acid Sequences
   >81B4vH33_49 H-CDR3
      VFYGEPYFPY (SEQ ID NO: 72)
   >81B4vH33_85T H-CDR3
      VFYGEPYFPY (SEQ ID NO: 72)
   >81B4vH33_90 H-CDR3
      VFYGEPYFPY (SEQ ID NO: 72)
   >81B4vH33_93 H-CDR3
      VFYGEPYFPY (SEQ ID NO: 72)
   >81B4vH50_22 H-CDR3
      VFYGEPYFPY (SEQ ID NO: 72)
   >81B4vH50_30 H-CDR3
      VFYGEPYFPY (SEQ ID NO: 72)
   >81B4vH51_13 H-CDR3
      VFYGEPYFPY (SEQ ID NO: 72)
   >81B4vH51_15 H-CDR3
      VFYGEPYFPY (SEQ ID NO: 72)
   >81B4vH52_83 H-CDR3
      VFYGEPYFPY (SEQ ID NO: 72)
   >73C5vH46_4 H-CDR3
      KGGYSGSWFAY (SEQ ID NO: 73)
   >73C5vH46_19 H-CDR3
      KGGYSGSWFAY (SEQ ID NO: 73)
   >73C5vH46_40 H-CDR3
      KGGYSGSWFAY (SEQ ID NO: 73)
   >73C5vH47_65 H-CDR3
      KGGYSGSWFAY (SEQ ID NO: 73)
   >73C5vH47_77 H-CDR3
      KGGYSGSWFAY (SEQ ID NO: 73)
   >73C5vH58_91 H-CDR3
      KGGYSGSWFAY (SEQ ID NO: 73)

In one aspect, a variable region of the present invention is linked to a constant region. For example, a variable region of the present invention is linked to a constant region shown below to form a heavy chain or a light chain of an antibody.

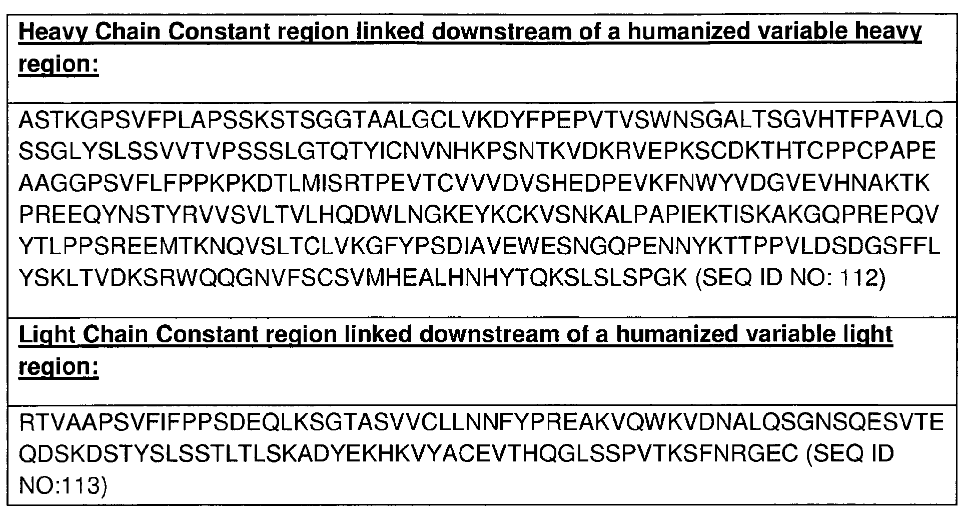

Representative light chain and heavy chain sequences of the present invention are shown below (humanized variable regions derived from antibodies 81B4 and 73C5 linked to constant regions).

### Light Chain Amino Acid Sequences

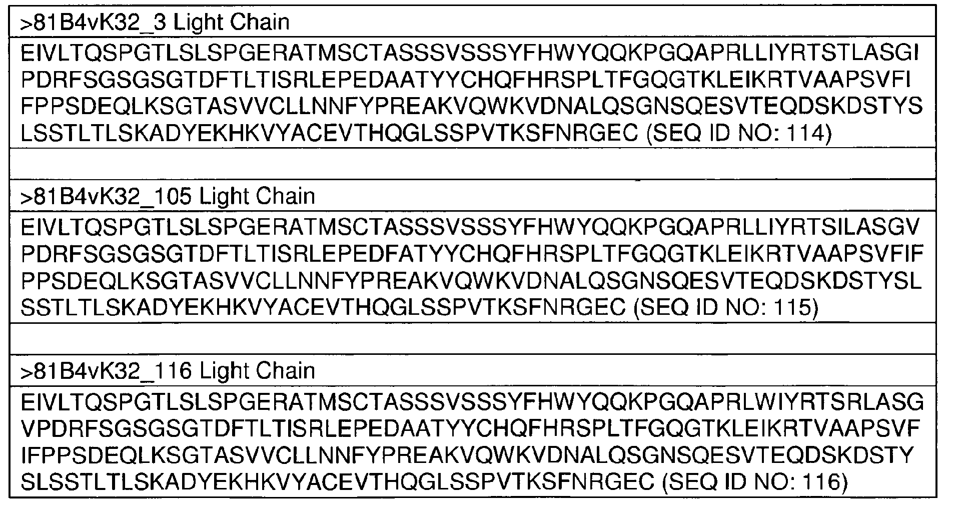

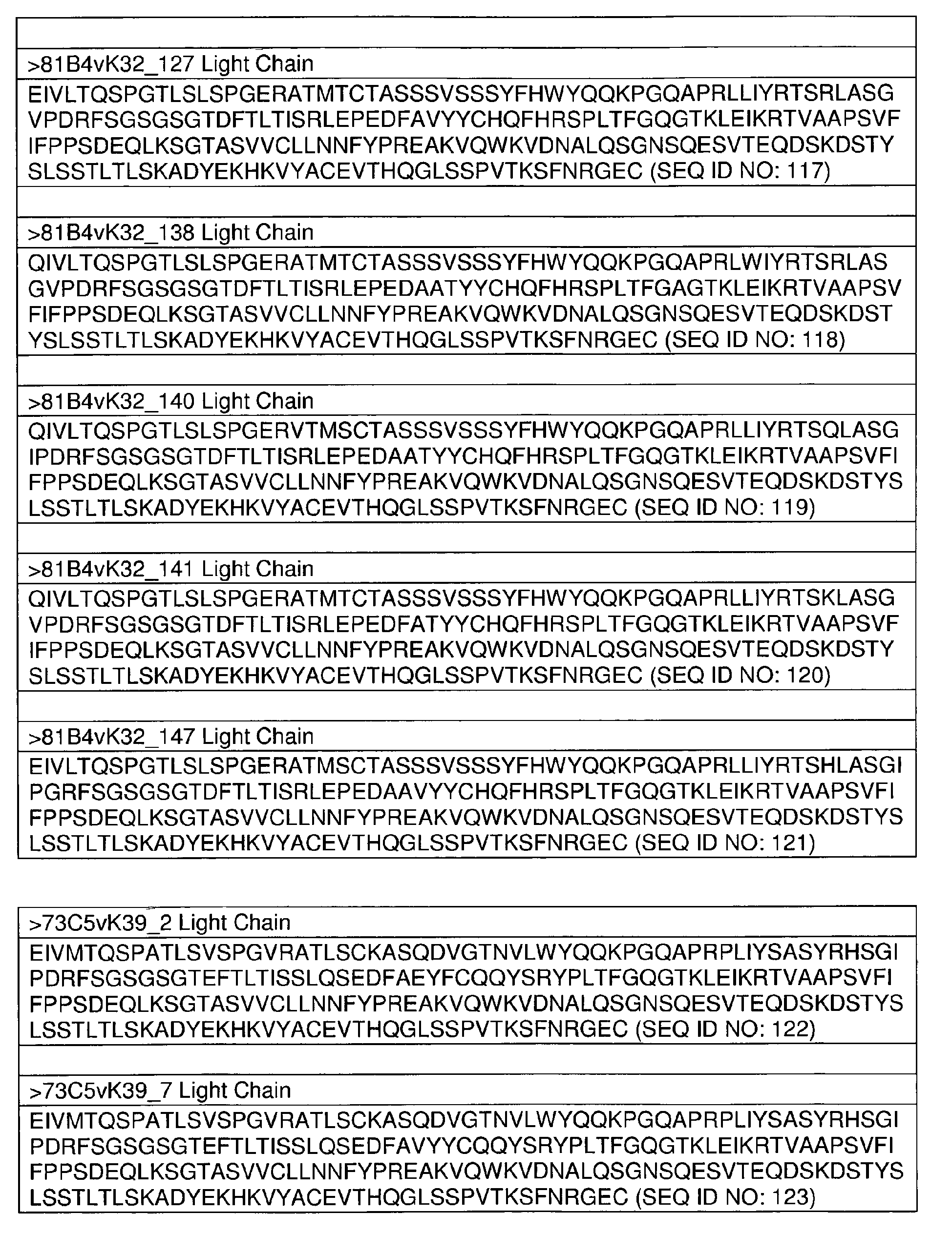

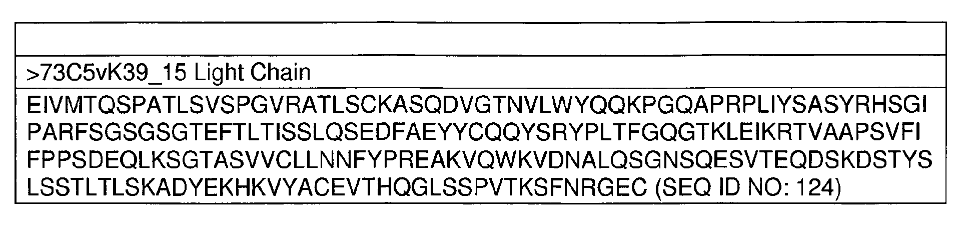

### Heavy Chain Amino Acid Sequences

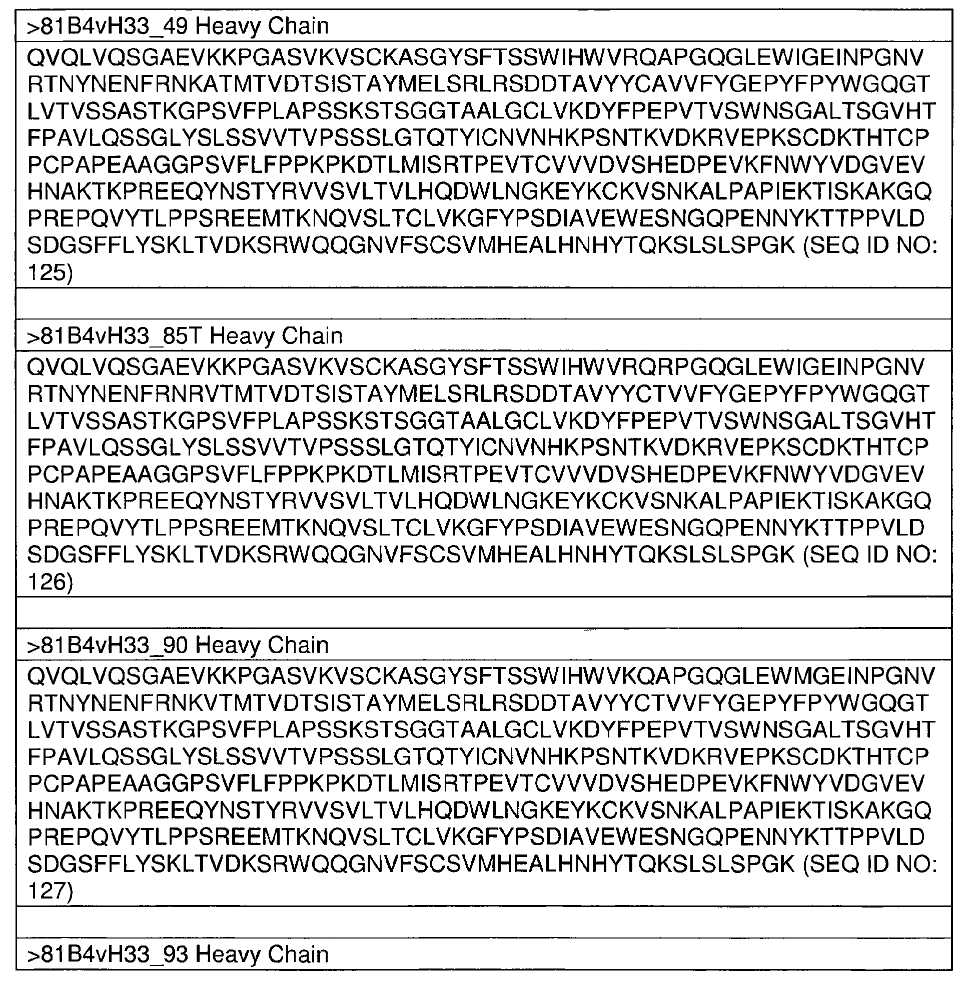

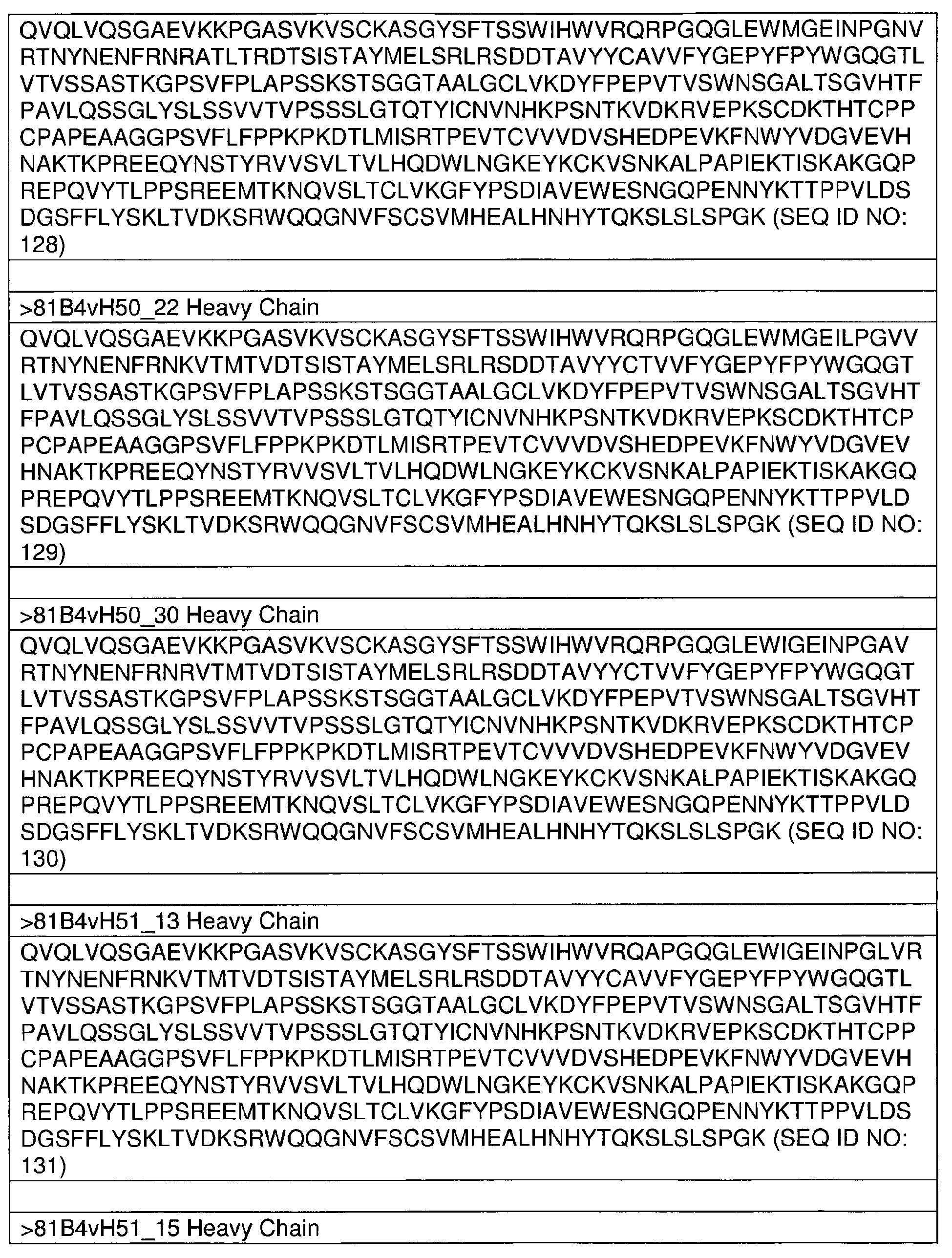

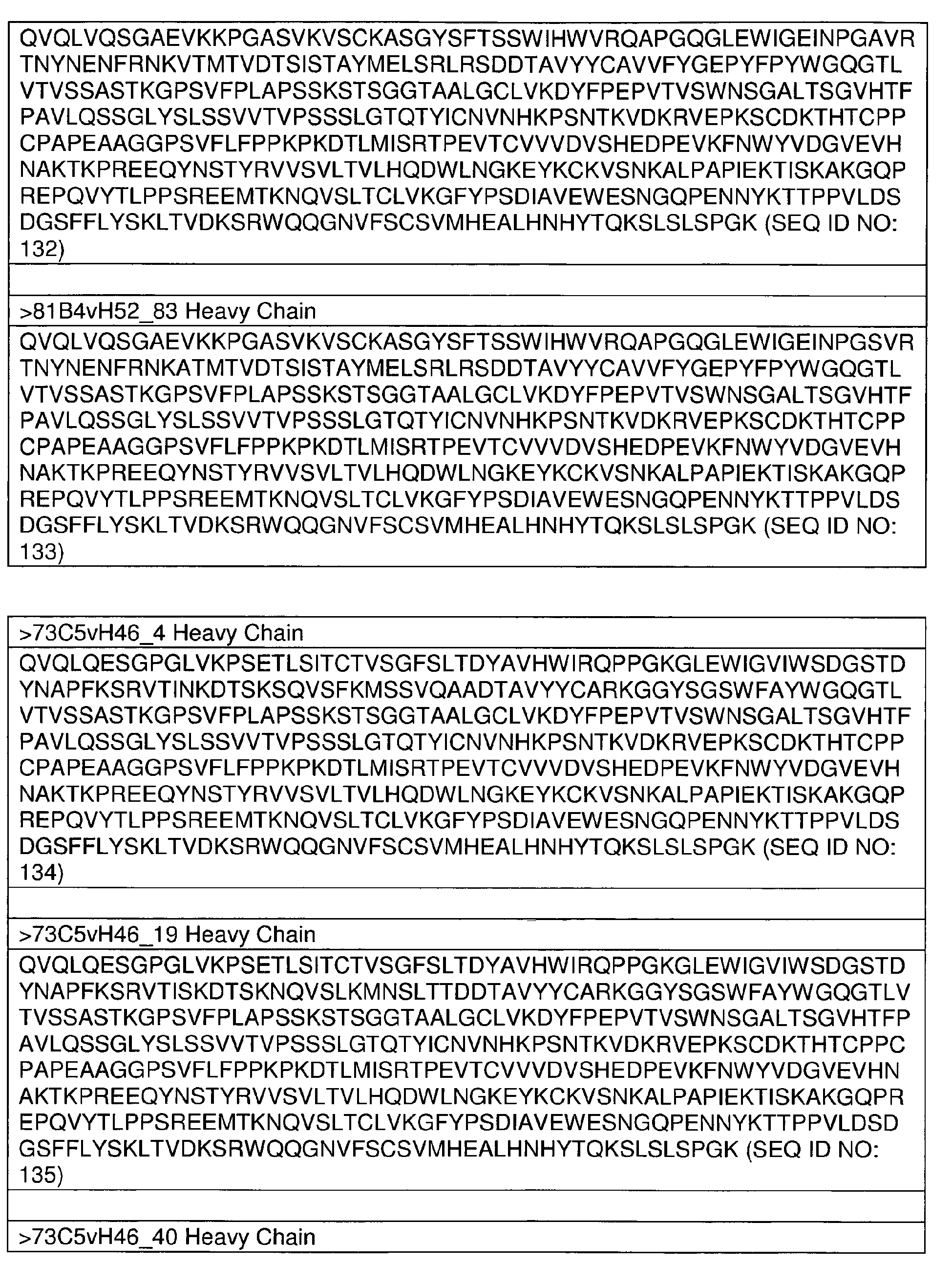

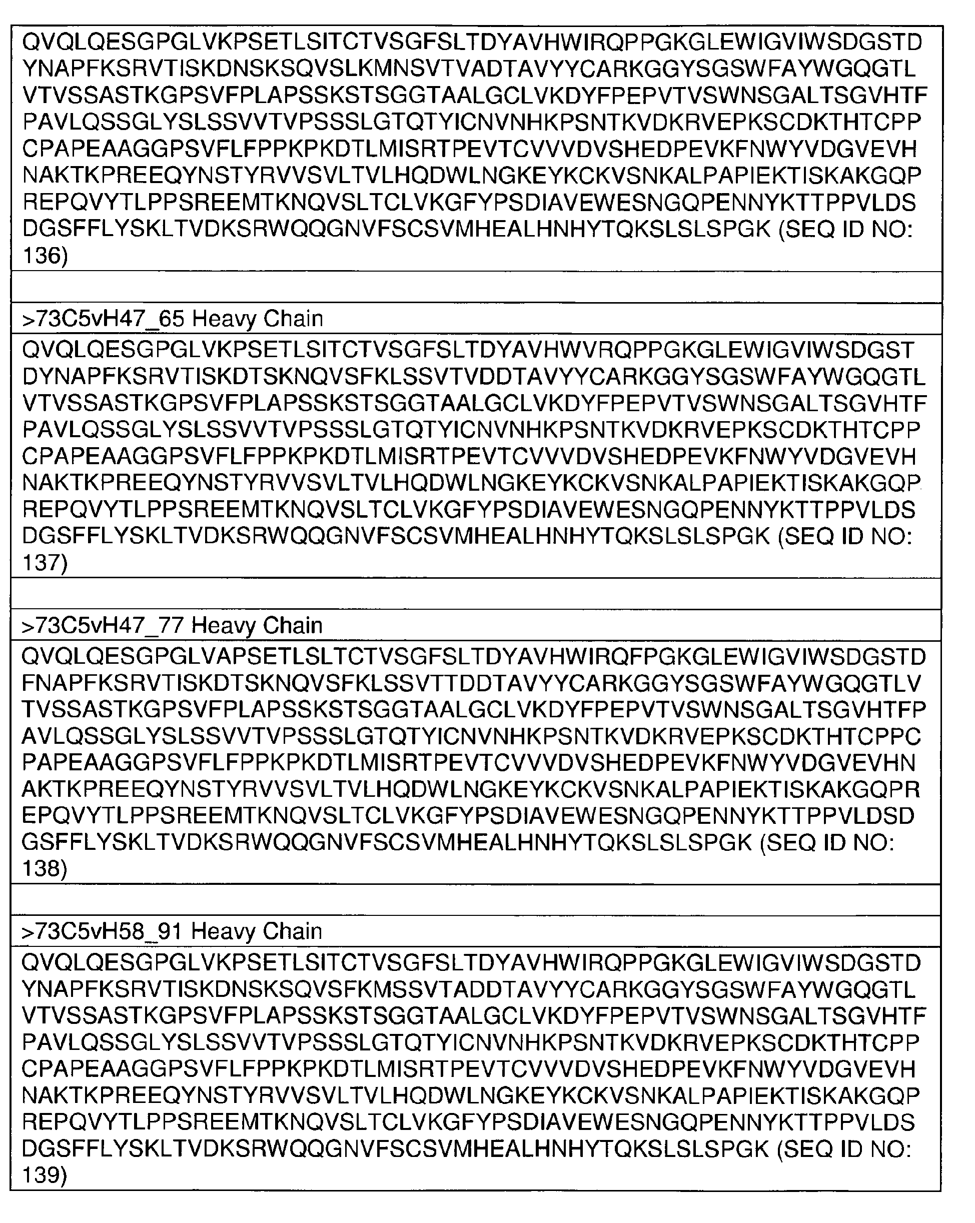

The CDRs listed above are defined using the Chothia numbering system (Al-Lazikani et al., (1997) JMB 273, 927-948).

In one aspect, an antibody of the present invention comprises 3 light chain CDRs and 3 heavy chain CDRs, for example as set forth above.

In one aspect, an antibody of the present invention comprises a light chain and a heavy chain variable region as set forth above. In one aspect, a light chain variable region of the invention is fused to a light chain constant region, for example a kappa or lambda constant region. In one aspect, a heavy chain variable region of the invention is fused to a heavy chain constant region, for example IgA, IgD, IgE, IgG or IgM, in particular, IgG₁, IgG₂, IgG₃ or IgG₄.

The present invention provides an anti-IL-36R antibody comprising a light chain comprising the amino acid sequence of SEQ ID NO: 115; and a heavy chain comprising the amino acid sequence of SEQ ID NO: 125 (Antibody B1).

The present invention provides an anti-IL-36R antibody comprising a light chain comprising the amino acid sequence of SEQ ID NO: 115; and a heavy chain comprising the amino acid sequence of SEQ ID NO: 126 (Antibody B2).

The present invention provides an anti-IL-36R antibody comprising a light chain comprising the amino acid sequence of SEQ ID NO: 115; and a heavy chain comprising the amino acid sequence of SEQ ID NO: 127 (Antibody B3).

The present invention provides an anti-IL-36R antibody comprising a light chain comprising the amino acid sequence of SEQ ID NO: 118; and a heavy chain comprising the amino acid sequence of SEQ ID NO: 125 (Antibody B4).

The present invention provides an anti-IL-36R antibody comprising a light chain comprising the amino acid sequence of SEQ ID NO: 118; and a heavy chain comprising the amino acid sequence of SEQ ID NO: 126 (Antibody B5).

The present invention provides an anti-IL-36R antibody comprising a light chain comprising the amino acid sequence of SEQ ID NO: 118; and a heavy chain comprising the amino acid sequence of SEQ ID NO: 127 Antibody B6).

The present invention provides an anti-IL-36R antibody comprising a light chain comprising the amino acid sequence of SEQ ID NO: 123; and a heavy chain comprising the amino acid sequence of SEQ ID NO: 138 (Antibody C3).

The present invention provides an anti-IL-36R antibody comprising a light chain comprising the amino acid sequence of SEQ ID NO: 123; and a heavy chain comprising the amino acid sequence of SEQ ID NO: 139 (Antibody C2).

The present invention provides an anti-IL-36R antibody comprising a light chain comprising the amino acid sequence of SEQ ID NO: 124; and a heavy chain comprising the amino acid sequence of SEQ ID NO: 138 (Antibody C1)

Representative antibodies of the present invention are shown below.

**Table B.**

| Anti body | **Light Chain Sequences** | **Heavy Chain Sequences** |
|---|---|---|
| B1 | EIVLTQSPGTLSLSPGERATMSCT ASSSVSSSYFHWYQQKPGQAPR LLIYRTSILASGVPDRFSGSGSGT DFTLTISRLEPEDFATYYCHQFHR SPLTFGQGTKLEIKRTVAAPSVFIF PPSDEQLKSGTASVVCLLNNFYP REAKVQWKVDNALQSGNSQESV TEQDSKDSTYSLSSTLTLSKADYE KHKVYACEVTHQGLSSPVTKSFN RGEC (SEQ ID NO: 115) | QVQLVQSGAEVKKPGASVKVSCKASGY SFTSSWIHWVRQAPGQGLEWIGEINPGN VRTNYNENFRNKATMTVDTSISTAYMEL SRLRSDDTAVYYCAVVFYGEPYFPYWG QGTLVTVSSASTKGPSVFPLAPSSKSTS GGTAALGCLVKDYFPEPVTVSWNSGALT SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL |
| | | SPGK (SEQ ID NO: 125) |
| B2 | EIVLTQSPGTLSLSPGERATMSCT ASSSVSSSYFHWYQQKPGQAPR LLIYRTSILASGVPDRFSGSGSGT DFTLTISRLEPEDFATYYCHQFHR SPLTFGQGTKLEIKRTVAAPSVFIF PPSDEQLKSGTASVVCLLNNFYP REAKVQWKVDNALQSGNSQESV TEQDSKDSTYSLSSTLTLSKADYE KHKVYACEVTHQGLSSPVTKSFN RGEC (SEQ ID NO: 115) | QVQLVQSGAEVKKPGASVKVSCKASGY SFTSSWIHWVRQRPGQGLEWIGEINPG NVRTNYNENFRNRVTMTVDTSISTAYME LSRLRSDDTAVYYCTVVFYGEPYFPYWG QGTLVTVSSASTKGPSVFPLAPSSKSTS GGTAALGCLVKDYFPEPVTVSWNSGALT SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 126) |
| B3 | EIVLTQSPGTLSLSPGERATMSCT ASSSVSSSYFHWYQQKPGQAPR LLIYRTSILASGVPDRFSGSGSGT DFTLTISRLEPEDFATYYCHQFHR SPLTFGQGTKLEIKRTVAAPSVFIF PPSDEQLKSGTASVVCLLNNFYP REAKVQWKVDNALQSGNSQESV TEQDSKDSTYSLSSTLTLSKADYE KHKVYACEVTHQGLSSPVTKSFN RGEC (SEQ ID NO: 115) | QVQLVQSGAEVKKPGASVKVSCKASGY SFTSSWIHWVKQAPGQGLEWMGEINPG NVRTNYNENFRNKVTMTVDTSISTAYME LSRLRSDDTAVYYCTVVFYGEPYFPYWG QGTLVTVSSASTKGPSVFPLAPSSKSTS GGTAALGCLVKDYFPEPVTVSWNSGALT SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 127) |
| B4 | QIVLTQSPGTLSLSPGERATMTCT ASSSVSSSYFHWYQQKPGQAPR LWIYRTSRLASGVPDRFSGSGSG TDFTLTISRLEPEDAATYYCHQFH RSPLTFGAGTKLEIKRTVAAPSVFI FPPSDEQLKSGTASVVCLLNNFYP | QVQLVQSGAEVKKPGASVKVSCKASGY SFTSSWIHWVRQAPGQGLEWIGEINPGN VRTNYNENFRNKATMTVDTSISTAYMEL SRLRSDDTAVYYCAVVFYGEPYFPYWG QGTLVTVSSASTKGPSVFPLAPSSKSTS GGTAALGCLVKDYFPEPVTVSWNSGALT |
| | REAKVQWKVDNALQSGNSQESV TEQDSKDSTYSLSSTLTLSKADYE KHKVYACEVTHQGLSSPVTKSFN RGEC (SEQ ID NO: 118) | SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 125) |
| B5 | QIVLTQSPGTLSLSPGERATMTCT ASSSVSSSYFHWYQQKPGQAPR LWIYRTSRLASGVPDRFSGSGSG TDFTLTISRLEPEDAATYYCHQFH RSPLTFGAGTKLEIKRTVAAPSVFI FPPSDEQLKSGTASVVCLLNNFYP REAKVQWKVDNALQSGNSQESV TEQDSKDSTYSLSSTLTLSKADYE KHKVYACEVTHQGLSSPVTKSFN RGEC (SEQ ID NO: 118) | QVQLVQSGAEVKKPGASVKVSCKASGY SFTSSWIHWVRQRPGQGLEWIGEINPG NVRTNYNENFRNRVTMTVDTSISTAYME LSRLRSDDTAVYYCTVVFYGEPYFPYWG QGTLVTVSSASTKGPSVFPLAPSSKSTS GGTAALGCLVKDYFPEPVTVSWNSGALT SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 126) |
| B6 | QIVLTQSPGTLSLSPGERATMTCT ASSSVSSSYFHWYQQKPGQAPR LWIYRTSRLASGVPDRFSGSGSG TDFTLTISRLEPEDAATYYCHQFH RSPLTFGAGTKLEIKRTVAAPSVFI FPPSDEQLKSGTASVVCLLNNFYP REAKVQWKVDNALQSGNSQESV TEQDSKDSTYSLSSTLTLSKADYE KHKVYACEVTHQGLSSPVTKSFN RGEC (SEQ ID NO: 118) | QVQLVQSGAEVKKPGASVKVSCKASGY SFTSSWIHWVKQAPGQGLEWMGEINPG NVRTNYNENFRNKVTMTVDTSISTAYME LSRLRSDDTAVYYCTVVFYGEPYFPYWG QGTLVTVSSASTKGPSVFPLAPSSKSTS GGTAALGCLVKDYFPEPVTVSWNSGALT SGVHTFPAVLQSSGLYSLSSVVTVPSSS LGTQTYICNVNHKPSNTKVDKRVEPKSC DKTHTCPPCPAPEAAGGPSVFLFPPKPK DTLMISRTPEVTCVVVDVSHEDPEVKFN WYVDGVEVHNAKTKPREEQYNSTYRVV SVLTVLHQDWLNGKEYKCKVSNKALPAP IEKTISKAKGQPREPQVYTLPPSREEMTK NQVSLTCLVKGFYPSDIAVEWESNGQPE NNYKTTPPVLDSDGSFFLYSKLTVDKSR |
| | | WQQGNVFSCSVMHEALHNHYTQKSLSL SPGK (SEQ ID NO: 127) |

**Table C**

| Anti body | **Liqht Chain Sequences** | **Heavy Chain Sequences** |
|---|---|---|
| C1 | EIVMTQSPATLSVSPGVRATLSCK ASQDVGTNVLWYQQKPGQAPRP LIYSASYRHSGIPARFSGSGSGTE FTLTISSLQSEDFAEYYCQQYSRY PLTFGQGTKLEIKRTVAAPSVFIFP PSDEQLKSGTASVVCLLNNFYPR EAKVQWKVDNALQSGNSQESVT EQDSKDSTYSLSSTLTLSKADYEK HKVYACEVTHQGLSSPVTKSFNR GEC (SEQ ID NO: 124) | QVQLQESGPGLVAPSETLSLTCTVSGFS LTDYAVHWIRQFPGKGLEWIGVIWSDGS TDFNAPFKSRVTISKDTSKNQVSFKLSSV TTDDTAVYYCARKGGYSGSWFAYWGQ GTLVTVSSASTKGPSVFPLAPSSKSTSG GTAALGCLVKDYFPEPVTVSWNSGALTS GVHTFPAVLQSSGLYSLSSVVTVPSSSL GTQTYICNVNHKPSNTKVDKRVEPKSCD KTHTCPPCPAPEAAGGPSVFLFPPKPKD TLMISRTPEVTCVVVDVSHEDPEVKFNW YVDGVEVHNAKTKPREEQYNSTYRVVS VLTVLHQDWLNGKEYKCKVSNKALPAPI EKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 138) |
| C2 | EIVMTQSPATLSVSPGVRATLSCK ASQDVGTNVLWYQQKPGQAPRP LIYSASYRHSGIPDRFSGSGSGTE FTLTISSLQSEDFAVYYCQQYSRY PLTFGQGTKLEIKRTVAAPSVFIFP PSDEQLKSGTASVVCLLNNFYPR EAKVQWKVDNALQSGNSQESVT EQDSKDSTYSLSSTLTLSKADYEK HKVYACEVTHQGLSSPVTKSFNR GEC (SEQ ID NO: 123) | QVQLQESGPGLVKPSETLSITCTVSGFSL TDYAVHWIRQPPGKGLEWIGVIWSDGST DYNAPFKSRVTISKDNSKSQVSFKMSSV TADDTAVYYCARKGGYSGSWFAYWGQ GTLVTVSSASTKGPSVFPLAPSSKSTSG GTAALGCLVKDYFPEPVTVSWNSGALTS GVHTFPAVLQSSGLYSLSSVVTVPSSSL GTQTYICNVNHKPSNTKVDKRVEPKSCD KTHTCPPCPAPEAAGGPSVFLFPPKPKD TLMISRTPEVTCVVVDVSHEDPEVKFNW YVDGVEVHNAKTKPREEQYNSTYRVVS VLTVLHQDWLNGKEYKCKVSNKALPAPI EKTISKAKGQPREPQVYTLPPSREEMTK NQVSLTCLVKGFYPSDIAVEWESNGQPE |
| | | NNYKTTPPVLDSDGSFFLYSKLTVDKSR WQQGNVFSCSVMHEALHNHYTQKSLSL SPGK (SEQ ID NO: 139) |
| C3 | EIVMTQSPATLSVSPGVRATLSCK ASQDVGTNVLWYQQKPGQAPRP LIYSASYRHSGIPDRFSGSGSGTE FTLTISSLQSEDFAVYYCQQYSRY PLTFGQGTKLEIKRTVAAPSVFIFP PSDEQLKSGTASVVCLLNNFYPR EAKVQWKVDNALQSGNSQESVT EQDSKDSTYSLSSTLTLSKADYEK HKVYACEVTHQGLSSPVTKSFNR GEC (SEQ ID NO: 123) | QVQLQESGPGLVAPSETLSLTCTVSGFS LTDYAVHWIRQFPGKGLEWIGVIWSDGS TDFNAPFKSRVTISKDTSKNQVSFKLSSV TTDDTAVYYCARKGGYSGSWFAYWGQ GTLVTVSSASTKGPSVFPLAPSSKSTSG GTAALGCLVKDYFPEPVTVSWNSGALTS GVHTFPAVLQSSGLYSLSSVVTVPSSSL GTQTYICNVNHKPSNTKVDKRVEPKSCD KTHTCPPCPAPEAAGGPSVFLFPPKPKD TLMISRTPEVTCVVVDVSHEDPEVKFNW YVDGVEVHNAKTKPREEQYNSTYRVVS VLTVLHQDWLNGKEYKCKVSNKALPAPI EKTISKAKGQPREPQVYTLPPSREEMTK NQVSLTCLVKGFYPSDIAVEWESNGQPE NNYKTTPPVLDSDGSFFLYSKLTVDKSR WQQGNVFSCSVMHEALHNHYTQKSLSL SPGK (SEQ ID NO: 138) |

The antibodies of the present invention are useful in methods for the treatment of various diseases or disorders, for example immunological, inflammatory, autoimmune diseases and respiratory diseases in humans. For example, the antibodies of the present invention are useful in methods for the treatment of psoriasis, rheumatoid arthritis, inflammatory bowel disease or psoriatic arthritis. For example, the antibodies of the present invention are useful in methods for the treatment of chronic obstructive pulmonary disorder (COPD) or asthma. For example, the antibodies of the present invention are useful in methods for the treatment of scleroderma, palmoplantar pustulosis, generalized pustular psoriasis, diabetic nephropathy, lupus nephritis, scleroderma, ankylosing spondylitis, deficiency in the IL-36 receptor antagonist autoimmune disease (DITRA), deficiency in the IL-1 receptor antagonist autoimmune disease (DIRA) or cryopyrin associated periodic syndromes (CAPS).

In some aspects, the humanized antibody displays blocking activity, whereby it decreases the binding of IL-36 ligand to IL-36 receptor by at least 45%, by at least 50%, by at least 55%, by at least 60%, by at least 65%, by at least 70%, by at least 75%, by at least 80%, by at least 85%, by at least 90%, or by at least 95%. The ability of an antibody to block binding of IL-36 ligand to the IL-36 receptor can be measured using competitive binding assays known in the art. Alternatively, the blocking activity of an antibody can be measured by assessing the biological effects of IL-36, such as the production of IL-8, IL-6, and GM-CSF to determine if signaling mediated by the IL-36 receptor is inhibited.

In a further aspect, the present invention provides a humanized anti-IL-36R antibody having favorable biophysical properties. In one aspect, a humanized anti-IL-36R antibody of the present invention is present in at least 90% monomer form, or in at least 92% monomer form, or in at least 95% monomer form in a buffer. In a further aspect, a humanized anti-IL-36R antibody of the present invention remains in at least 90% monomer form, or in at least 92% monomer form, or in at least 95% monomer form in a buffer for one month or for four months.

In one aspect, a humanized antibody of the present invention is Antibody B1, Antibody B2, Antibody B3, Antibody B4, Antibody B5, Antibody B6, Antibody C1, Antibody C2, or Antibody C3. Accordingly, in one embodiment, a humanized antibody of the present invention comprises the light chain sequence of SEQ ID NO:115 and the heavy chain sequence of SEQ ID NO:125 (Antibody B1). In another embodiment, a humanized antibody of the present invention comprises the light chain sequence of SEQ ID NO:115 and the heavy chain sequence of SEQ ID NO:126 (Antibody B2). In another embodiment, a humanized antibody of the present invention comprises the light chain sequence of SEQ ID NO:115 and the heavy chain sequence of SEQ ID NO:127 (Antibody B3). In another embodiment, a humanized antibody of the present invention comprises the light chain sequence of SEQ ID NO:118 and the heavy chain sequence of SEQ ID NO:125 (Antibody B4). In another embodiment, a humanized antibody of the present invention comprises the light chain sequence of SEQ ID NO:118 and the heavy chain sequence of SEQ ID NO:126 (Antibody B5). In another embodiment, a humanized antibody of the present invention comprises the light chain sequence of SEQ ID NO:118 and the heavy chain sequence of SEQ ID NO:127 (Antibody B6). In another embodiment, a humanized antibody of the present invention comprises the light chain sequence of SEQ ID NO:124 and the heavy chain sequence of SEQ ID NO:138 (Antibody C1). In another embodiment, a humanized antibody of the present invention comprises the light chain sequence of SEQ ID NO:123 and the heavy chain sequence of SEQ ID NO:139 (Antibody C2). In another embodiment, a humanized antibody of the present invention comprises the light chain sequence of SEQ ID NO:123 and the heavy chain sequence of SEQ ID NO:138 (Antibody C3).

In a further embodiment, a humanized antibody of the present invention consists of the light chain sequence of SEQ ID NO:115 and the heavy chain sequence of SEQ ID NO:125 (Antibody B1). In another embodiment, a humanized antibody of the present invention consists of the light chain sequence of SEQ ID NO:115 and the heavy chain sequence of SEQ ID NO:126 (Antibody B2). In another embodiment, a humanized antibody of the present invention consists of the light chain sequence of SEQ ID NO:115 and the heavy chain sequence of SEQ ID NO:127 (Antibody B3). In another embodiment, a humanized antibody of the present invention consists of the light chain sequence of SEQ ID NO:118 and the heavy chain sequence of SEQ ID NO:125 (Antibody B4). In another embodiment, a humanized antibody of the present invention consists of the light chain sequence of SEQ ID NO:118 and the heavy chain sequence of SEQ ID NO:126 (Antibody B5). In another embodiment, a humanized antibody of the present invention consists of the light chain sequence of SEQ ID NO:118 and the heavy chain sequence of SEQ ID NO:127 (Antibody B6). In another embodiment, a humanized antibody of the present invention consists of the light chain sequence of SEQ ID NO:124 and the heavy chain sequence of SEQ ID NO:138 (Antibody C1). In another embodiment, a humanized antibody of the present invention consists of the light chain sequence of SEQ ID NO:123 and the heavy chain sequence of SEQ ID NO:139 (Antibody C2). In another embodiment, a humanized antibody of the present invention consists of the light chain sequence of SEQ ID NO:123 and the heavy chain sequence of SEQ ID NO:138 (Antibody C3).

In some embodiments, the humanized anti-IL-36R antibodies, including antigen-binding fragments thereof, such as heavy and light chain variable regions, comprise an amino acid sequence of the residues derived from Antibody B1, Antibody B2, Antibody B3, Antibody B4, Antibody B5, Antibody B6, Antibody C1, Antibody C2, or Antibody C3.

In a further embodiment, the present invention provides an anti-IL-36R antibody or antigen-binding fragment thereof that competitively binds to human IL-36R with an antibody of the present invention, for example Antibody B1, Antibody B2, Antibody B3, Antibody B4, Antibody B5, Antibody B6, Antibody C1, Antibody C2 or Antibody C3 described herein. The ability of an antibody or antigen-binding fragment to competitively bind to IL-36R can be measured using competitive binding assays known in the art.

The humanized anti-IL-36R antibodies optionally include specific amino acid substitutions in the consensus or germline framework regions. The specific substitution of amino acid residues in these framework positions can improve various aspects of antibody performance including binding affinity and/or stability, over that demonstrated in humanized antibodies formed by "direct swap" of CDRs or HVLs into the human germline framework regions.

In some embodiments, the present invention describes other monoclonal antibodies with a light chain variable region having the amino acid sequence set forth in any one of SEQ ID NO:1-10. In some embodiments, the present invention describes other monoclonal antibodies with a heavy chain variable region having the amino acid sequence set forth in any one of SEQ ID NO:11-20. Placing such CDRs into FRs of the human consensus heavy and light chain variable domains will yield useful humanized antibodies of the present invention.

In particular, the present invention provides monoclonal antibodies with the combinations of light chain variable and heavy chain variable regions of SEQ ID NO:1/11, 2/12, 3/13, 4/14, 5/15, 6/16, 7/17, 8/18, 9/19, 10/20. Such variable regions can be combined with human constant regions.

In some embodiments, the present invention describes other humanized antibodies with light chain variable region sequences having the amino acid sequence set forth in any one of SEQ ID NO:76-86. In some embodiments, the present invention describes other humanized antibodies with heavy chain variable region sequences having the amino acid sequence set forth in any one of SEQ ID NO:87-101. In particular, the present invention provides monoclonal antibodies with the combinations of light chain variable and heavy chain variable regions of SEQ ID NO: 77/89, 80/88, 80/89, 77/87, 77/88, 80/87, 86/100, 85/101, 85/100. Such variable regions can be combined with human constant regions.

In a further embodiment, the present invention relates to an anti-IL-36R antibody or antigen-binding fragment thereof comprising a humanized light chain variable domain comprising the CDRs of SEQ ID NO:77 and framework regions having an amino acid sequence at least 90% identical, at least 93% identical or at least 95% identical to the amino acid sequence of the framework regions of the variable domain light chain amino acid sequence of SEQ ID NO:77 and a humanized heavy chain variable domain comprising the CDRs of SEQ ID NO:89 and framework regions having an amino acid sequence at least 90% identical, at least 93% identical or at least 95% identical to the amino acid sequence of the framework regions of the variable domain heavy chain amino acid sequence of SEQ ID NO:89. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:80 and framework regions having an amino acid sequence at least 90% identical, at least 93% identical or at least 95% identical to the amino acid sequence of the framework regions of the variable domain light chain amino acid sequence of SEQ ID NO:80 and a humanized heavy chain variable domain comprising the CDRs of SEQ ID NO:88 and framework regions having an amino acid sequence at least 90% identical, at least 93% identical or at least 95% identical to the amino acid sequence of the framework regions of the variable domain heavy chain amino acid sequence of SEQ ID NO:88. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:80 and framework regions having an amino acid sequence at least 90% identical, at least 93% identical or at least 95% identical to the amino acid sequence of the framework regions of the variable domain light chain amino acid sequence of SEQ ID NO:80 and a humanized heavy chain variable domain comprising the CDRs of SEQ ID NO:89 and framework regions having an amino acid sequence at least 90% identical, at least 93% identical or at least 95% identical to the amino acid sequence of the framework regions of the variable domain heavy chain amino acid sequence of SEQ ID NO:89. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:77 and framework regions having an amino acid sequence at least 90% identical, at least 93% identical or at least 95% identical to the amino acid sequence of the framework regions of the variable domain light chain amino acid sequence of SEQ ID NO:77 and a humanized heavy chain variable domain comprising the CDRs of SEQ ID NO:87 and framework regions having an amino acid sequence at least 90% identical, at least 93% identical or at least 95% identical to the amino acid sequence of the framework regions of the variable domain heavy chain amino acid sequence of SEQ ID NO:87. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:77 and framework regions having an amino acid sequence at least 90% identical, at least 93% identical or at least 95% identical to the amino acid sequence of the framework regions of the variable domain light chain amino acid sequence of SEQ ID NO:77 and a humanized heavy chain variable domain comprising the CDRs of SEQ ID NO:88 and framework regions having an amino acid sequence at least 90% identical, at least 93% identical or at least 95% identical to the amino acid sequence of the framework regions of the variable domain heavy chain amino acid sequence of SEQ ID NO:88. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:80 and framework regions having an amino acid sequence at least 90% identical, at least 93% identical or at least 95% identical to the amino acid sequence of the framework regions of the variable domain light chain amino acid sequence of SEQ ID NO:80 and a humanized heavy chain variable domain comprising the CDRs of SEQ ID NO:87 and framework regions having an amino acid sequence at least 90% identical, at least 93% identical or at least 95% identical to the amino acid sequence of the framework regions of the variable domain heavy chain amino acid sequence of SEQ ID NO:87. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:86 and framework regions having an amino acid sequence at least 90% identical, at least 93% identical or at least 95% identical to the amino acid sequence of the framework regions of the variable domain light chain amino acid sequence of SEQ ID NO:86 and a humanized heavy chain variable domain comprising the CDRs of SEQ ID NO:100 and framework regions having an amino acid sequence at least 90% identical, at least 93% identical or at least 95% identical to the amino acid sequence of the framework regions of the variable domain heavy chain amino acid sequence of SEQ ID NO:100. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:85 and framework regions having an amino acid sequence at least 90% identical, at least 93% identical or at least 95% identical to the amino acid sequence of the framework regions of the variable domain light chain amino acid sequence of SEQ ID NO:85 and a humanized heavy chain variable domain comprising the CDRs of SEQ ID NO:101 and framework regions having an amino acid sequence at least 90% identical, at least 93% identical or at least 95% identical to the amino acid sequence of the framework regions of the variable domain heavy chain amino acid sequence of SEQ ID NO:101. In one embodiment, the anti-IL-36R antibody is a humanized monoclonal antibody.

In a further embodiment, the present invention relates to an anti-IL-36R antibody or antigen-binding fragment thereof comprising a humanized light chain variable domain comprising the CDRs of SEQ ID NO:85 and framework regions having an amino acid sequence at least 90% identical, at least 93% identical or at least 95% identical to the amino acid sequence of the framework regions of the variable domain light chain amino acid sequence of SEQ ID NO:85 and a humanized heavy chain variable domain comprising the CDRs of SEQ ID NO:100 and framework regions having an amino acid sequence at least 90% identical, at least 93% identical or at least 95% identical to the amino acid sequence of the framework regions of the variable domain heavy chain amino acid sequence of SEQ ID NO:100. In one embodiment, the anti-IL-36R antibody is a humanized monoclonal antibody.

In some specific embodiments, the humanized anti-IL-36R antibodies disclosed herein comprise at least a heavy or a light chain variable domain comprising the CDRs or HVLs of the murine monoclonal antibodies or humanized antibodies as disclosed herein and the FRs of the human germline heavy and light chain variable domains.

In one further aspect, the present invention provides an anti-IL-36R antibody or antigen-binding fragment thereof comprising a light chain CDR1 (L-CDR1) sequence of any one of SEQ ID NO:21-29; a light chain CDR2 (L-CDR2) sequence of any one of SEQ ID NO:30-38; a light chain CDR3 (L-CDR3) sequence of any one of SEQ ID NO:39-47; a heavy chain CDR1 (H-CDR1) sequence of any one of SEQ ID NO:48-56; a heavy chain CDR2 (H-CDR2) sequence of any one of SEQ ID NO:57-66; and a heavy chain CDR3 (H-CDR3) sequence of any one of SEQ ID NO:67-75. In one aspect, the anti-IL-36R antibody or antigen-binding fragment thereof comprises a light chain variable region comprising a L-CDR1 listed above, a L-CDR2 listed above and a L-CDR3 listed above, and a heavy chain variable region comprising a H-CDR1 listed above, a H-CDR2 listed above and a H-CDR3 listed above.

In a further aspect, the present invention provides an anti-IL-36R antibody or antigen-binding fragment thereof comprising:
a) a L-CDR1, a L-CDR2, a L-CDR3, a H-CDR1, a H-CDR2 and a H-CDR3 sequence of SEQ ID NO:21, 30, 39, 48, 57 and 67, respectively; or
b) a L-CDR1, a L-CDR2, a L-CDR3, a H-CDR1, a H-CDR2 and a H-CDR3 sequence of SEQ ID NO:22, 31, 40, 49, 58 and 68, respectively; or
c) a L-CDR1, a L-CDR2, a L-CDR3, a H-CDR1, a H-CDR2 and a H-CDR3 sequence of SEQ ID NO:23, 32, 41, 50, 59 and 69, respectively; or
d) a L-CDR1, a L-CDR2, a L-CDR3, a H-CDR1, a H-CDR2 and a H-CDR3 sequence of SEQ ID NO:24, 33, 42, 51, 60 and 70, respectively; or
e) a L-CDR1, a L-CDR2, a L-CDR3, a H-CDR1, a H-CDR2 and a H-CDR3 sequence of SEQ ID NO:25, 34, 43, 52, 61 and 71, respectively; or
f) a L-CDR1, a L-CDR2, a L-CDR3, a H-CDR1, a H-CDR2 and a H-CDR3 sequence of SEQ ID NO:26, 35, 44, 53, 62 and 72, respectively; or
g) a L-CDR1, a L-CDR2, a L-CDR3, a H-CDR1, a H-CDR2 and a H-CDR3 sequence of SEQ ID NO:27, 36, 45, 54, 63 and 73, respectively; or
h) a L-CDR1, a L-CDR2, a L-CDR3, a H-CDR1, a H-CDR2 and a H-CDR3 sequence of SEQ ID NO:27, 36, 45, 54, 64 and 74, respectively; or
i) a L-CDR1, a L-CDR2, a L-CDR3, a H-CDR1, a H-CDR2 and a H-CDR3 sequence of SEQ ID NO:27, 36, 45, 54, 64 and 73, respectively; or
j) a L-CDR1, a L-CDR2, a L-CDR3, a H-CDR1, a H-CDR2 and a H-CDR3 sequence of SEQ ID NO:28, 37, 46, 55, 65 and 74, respectively; or
k) a L-CDR1, a L-CDR2, a L-CDR3, a H-CDR1, a H-CDR2 and a H-CDR3 sequence of SEQ ID NO:29, 38, 47, 56, 66 and 75, respectively.

In a further aspect, the present invention provides an anti-IL-36R antibody or antigen-binding fragment thereof comprising:
a) a L-CDR1, a L-CDR2, a L-CDR3, a H-CDR1, a H-CDR2 and a H-CDR3 sequence of SEQ ID NO:26, 103, 44, 53, 62 and 72, respectively; or
b) a L-CDR1, a L-CDR2, a L-CDR3, a H-CDR1, a H-CDR2 and a H-CDR3 sequence of SEQ ID NO:26, 104, 44, 53, 62 and 72, respectively; or
c) a L-CDR1, a L-CDR2, a L-CDR3, a H-CDR1, a H-CDR2 and a H-CDR3 sequence of SEQ ID NO:27, 36, 45, 107, 63 and 73, respectively; or
d) a L-CDR1, a L-CDR2, a L-CDR3, a H-CDR1, a H-CDR2 and a H-CDR3 sequence of SEQ ID NO:27, 36, 45, 107, 64 or 73, respectively.

In one aspect, the anti-IL-36R antibody or antigen-binding fragment thereof comprises a light chain variable region comprising a L-CDR1, L-CDR2 and L-CDR3 combination listed above, and a heavy chain variable region comprising a H-CDR1, H-CDR2 and H-CDR3 combination listed above.

In specific embodiments, it is contemplated that chimeric antibodies with switched CDR regions (i.e., for example switching one or two CDRs of one of the mouse antibodies or humanized antibody derived therefrom with the analogous CDR from another mouse antibody or humanized antibody derived therefrom) between these exemplary immunoglobulins may yield useful antibodies.

In certain embodiments, the humanized anti-IL-36R antibody is an antibody fragment. Various antibody fragments have been generally discussed above and there are techniques that have been developed for the production of antibody fragments. Fragments can be derived via proteolytic digestion of intact antibodies (see, e.g., Morimoto et al., 1992, Journal of Biochemical and Biophysical Methods 24:107-117; and Brennan et al., 1985, Science 229:81). Alternatively, the fragments can be produced directly in recombinant host cells. For example, Fab'-SH fragments can be directly recovered from E. coli and chemically coupled to form F(ab')₂ fragments (see, e.g., Carter et al., 1992, Bio/Technology 10:163-167). By another approach, F(ab')₂ fragments can be isolated directly from recombinant host cell culture. Other techniques for the production of antibody fragments will be apparent to the skilled practitioner. Accordingly, in one aspect, the present invention provides antibody fragments comprising the CDRs described herein, in particular one of the combinations of L-CDR1, L-CDR2, L-CDR3, H-CDR1, H-CDR2 and H-CDR3 described herein. In a further aspect, the present invention provides antibody fragments comprising the variable regions described herein, for example one of the combinations of light chain variable regions and heavy chain variable regions described herein.

Certain embodiments include an F(ab')₂ fragment of a humanized anti-IL-36R antibody comprise a light chain sequence of any of SEQ ID NO: 115 or 118 in combination with a heavy chain sequence of SEQ ID NO: 125, 126 or 127. Such embodiments can include an intact antibody comprising such an F(ab')₂.

Certain embodiments include an F(ab')₂ fragment of a humanized anti-IL-36R antibody comprise a light chain sequence of any of SEQ ID NO: 123 or 124 in combination with a heavy chain sequence of SEQ ID NO: 138 or 139. Such embodiments can include an intact antibody comprising such an F(ab')₂.

In some embodiments, the antibody or antibody fragment includes a constant region that mediates effector function. The constant region can provide antibody-dependent cellular cytotoxicity (ADCC), antibody-dependent cellular phagocytosis (ADCP) and/or complement-dependent cytotoxicity (CDC) responses against an IL-36R expressing target cell. The effector domain(s) can be, for example, an Fc region of an Ig molecule.

The effector domain of an antibody can be from any suitable vertebrate animal species and isotypes. The isotypes from different animal species differ in the abilities to mediate effector functions. For example, the ability of human immunoglobulin to mediate CDC and ADCC/ADCP is generally in the order of IgM≈IgG₁≈IgG₃>IgG₂>IgG₄ and IgG₁≈IgG₃>IgG₂/IgM/IgG₄, respectively. Murine immunoglobulins mediate CDC and ADCC/ADCP generally in the order of murine IgM≈IgG₃>>IgG_{2b}>IgG₂ₐ>>IgG₁ and IgG_{2b}>IgG₂ₐ>IgG₁>>IgG₃, respectively. In another example, murine IgG₂ₐ mediates ADCC while both murine IgG₂ₐ and IgM mediate CDC.

### Pharmaceutical Compositions and Administration Thereof

The antibodies of the present invention can be administered either alone or in combination with other agents. Examples of antibodies for use in such pharmaceutical compositions are those that comprise an antibody or antibody fragment having the light chain variable region amino acid sequence of any of SEQ ID NO: 1-10. Examples of antibodies for use in such pharmaceutical compositions are also those that comprise a humanized antibody or antibody fragment having the heavy chain variable region amino acid sequence of any of SEQ ID NO: 11-20.

Further examples of antibodies for use in such pharmaceutical compositions are also those that comprise a humanized antibody or antibody fragment having the light chain variable region amino acid sequence of any of SEQ ID NO:76-86. Preferred antibodies for use in such pharmaceutical compositions are also those that comprise a humanized antibody or antibody fragment having the heavy chain variable region amino acid sequence of any of SEQ ID NO:87-101.

Further examples of antibodies for use in such pharmaceutical compositions are also those that comprise a humanized antibody or antibody fragment having the light chain variable region and heavy chain variable region of any of SEQ ID NO: 77 and 89, SEQ ID NO: 80 and 88, SEQ ID NO: 80 and 89, SEQ ID NO: 77 and 87, SEQ ID NO: 77 and 88, SEQ ID NO: 80 and 87, SEQ ID NO: 86 and 100, SEQ ID NO: 85 and 101, or SEQ ID NO: 85 and 10.

Further examples of antibodies for use in such pharmaceutical compositions are also those that comprise a humanized antibody having the light chain region amino acid sequence of any of SEQ ID NO:115, 118, 123 or 124. Preferred antibodies for use in such pharmaceutical compositions are also those that comprise humanized antibody having the heavy chain variable region amino acid sequence of any of SEQ ID NO:125, 126, 127, 138 or 139.

Further examples of antibodies for use in such pharmaceutical compositions are also those that comprise Antibody B1, Antibody B2, Antibody B3, Antibody B4, Antibody B5, Antibody B6, Antibody C1, Antibody C2 or Antibody C3.

Various delivery systems are known and can be used to administer the IL-36R binding agent. Methods of introduction include but are not limited to intradermal, intramuscular, intraperitoneal, intravenous, subcutaneous, intranasal, epidural, and oral routes. The IL-36R binding agent can be administered, for example by infusion, bolus or injection, and can be administered together with other biologically active agents such as chemotherapeutic agents. Administration can be systemic or local. In preferred embodiments, the administration is by subcutaneous injection. Formulations for such injections may be prepared in for example prefilled syringes that may be administered once every other week.

In specific embodiments, the IL-36R binding agent composition is administered by injection, by means of a catheter, by means of a suppository, or by means of an implant, the implant being of a porous, non-porous, or gelatinous material, including a membrane, such as a sialastic membrane, or a fiber. Typically, when administering the composition, materials to which the anti-IL-36R antibody or agent does not absorb are used.

In another aspect, the invention provides an article of manufacture comprising a subcutaneous administration device, which delivers to a patient a fixed dose of an antibody of the present invention. In some embodiments, the subcutaneous administration device is a pre-filled syringe, an autoinjector, or a large volume infusion device. For example, MyDose^{™} product from Roche, a single use infusion device that enables the subcutaneous administration of large quantities of liquid medication, may be used as the administration device. Numerous reusable pen and autoinjector delivery devices have applications in the subcutaneous delivery of a pharmaceutical composition of the present invention. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, Ind.), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, N.J.), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen delivery devices having applications in subcutaneous delivery of a pharmaceutical composition of the present invention include, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, Calif.), the PENLET^{™} (Haselmeier, Stuttgart, Germany), the EPIPEN (Dey, L.P.), and the HUMIRA^{™} Pen (Abbott Labs, Abbott Park III.), YPSOMATE^{™}, YPSOMATE 2.25^{™}, VAIROJECT^{™} (Ypsomed AG, Burgdorf, Switzerland) to name only a few. Additional information relating to example delivery devices that could be used with an antibody of the present invention may be found, for example, in CH705992A2, WO2009/040602, WO2016/169748, WO2016/179713.

In other embodiments, the anti-IL-36R antibody or agent is delivered in a controlled release system. In one embodiment, a pump may be used (see, e.g., Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, e.g., Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

An IL-36R binding agent (e.g., an anti-IL-36R antibody) can be administered as pharmaceutical compositions comprising a therapeutically effective amount of the binding agent and one or more pharmaceutically compatible ingredients.

In one embodiment, the anti-IL-36R antibody or an antigen binding fragment thereof (disclosed herein) is present in a pharmaceutical formulation (as described in co-pending U.S. provisional application No. 62/815,405, filed March 8, 2019, the entire content of which is hereby incorporated herein by reference in its entirety) suitable for administration to a mammal or patient according to any one of the aspects described herein. Various examples to this embodiment are described as numbered clauses (1, 2, 3, etc.) below for convenience. These are provided as examples and do not limit the subject technology. It is noted that any of the dependent clauses may be combined in any combination, and placed into a respective independent clause, e.g., clause 1. The other clauses can be presented in a similar manner.
1. A pharmaceutical formulation including:
   a. An anti-IL-36R antibody or an antigen binding fragment thereof, as disclosed herein, present at a concentration within the range from about 0.5 mg/mL to about 220 mg/mL; and
   b. A pharmaceutically acceptable buffer present at a concentration within the range from about 20 mM to about 80 mM;
   wherein the formulation is characterized by a pH within the range from about 5 to about 8 when in aqueous form.
2. The formulation of clause 1, wherein the formulation is in liquid or powder form.
3. The formulation of clause 1, wherein the anti-IL-36R antibody is present at a concentration of within the range from about 10 mg/mL to about 200 mg/mL.
4. The formulation of clause 1, wherein the anti-IL-36R antibody is present at a concentration of about 20 mg/mL.
5. The formulation of clause 1, wherein the anti-IL-36R antibody is present at a concentration of about 60 mg/mL.
6. The formulation of clause 1, wherein the anti-IL-36R antibody is present at a concentration of about 150 mg/mL.
7. The formulation of clause 1, wherein the buffer comprises histidine, phosphate, succinate, citrate, acetate or TRIS.
8. The formulation of clause 1, wherein the buffer comprises citrate or acetate.
9. The formulation of clause 1, wherein the buffer comprises histidine.
10. The formulation of clause 1, wherein the buffer comprises acetate.
11. The formulation of clause 1, wherein the formulation further comprises a pharmaceutically acceptable tonicifying agent present at a concentration within the range from about 100 mM to about 250 mM.
12. The formulation of clause 11, wherein the tonicifying agent is one or more sugar and/or polyol.
13. The formulation of clause 11, wherein the tonicifying agent is one or more sugar and/or polyol including sucrose, trehalose, sorbitol, magnesium sulfate (MgSO₄), glycerol, mannitol or dextrose.
14. The formulation of clause 11, wherein the tonicifying agent comprises sucrose or trehalose.
15. The formulation of clause 11, wherein the tonicifying agent comprises sucrose.
16. The formulation of clause 11, wherein the tonicifying agent comprises trehalose.
17. The formulation of clause 1, wherein the formulation further comprises a pharmaceutically acceptable stabilizer present at a concentration within the range from about 0 mM to about 80 mM.
18. The formulation of clause 17, wherein the stabilizer comprises an amino acid including arginine, histidine, glycine, cysteine, proline, methionine, lysine, aspartate, glutamate or pharmaceutically acceptable salts thereof.
19. The formulation of clause 17, wherein the stabilizer comprises L-arginine or pharmaceutically acceptable salts thereof.
20. The formulation of clause 1, wherein the formulation further comprises a pharmaceutically acceptable salt present at a concentration of within the range from about 0 to about 150 mM.
21. The formulation of clause 20, wherein the salt comprises sodium chloride (NaCl), magnesium chloride (MgCl₂), potassium chloride (KCI), lithium chloride (LiCI), calcium chloride (CaCl₂), boric acid salts or zinc chloride (ZnCl₂).
22. The formulation of clause 20, wherein the salt comprises sodium chloride (NaCl).
23. The formulation of clause 1, wherein the formulation further comprises a pharmaceutically acceptable surfactant present at a concentration within the range from about 0 g/L to about 1.5 g/L.
24. The formulation of clause 23, wherein the surfactant comprises poloxamer 188, polysorbate 20, polysorbate 40, polysorbate 60 or polysorbate 80.
25. The formulation of clause 23, wherein the surfactant comprises polysorbate 20, polysorbate 40, polysorbate 60 or polysorbate 80.
26. The formulation of clause 23, wherein the surfactant comprises polysorbate 20.
27. The formulation of clause 23, wherein the surfactant comprises polysorbate 80.
28. A pharmaceutical formulation including:
   a. an anti-IL-36R antibody or an antigen binding fragment thereof, as disclosed herein, present at a concentration within the range from about 10 mg/mL to about 200 mg/mL;
   b. an acetate and/or histidine buffer present at a concentration within the range from about 20 mM to about 80 mM;
   c. sucrose and-/-or trehalose present at a concentration within the range from about 100 mM to about 250 mM;
   d. L-arginine and-/-or pharmaceutically acceptable salts thereof present at a concentration within the range from about 0 mM to about 80 mM;
   e. sodium chloride (NaCl) present at a concentration of within the range from about 0 to about 150 mM; and
   f. polysorbate 20 and/or polysorbate 80 present at a concentration within the range from about 0 g/L to about 1.5 g/L;
   wherein the formulation is characterized by a pH within the range from about 5 to about 7 when in aqueous form.
29. A pharmaceutical formulation including:
   a. an anti-IL-36R antibody or an antigen binding fragment thereof, as disclosed herein, present at a concentration of about 20 mg/mL;
   b. an citrate buffer present at a concentration at a concentration of about 25 mM;
   c. sucrose and/or trehalose present at a concentration of about 200 mM;
   d. polysorbate 80 present at a concentration of about 0.4 g/L;
   wherein the formulation is characterized by a pH within the range from about 6 to about 7 when in aqueous form.
30. A pharmaceutical formulation including:
   a. an anti-IL-36R antibody or an antigen binding fragment thereof, as disclosed herein, present at a concentration of about 60 mg/mL;
   b. an acetate buffer present at a concentration at a concentration of about 45 mM;
   c. sucrose and/or trehalose present at a concentration of about 150 mM;
   d. L-arginine or pharmaceutically acceptable salts thereof present at a concentration of about 25 mM; and
   e. polysorbate 20 present at a concentration of about 0.4 g/L;
   wherein the formulation is characterized by a pH within the range from about 5 to about 6 when in aqueous form.
31. A pharmaceutical formulation including:
   a. an anti-IL-36R antibody or an antigen binding fragment thereof, as disclosed herein, present at a concentration of about 150 mg/mL;
   b. an acetate buffer present at a concentration at a concentration of about 45 mM;
   c. sucrose or trehalose present at a concentration of about 150 mM;
   d. L-arginine or pharmaceutically acceptable salts thereof present at a concentration of about 25 mM; and
   e. polysorbate 20 present at a concentration of about 0.4 g/L;
   wherein the formulation is characterized by a pH within the range from about 5 to about 6 when in aqueous form.
32. The pharmaceutical formulation of any one of clauses 1-31, wherein the formulation is characterized by an osmolality within the range from about 210 mOsmol/kg to about 390 mOsm/kg.
33. The pharmaceutical formulation of any one of clauses 1-32, wherein less than about 5% of the antibody is present in an aggregate form in the formulation.
34. The pharmaceutical formulation of any one of clauses 1-33, wherein the formulation is sterile.
35. The pharmaceutical formulation of any one of clauses 1-34, wherein the formulation is stable upon freezing and thawing.
36. The pharmaceutical formulation of any of clauses 1-35, wherein the formulation comprises water or is reconstituted with water.
37. The pharmaceutical formulation of any of clauses 1-36, wherein the formulation has a pH of between about 5 to about 6 in liquid form or when reconstituted with water.
38. The pharmaceutical formulation of any of clauses 1-37, wherein the formulation has a pH of about 6 in liquid or when reconstituted with water.
39. The pharmaceutical formulation of any of clauses 1-37, wherein the formulation has at least one feature selected from the group consisting of:
   (i) Increased shelf life
   (ii) better temperature stability,
   (iii) decreased formation of aggregates,
   (iv) better chemical stability,
   (v) decreased viscosity, and
   as compared to a reference formulation.
40. The pharmaceutical formulation of any of clauses 1-37, wherein the formulation having at least one feature selected from the group consisting of:
   (a) decreased percentage of aggregates as measured by High Performance Size Exclusion Chromatography (HP-SEC),
   (b) higher percentage of monomers as measured by HP-SEC,
   (c) higher percentage of main peak (less degradation of charge variants) measured by CEX,
   (d) lower percentage of subvisual particles such as ≥ 10 µm and ≥ 25 µm, and
   (e) lower turbidity value in Formazin Nephelometry Units (FNU), after storage at about 40°C as compared to the reference formulation.
41. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation is selected from the group consisting of:
   I. formulation including about 20 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0;
   II. formulation including about 60 mg/mL of the anti-IL-36R antibody, about 45 mM acetate, about 150 mM sucrose, about 25 mM L-Arginine, about 0.4 g/L Polysorbate 20, with a pH of about 5.5;
   III. formulation including about 20 mg/mL of the anti-IL-36R antibody, about 45 mM acetate, about 180 mM sucrose, about 25 mM Glycine, about 0.4 g/L Polysorbate 80, with a pH of about 5.5;
   IV. formulation including about 150 mg/mL of the anti-IL-36R antibody, about 25 mM citrate, about 150 mM trehalose, about 25 mM methionine, about 0.2 g/L Polysorbate 20, with a pH of about 6.0;
   V. formulation including about 150 mg/mL of the anti-IL-36R antibody, about 25 mM histidine, about 180 mM sucrose, about 20 mM mannitol, about 0.2 g/L Polysorbate 20, with a pH of about 6.5;
   VI. formulation including about 20 mg/mL of the anti-IL-36R antibody, about 25 mM citrate, about 200 mM sucrose, about 0.4 g/L Polysorbate 80, with a pH of about 6.5;
   VII. formulation including about 150 mg/mL of the anti-IL-36R antibody, about 45 mM acetate, about 150 mM sucrose, about 25 mM L-Arginine, about 0.4 g/L Polysorbate 20, with a pH of about 5.5;
   VIII. formulation including about 15 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
   IX. formulation including about 80 mg/mL of the anti-IL-36R antibody, about 25 mM acetate, about 100 mM mannitol, about 50 mM NaCl, about 0.2 g/L Polysorbate 20, with a pH of about 5.5;
   X. formulation including about 100 mg/mL of the anti-IL-36R antibody, about 20 mM succinate, about 220 mM sucrose, about 0.1 g/L Polysorbate 80, with a pH of about 6.0; and
   XI. formulation including about 60 mg/mL of the anti-IL-36R antibody, about 25 mM citrate, about 0.4 g/L Polysorbate 20, with a pH of about 6.5.
42. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 20 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0.
43. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 60 mg/mL of the anti-IL-36R antibody, about 45 mM acetate, about 150 mM sucrose, about 25 mM L-Arginine, about 0.4 g/L Polysorbate 20, with a pH of about 5.5.
44. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 20 mg/mL of the anti-IL-36R antibody, about 45 mM acetate, about 180 mM sucrose, about 25 mM Glycine, about 0.4 g/L Polysorbate 80, with a pH of about 5.5.
45. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 150 mg/mL of the anti-IL-36R antibody, about 25 mM citrate, about 150 mM trehalose, about 25 mM methionine, about 0.2 g/L Polysorbate 20, with a pH of about 6.0.
46. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 150 mg/mL of the anti-IL-36R antibody, about 25 mM histidine, about 180 mM sucrose, about 20 mM mannitol, about 0.2 g/L Polysorbate 20, with a pH of about 6.5.
47. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 20 mg/mL of the anti-IL-36R antibody, about 25 mM citrate, about 200 mM sucrose, about 0.4 g/L Polysorbate 80, with a pH of about 6.5.
48. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 150 mg/mL of the anti-IL-36R antibody, about 45 mM acetate, about 150 mM sucrose, about 25 mM L-Arginine, about 0.4 g/L Polysorbate 20, with a pH of about 5.5.
49. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 15 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0.
50. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 80 mg/mL of the anti-IL-36R antibody, about 25 mM acetate, about 100 mM mannitol, about 50 mM NaCl, about 0.2 g/L Polysorbate 20, with a pH of about 5.5.
51. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 100 mg/mL of the anti-IL-36R antibody, about 20 mM succinate, about 220 mM sucrose, about 0.1 g/L Polysorbate 80, with a pH of about 6.0.
52. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation includes about 60 mg/mL of the anti-IL-36R antibody, about 25 mM citrate, about 0.4 g/L Polysorbate 20, with a pH of about 6.5.
53. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 20 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0.
54. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 60 mg/mL of the anti-IL-36R antibody, about 45 mM acetate, about 150 mM sucrose, about 25 mM L-Arginine, about 0.4 g/L Polysorbate 20, with a pH of about 5.5.
55. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 20 mg/mL of the anti-IL-36R antibody, about 45 mM acetate, about 180 mM sucrose, about 25 mM Glycine, about 0.4 g/L Polysorbate 80, with a pH of about 5.5.
56. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 150 mg/mL of the anti-IL-36R antibody, about 25 mM citrate, about 150 mM trehalose, about 25 mM methionine, about 0.2 g/L Polysorbate 20, with a pH of about 6.0.
57. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 150 mg/mL of the anti-IL-36R antibody, about 25 mM histidine, about 180 mM sucrose, about 20 mM mannitol, about 0.2 g/L Polysorbate 20, with a pH of about 6.5.
58. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 20 mg/mL of the anti-IL-36R antibody, about 25 mM citrate, about 200 mM sucrose, about 0.4 g/L Polysorbate 80, with a pH of about 6.5.
59. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 150 mg/mL of the anti-IL-36R antibody, about 45 mM acetate, about 150 mM sucrose, about 25 mM L-Arginine, about 0.4 g/L Polysorbate 20, with a pH of about 5.5.
60. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 15 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0.
61. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 80 mg/mL of the anti-IL-36R antibody, about 25 mM acetate, about 100 mM mannitol, about 50 mM NaCl, about 0.2 g/L Polysorbate 20, with a pH of about 5.5.
62. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 100 mg/mL of the anti-IL-36R antibody, about 20 mM succinate, about 220 mM sucrose, about 0.1 g/L Polysorbate 80, with a pH of about 6.0.
63. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation includes: about 60 mg/mL of the anti-IL-36R antibody, about 25 mM citrate, about 0.4 g/L Polysorbate 20, with a pH of about 6.5.
64. A pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   iii. a light chain including an amino acid sequence set forth as SEQ ID NO:118 and a heavy chain including an amino acid sequence set forth as SEQ ID NO:127;
   wherein the formulation is selected from the group consisting of:
   I. formulation including about 20 mg/mL to about 150 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0;
   II. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
   III. formulation including about 20 mg/mL to about 150 mg/mL of the anti-IL-36R antibody, about 45 mM acetate, about 180 mM sucrose, about 25 mM Glycine, about 0.4 g/L Polysorbate 80, with a pH of about 5.5;
   IV. formulation including about 20 mg/mL to about 150 mg/mL of the anti-IL-36R antibody, about 25 mM citrate, about 150 mM trehalose, about 25 mM methionine, about 0.2 g/L Polysorbate 20, with a pH of about 6.0;
   V. formulation including about 20 mg/mL to about 150 mg/mL of the anti-IL-36R antibody, about 25 mM histidine, about 180 mM sucrose, about 20 mM mannitol, about 0.2 g/L Polysorbate 20, with a pH of about 6.5;
   VI. formulation including about 20 mg/mL to about 150 mg/mL of the anti-IL-36R antibody, about 25 mM citrate, about 200 mM sucrose, about 0.4 g/L Polysorbate 80, with a pH of about 6.5;
   VII. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
   VIII. formulation including about 20 mg/mL to about 150 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
   IX. formulation including about 20 mg/mL to about 150 mg/mL of the anti-IL-36R antibody, about 25 mM acetate, about 100 mM mannitol, about 50 mM NaCl, about 0.2 g/L Polysorbate 20, with a pH of about 5.5;
   X. formulation including about 20 mg/mL to about 150 mg/mL of the anti-IL-36R antibody, about 20 mM succinate, about 220 mM sucrose, about 0.1 g/L Polysorbate 80, with a pH of about 6.0; and
   XI. formulation including about 20 mg/mL to about 150 mg/mL of the anti-IL-36R antibody, about 25 mM citrate, about 0.4 g/L Polysorbate 20, with a pH of about 6.5.
65. A pharmaceutical formulation including:
   an anti-IL-36R antibody or antigen-binding fragment thereof, including:
   a light chain variable region comprising the amino acid sequence of SEQ ID NO: 77; and a heavy chain variable region comprising the amino acid sequence of SEQ ID NO: 87;or
   a light chain variable region comprising the amino acid sequence of SEQ ID NO: 77; and a heavy chain variable region comprising the amino acid sequence of SEQ ID NO: 88; or
   a light chain variable region comprising the amino acid sequence of SEQ ID NO: 77; and a heavy chain variable region comprising the amino acid sequence of SEQ ID NO: 89; or
   a light chain variable region comprising the amino acid sequence of SEQ ID NO: 80; and a heavy chain variable region comprising the amino acid sequence of SEQ ID NO: 87;or
   a light chain variable region comprising the amino acid sequence of SEQ ID NO: 80; and a heavy chain variable region comprising the amino acid sequence of SEQ ID NO: 88; or
   a light chain variable region comprising the amino acid sequence of SEQ ID NO: 80; and a heavy chain variable region comprising the amino acid sequence of SEQ ID NO: 89;
   wherein the formulation is selected from the group consisting of:
   I. formulation including about 20 mg/mL to about 150 mg/mL of the anti-IL-36R antibody, about 40 mM histidine, about 120 mM sucrose, about 50 mM L-Arginine, about 5 mM NaCl and about 1.0 g/L Polysorbate 20, with a pH of about 6.0;
   II. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
   III. formulation including about 20 mg/mL to about 150 mg/mL of the anti-IL-36R antibody, about 45 mM acetate, about 180 mM sucrose, about 25 mM Glycine, about 0.4 g/L Polysorbate 80, with a pH of about 5.5;
   IV. formulation including about 20 mg/mL to about 150 mg/mL of the anti-IL-36R antibody, about 25 mM citrate, about 150 mM trehalose, about 25 mM methionine, about 0.2 g/L Polysorbate 20, with a pH of about 6.0;
   V. formulation including about 20 mg/mL to about 150 mg/mL of the anti-IL-36R antibody, about 25 mM histidine, about 180 mM sucrose, about 20 mM mannitol, about 0.2 g/L Polysorbate 20, with a pH of about 6.5;
   VI. formulation including about 20 mg/mL to about 150 mg/mL of the anti-IL-36R antibody, about 25 mM citrate, about 200 mM sucrose, about 0.4 g/L Polysorbate 80, with a pH of about 6.5;
   VII. formulation including about 20 mg/mL to about 150 mg/mL of the anti-IL-36R antibody, about 45 mM acetate, about 150 mM sucrose, about 25 mM L-Arginine, about 0.4 g/L Polysorbate 20, with a pH of about 5.5;
   VIII. formulation including about 20 mg/mL to about 150 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
   IX. formulation including about 20 mg/mL to about 150 mg/mL of the anti-IL-36R antibody, about 25 mM acetate, about 100 mM mannitol, about 50 mM NaCl, about 0.2 g/L Polysorbate 20, with a pH of about 5.5;
   X. formulation including about 20 mg/mL to about 150 mg/mL of the anti-IL-36R antibody, about 20 mM succinate, about 220 mM sucrose, about 0.1 g/L Polysorbate 80, with a pH of about 6.0; and
   XI. formulation including about 20 mg/mL to about 150 mg/mL of the anti-IL-36R antibody, about 25 mM citrate, about 0.4 g/L Polysorbate 20, with a pH of about 6.5.
66. A pharmaceutical product including a vial or syringe including the pharmaceutical formulation according to any of the preceding clauses for use in any one of the aspects of the present invention.
67. The pharmaceutical product according to clause 66 further including a pre-assembled injection device.
68. The pharmaceutical product of clause 67 wherein the pre-assembled injection device is an autoinjector or a syringe with or without a needle safety device.
69. A pre-assembled injection device including a pharmaceutical formulation according to any of the preceding clauses for use in any one of the aspects of the present invention.
70. The pre-assembled injection device according to clause 69, wherein said device is an autoinjector or a syringe with or without a needle safety device.
71. The pre-assembled injection device according to clause 69, wherein said formulation is suitable for intravenous, subcutaneous or intramuscular administration.
72. The pre-assembled injection device according to clause 70, wherein the autoinjector or the syringe with or without needle safety device includes a pharmaceutical formulation including:
   an anti-IL-36R antibody or antigen-binding fragment thereof, including:
   i. a light chain including an amino acid sequence set forth as SEQ ID NO:118 and a heavy chain including an amino acid sequence set forth as SEQ ID NO:125; or
   ii. a light chain including an amino acid sequence set forth as SEQ ID NO:118 and a heavy chain including an amino acid sequence set forth as SEQ ID NO:126; or
   a light chain including an amino acid sequence set forth as SEQ ID NO:118 and a heavy chain including an amino acid sequence set forth as SEQ ID NO:127; wherein the formulation is selected from the group consisting of:
   I. formulation including about 20 mg/ml of the anti-IL-36R antibody, about 40 mM histidine, about 120 mM sucrose, about 50 mM L-Arginine, about 5 mM NaCl and about 1.0 g/L Polysorbate 20, with a pH of about 6.0;
   II. formulation including about 60 mg/mL of the anti-IL-36R antibody, about 45 mM acetate, about 150 mM sucrose, about 25 mM L-Arginine, about 0.4 g/L Polysorbate 20, with a pH of about 5.5;
   III. formulation including about 20 mg/mL of the anti-IL-36R antibody, about 45 mM acetate, about 180 mM sucrose, about 25 mM Glycine, about 0.4 g/L Polysorbate 80, with a pH of about 5.5;
   IV. formulation including about 150 mg/mL of the anti-IL-36R antibody, about 25 mM citrate, about 150 mM trehalose, about 25 mM methionine, about 0.2 g/L Polysorbate 20, with a pH of about 6.0;
   V. formulation including about 150 mg/mL of the anti-IL-36R antibody, about 25 mM histidine, about 180 mM sucrose, about 20 mM mannitol, about 0.2 g/L Polysorbate 20, with a pH of about 6.5;
   VI. formulation including about 20 mg/mL of the anti-IL-36R antibody, about 25 mM citrate, about 200 mM sucrose, about 0.4 g/L Polysorbate 80, with a pH of about 6.5;
   VII. formulation including about 150 mg/mL of the anti-IL-36R antibody, about 45 mM acetate, about 150 mM sucrose, about 25 mM L-Arginine, about 0.4 g/L Polysorbate 20, with a pH of about 5.5;
   VIII. formulation including about 15 mg/mL of the anti-IL-36R antibody, about 35 mM histidine, about 180 mM trehalose, about 25 mM L-Arginine, about 3 mM NaCl, about 0.4 g/L Polysorbate 80, with a pH of about 6.0;
   IX. formulation including about 80 mg/mL of the anti-IL-36R antibody, about 25 mM acetate, about 100 mM mannitol, about 50 mM NaCl, about 0.2 g/L Polysorbate 20, with a pH of about 5.5;
   X. formulation including about 100 mg/mL of the anti-IL-36R antibody, about 20 mM succinate, about 220 mM sucrose, about 0.1 g/L Polysorbate 80, with a pH of about 6.0; and
   XI. formulation including about 60 mg/mL of the anti-IL-36R antibody, about 25 mM citrate, about 0.4 g/L Polysorbate 20, with a pH of about 6.5.
73. The pre-assembled injection device according to clause 70, wherein the autoinjector or the syringe with a needle safety device includes:
   a. about 300 mg of the antibody in about 2 mL formulation volume; or
   b. about 225 mg of the antibody in about 1.5 mL formulation volume; or
   c. about 150 mg of the antibody in about 1 mL formulation volume; or
   d. about 75 mg of the antibody in about 0.5 mL formulation volume; or
   e. about 60 mg of the antibody in about 0.4 mL formulation volume.
74. The vial according to clause 66, wherein the vial includes:
   a. about 1200 mg of the antibody in about 20 mL formulation volume; or
   b. about 900 mg of the antibody in about 15 mL formulation volume; or
   c. about 600 mg of the antibody in about 10 mL formulation volume; or
   d. about 300 mg of the antibody in about 150 mL formulation volume; or
   e. about 1500 mg of the antibody in about 2.5 mL formulation volume.

A pharmaceutical product, including: a vial including about 100 mg to 1500 mg of an anti-IL-36R antibody in powder form; instructions for reconstitution of the anti-IL-36R antibody; and instructions for preparing the reconstituted antibody for infusion, wherein the anti-IL-36R antibody comprises a light chain including an amino acid sequence set forth as SEQ ID NO:118 and a heavy chain including an amino acid sequence set forth as any one of SEQ ID Nos:125, 126 or 127; and the reconstitution instructions require reconstitution with water for injection to an extractable volume from 1 to 50 mL.Further, the pharmaceutical composition can be provided as a pharmaceutical kit comprising (a) a container containing a IL-36R binding agent (e.g., an anti-IL-36R antibody) in lyophilized form and (b) a second container containing a pharmaceutically acceptable diluent (e.g., sterile water) for injection. The pharmaceutically acceptable diluent can be used for reconstitution or dilution of the lyophilized anti-IL-36R antibody or agent. Optionally associated with such container(s) can be a notice in the form prescribed by a governmental agency regulating the manufacture, use or sale of pharmaceuticals or biological products, which notice reflects approval by the agency of manufacture, use or sale for human administration.

Such combination therapy administration can have an additive or synergistic effect on disease parameters (e.g., severity of a symptom, the number of symptoms, or frequency of relapse).

With respect to therapeutic regimens for combinatorial administration, in a specific embodiment, an anti-IL-36R antibody or IL-36R binding agent is administered concurrently with a therapeutic agent. In another specific embodiment, the therapeutic agent is administered prior or subsequent to administration of the anti-IL-36R antibody or IL-36R binding agent, by at least an hour and up to several months, for example at least an hour, five hours, 12 hours, a day, a week, a month, or three months, prior or subsequent to administration of the anti-IL-36R antibody or IL-36R binding agent.

The invention is further described in the following examples, which are not intended to limit the scope of the invention.

### Examples

### Example 1: Randomized, Double-blind, Placebo-controlled, Multicenter Study to Evaluate the Safety and Efficacy of anti-IL36R antibody induction Therapy in patients with moderate-to-severely active ulcerative colitis who have failed previous biologics therapy

An antibody of the present invention (disclosed herein and also in U.S. Patent No. 9,023,995) is a humanized antagonistic monoclonal IgG1 antibody that blocks human IL36R signaling. Binding of the anti-IL-36R antibody to IL36R is anticipated to prevent the subsequent activation of IL36R by cognate ligands (IL36 α, β and γ) and downstream activation of proinflammatory and pro-fibrotic pathways with the aim to reduce epithelial cell/ fibroblast/immune cell-mediated inflammation and interrupt the inflammatory response that drives pathogenic cytokine production in inflammatory diseases including generalized pustular psoriasis (GPP), palmoplantar pustulosis (PPP) and inflammatory bowel disease (IBD).

Preclinical profiles of the anti-IL-36R antibody and clinical data from healthy volunteer trials suggest that the anti-IL-36R antibody is safe, tolerable and may address an unmet medical need in UC patients by a dual anti-inflammatory and anti-fibrotic mechanism of action.

In this trial, data on pharmacokinetics of 12 weeks of anti-IL-36R antibody treatment in UC patients will be collected and correlated with the pharmacodynamic (PD) treatment response. These data will help understand the PK characteristics of the anti-IL-36R antibody in UC, which may differ from those in healthy volunteers and patients with other diseases due to the expected intestinal protein loss subsequent to mucosal inflammation and ulceration in the colon.

### ABBREVIATIONS

- ADA: Anti-drug antibodies
- ADCC: antibody-dependent cellular cytotoxicity
- AE: Adverse Event
- AESI: Adverse Event of Special Interest
- AUC: Area under the Curve
- b.i.d.: bis in die (twice daily dosing)
- BL: Base Line
- CCDS: Company Core Data Sheet
- BIO: Biologics
- CD: Crohn's disease
- CDC: complement-dependent cytotoxicity
- CI: Confidence Interval
- CML: Clinical Monitor Local
- CRA: Clinical Research Associate
- CRF: Case Report Form, paper or electrocic (sometimes referred to as "eCRF")
- CTCAE: Common Terminology Criteria for Adverse Events
- CTP: Clinical Trial Protocol
- CTR: Clinical Trial Report
- DILI: Drug Induced Liver Injury
- DMC: Data Monitoring Committee
- ECG: Electrocardiogram
- EDC: Electronic Data Capture
- EOT: End of Treatment
- EOS: End of Study
- ePRO: Electronic Patient Reported Outcome
- EQ-5D(-5L): Questionnaire developed by EuroQoL Group
- ESS Mayo: Endoscopy Score
- EudraCT: European Clinical Trials Database
- FACIT: Functional Assessment of Chronic Illness Therapy
- FAS: Full Analysis Set
- FC: Flow Chart
- FcR: Fc receptor - a protein found on the surface of certain cells
- FIH: First in human
- GCP: Good Clinical Practice
- GPP: generalized pustular psoriasis
- HPC: Human Pharmacology Centre
- HCRU: Healthcare resource utilisation
- HT29: a human colorectal adenocarcinoma cell line with epithelial morphology
- IB: Investigator's Brochure
- IBD: inflammatory bowel disease
- IBDQ: Inflammatory Bowel Disease Questionnaire
- IC90: Inhibitory concentration of 90 (mg/mL)
- IEC: Independent Ethics Committee
- IFNγ: Interferon gamma
- IgG1: Immunglobulin G1
- IHC: immunohistochemistry
- IL36R: Interleukin 36R
- IRB: Institutional Review Board
- IRT: Interactive Response Technology
- ISF: Investigator Site File
- ITE: indirect target engagement
- i.v.: intravenous
- LoEE: List of Essential Element
- MCS: Mayo Clinical Score
- mMCS: modified Mayo Clinical Score
- MedDRA: Medical Dictionary for Drug Regulatory Activities
- mESS: modified Endoscopic Subscore
- MST: Medical Sub Team
- NF-κB: Nuclear factor 'kappa-light-chain-enhancer' of activated B-cells
- NOAEL: No-observed-adverse-effect level
- OPU: Operative Unit
- PBO: Placebo
- PBMC: Peripheral Blood Mononuclear Cell
- PD: Pharmacodynamics
- PK: Pharmacokinetics
- p.o.: per os (oral)
- PoC: proof of concept
- PPP: palmoplantar pustulosis
- q.d.: quaque die (once a day)
- q.w.: quatery week (every 4th week)
- eDC: electronic Data Capturing
- RBS: Rectal Bleeding Score
- RCTC: Rheumatology Common Toxicity Criteria
- REP: Residual Effect Period
- RS: Randomized Set
- SAE: Serious Adverse Event
- s.c.: subcutaneous
- SF-36: 36 question instrument to measure health-related quality of life
- SFS: Stool Frequency Score
- SMC: Safety Monitoring Committee
- SmPC: Summary of Product Characteristics
- SUSAR: Suspected Unexpected Serious Adverse Reactions
- TMDD: target-mediated drug disposition
- TB: test blood test that aids in the detection of Mycobacterium tuberculosis, the bacteria which causes tuberculosis (TB)
- TCM: Trial Clinical Monitor
- TDMAP: Trial Data Management and Analysis Plan
- TGF-β: tissue growth factor
- t.i.d.: ter in die (3 times a day)
- TMF: Trial Master File
- TNF: Tumor necrosis factor
- TNFi: TNFα inhibitor
- TSAP: Trial Statistical Analysis Plan
- UC: Ulcerative Colitis
- WHO: World Health Organization
- WOCBP: Woman of childbearing potential
- WPAI-UC: Work Productivity and Activity Impairment Questionnaire, Ulcerative Colitis -specific version

### TRIAL OBJECTIVES AND ENDPOINTS

This trial has two sequentially enrolling parts with different objectives. The primary objectives of this trial are
- to prove the concept of clinical activity of an anti-IL-36R antibody in patients with moderate-to-severely active ulcerative colitis who have failed previous biologic treatments and to identify efficacious and safe dose regimens in Part 1 (Phase II)
- to confirm efficacy and safety of an anti-IL-36R antibody in patients with moderate-to-severely active ulcerative colitis who have failed previous biologic treatments in Part 2 (Phase III)

Both parts of the trial share the same endpoints and definitions, though primary, key secondary and secondary endpoints will only be included in the statistical testing strategy in part 2 (Phase III).

### Primary endpoint (Part 1 and Part 2)

- Proportion of patients with Clinical Remission at week 12 (defined as mMCS SFS=0 or 1, if drop ≥1 from BL; and RBS=0; and mESS≤1)

### Key Secondary endpoints (Part 2 I Phase III only)

- Proportion of patients with Mucosal Healing at week 12 (defined as mESS ≤ 1)
- Proportion of patients with Clinical response at week 12 (defined as total MCS reduction ≥3 pts. and ≥30% from BL; AND RBS drop from baseline by ≥1 pt., or absolute RBS ≤1 pt)

### Secondary endpoint (Part 1 and Part 2)

- Change in IBDQ score from baseline at week 12
- Proportion of patients with combined Mucosal healing and histologic remission at week 12
(defined as mESS ≤ 1 and Robarts Histology Index ≤ 6)

### Secondary endpoints (Part 1 / Phase II only)

- Proportion of patients with Mucosal Healing at week 12
- Proportion of patients with Clinical response at week 12

### FURTHER OBJECTIVES AND FURTHER ENDPOINTS

### Further objectives

- to determine the safety and tolerability of an anti-IL-36R antibody of the present invention in patients with moderate-to-severely active ulcerative colitis who have failed previous biologic treatments

### Further endpoints

The following endpoints will be assessed in both Part 1 and Part 2.
- Proportion of patients with Symptomatic remission at Week 12. Symptomatic remission is defined by a total MCS of 2 points or lower, with no individual subscore exceeding 1 point, and both rectal bleeding and stool frequency subscore of 0.
- Proportion of patients with Histological remission (RHI ≤ 6) at weeks 12
- Changes in Robarts Histology index (RHI) at week 12
- Proportion of patients with Total clinical remission at week 12, (defined by total MCS ≤2 and no individual subscore (SFS; RBS; mESS; PGA) >1).
- Total Mayo score at week 12
- Total Mayo score change from BL at week 12
- Modified Mayo score at week 12
- Modified Mayo score change from BL at week 12
- Partial Mayo score (defined as total of SFS, RBS and PGA, i.e. excl. mESS) over time
- Partial Mayo score change from BL over time
- Change in IBDQ score from baseline over time
- Changes in CRP over time
- Changes in FCP over time
- Proportion of patients with IBDQ response over time (response being defined by a decrease from BL of at least 16 points)
- Proportion of patients with IBDQ remission over time (remission being defined as an IBDQ score of at least 170)
- Changes in EQ-5D(-5L) score from BL at over time

The following assesssments will only be conducted in part 2:
- FACIT-Fatigue score change from BL at week 8 and 12
- Proportion of FACIT-Fatigue responders at week 8 and 12 (response being defined by a decrease from BL of at least 3 points)
- SF-36 subscales, physical, and mental summary score change from BL at week 8 and 12
- Proportion of SF-36 physical and mental summary score responders at week 8 and 12 (response being defined by an increase from BL of at least 4 points)
- WPAI-UC score change from BL at week 8 and 12
- Proportion of patients with a colectomy through 12 weeks
- Proportion of patients with a UC-related hospitalisation through 12 weeks
- Proportion of patients with all-cause hospitalisation through 12 weeks

**Table 2: Definition of Clinical Outcomes**

| **Type of Response** | **MCS subscore** | | | | **Total MCS** | **Modified MCS** |
|---|---|---|---|---|---|---|
| | **RBS** | **mESS** | **SFS** | **PGA** | | |
| **Clinical remission (CR)** | 0 | <1 | 0 or 1, and drop ≥1 from baseline | | | ≤2 |
| **Total Clinical remission (tCR)** | ≤1 | ≤1 | ≤1 | ≤1 | ≤2 | |
| **Clinical response** | <1 or Reduction from BL ≤1 | not missing | not missing | not missing | Reduction from BL ≥3 and ≥30% | |
| **Symptomatic remission** | 0 | ≤1 | 0 | ≤1 | ≤2 | |

| | | | | | | |
|---|---|---|---|---|---|---|
| • Abbreviations: RBS - rectal bleeding score: mESS - modified endoscopic subscore; SFS - stool frequency score; PGA - physician's global assessment; • If greyed out, tire corresponding criterion (in column) is not relevant to evaluate the corresponding type of response(in row) • Total MCS is calculated as the sum of RBS, mESS, SFS and PGA • Modified MCS is calculated as the sum of RES, mESS and SFS • Partial MCS is calculated as the sum of RBS, SFS and PGA | | | | | | |

### DESCRIPTION OF DESIGN AND TRIAL POPULATION

The distinct trial parts are defined as follows:

### Part 1 / Proof of Concept

Part 1 of the trial will consist of a screening period of up to a maximum of 5 weeks and a 12 week, parallel group, placebo-controlled, double-blind intravenous induction therapy period, and a 12 week safety follow-up period for patients not rollingover into maintenance trial 1368-0017.

Approximately 160 eligible patients with moderate to severe ulcerative colitis who have failed previous biologic treatments in the past will be randomised in a 1:1:1:1 ratio to one of the 4 following treatment groups:
Group 1: Placebo i.v., q4w (n=40)
Group 2: anti-IL-36R antibody 300 mg i.v., single dose (SD) (n=40)
Group 3: anti-IL-36R antibody 450 mg i.v., q4w (n=40)
Group 4: anti-IL-36R antibody 1200 mg i.v., q4w (n=40)

Part 1 will explore the efficacy (proof of concept of clinical activity) and safety of three different dose regimens of an anti-IL-36R antibody of the present invention compared to placebo as induction treatment in ulcerative colitis; the PK/PD relationship in ulcerative colitis will also be established and help select the two dose regimens for part 2. A total of 160 patients will be randomized (1:1:1:1) to receive 12 weeks of treatment with the anti-IL-36R antibody 300 mg i.v. SD, the anti-IL-36R antibody 450 mg i.v. q4w; the anti-IL-36R antibody 1200 mg i.v. q4w, or matching placebo. At week 12, patients will be evaluated for the primary endpoint of clinical remission. Patients who terminate study drug early will continue safety follow up in this study until 16 weeks after last study drug administration, while patients who complete the 12-week primary endpoint assessment in part 1 of this study will enter the maintenance trial 1368-0017, which offers open label active treatment with the anti-IL-36R antibody guided by the outcome of Part 1 of the induction trial:
- patients meeting the clinical response endpoint (cf. Section 5.1) at week 12 will receive long-term maintenance treatment with SC maintenance dosing of the anti-IL-36R antibody for up to 7 years.
- patients not achieving the clinical response (whether on active drug or placebo) at week 12 of Part 1 of the trial will undergo a further 12 weeks open label re-induction with a high dose i.v. regimen of the anti-IL-36R antibody in trial 1368-0017, after which eligibility will be re-assessed. If the patient subsequently meets the clinical response definition, then patients will be entered into the s.c. maintenance part, while non-responders will be discontinued from the trial 1368-0017.

### Part 2 / Confirmatory Efficacy/Safety Evaluation

Part 2 of the trial will consist of a screening period of up to a maximum of 5 weeks, a 12 week, parallel group, placebo-controlled, double-blind intravenous induction therapy period, and a 12 week safety follow-up period for patients not rollingover into subsequent maintenance study 1368-0020.

Approximately 390 eligible patients with moderate to severe UC who have failed previous biologic treatments in the past will be randomised in a 1:1:1 ratio to one of the 3 following treatment groups:
Group 1: Placebo i.v. q4w (n= 130)
Group 2: anti-IL-36R antibody selected low dose i.v. q4w (n= 130)
Group 3: anti-IL-36R antibody selected high dose i.v. q4w (n= 130)

Patients who discontinue study drug early will be followed up in this study until 16 weeks after last study drug administration, while patients who complete the 12-week primary endpoint assessment in part 2 of this study will enter the pivotal maintenance trial 1368-0020, which offers a randomized maintenance treatment guided by the outcome of Part 2 of the induction trial:
- patients on an anti-IL-36R antibody treatment who meet the clinical response endpoint at week 12 will be entered into the double-blind, placebo controlled randomized withdrawal study (1368-0020) and randomized to receive subcutaneous treatment with the anti-IL-36R antibody or placebo until week 52.
- patients on placebo treatment who meet the clinical response endpoint at week 12 will be entered into the double-blind, placebo controlled randomized withdrawal study (1368-0020) and mock-randomized to receive subcutaneous treatment with placebo until week 52.
- patients not achieving a clinical response (whether on active drug or placebo) will undergo a further 12 weeks open label i.v. re-induction with the anti-IL-36R antibody in study 1368-0020, after which eligibility will be re-assessed. Patients who subsequently meet the clinical response definition, will be randomized into the randomized withdrawal part of study 1368-0020, while non-responders will be discontinued from the trial 1368-0020.

The placebo control group in Parts 1 and 2 is thus required to compare both efficacy and safety of the anti-IL-36R antibody in patients with moderate-to-severely active ulcerative colitis who failed previous biologic treatments.

The selection of target patients with moderate-to-severely active ulcerative colitis who failed at least one previous biologic treatment (defined as an inadequate response or loss of response to TNF antagonists and/or vedolizumab) is based on the high unmet medical need in such patients, where persistent mucosal inflammation is associated with an increased risk of disease progression, colon cancer and other complications. The term "failed a previous therapy" or "failed at least one previous biologic treatment" or an equivalent thereof, refers to an inadequate response to previous or current treatment with a TNF antagonist, vedolizumab and/or tofacitinib because of toxicity and/or inadequate efficacy. The inadequate response can be assessed by, for example, determining the proportion of patients with Clinical Remission at week 12 (Clinical Remission being defined as mMCS SFS=0 or 1, if drop ≥1 from BL; and RBS=0; and mESS≤1), or by determining the proportion of patients with Mucosal Healing at week 12 (Muscosal Healing being defined as defined as mESS ≤ 1), or by determing the proportion of patients with Clinical response at week 12 (Clinical response being defined as total MCS reduction ≥3 pts. and ≥30% from BL; AND RBS drop from baseline by ≥1 pt., or absolute RBS ≤1 pt), or by determing the change in IBDQ score from baseline at week 12, or by determing the proportion of patients with combined Mucosal healing and histologic remission at week 12 (Combined Mucosal Healing being defined as mESS ≤ 1 and Robarts Histology Index ≤ 6)

A patient who experiences "toxicity" from previous or current treatment with a therapy such as a TNF antagonist, vedolizumab and/or tofacitinib experiences one or more negative side-effects associated therewith such as infection (especially serious infections), congestive heart failure, demyelination (leading to multiple sclerosis), hypersensitivity, neurologic events, autoimmunity, non-Hodgkin's lymphoma, tuberculosis (TB), autoantibodies, etc.

A patient who experiences "inadequate efficacy" continues to have active disease following previous or current treatment with a TNF antagonist, vedolizumab and/or tofacitinib. For instance, the patient may have active disease activity after 1 month or 3 months of therapy with the TNF antagonist, vedolizumab and/or tofacitinib.

### Inclusion criteria

Each patient must meet all of the following inclusion criteria to be included into the trial:
1. 18 - 75 years, at date of signing informed consent, males or females
2. Diagnosis of ulcerative colitis ≥ 3 months prior to screening by clinical and endoscopic evidence corroborated by a histopathology report
3. Moderate to severe activity (total MCS (Mayo Clinical Score) 6 to 12 AND mESS (modified Endoscopic Subscore) ≥ 2 within 7-28 days prior to first dose)
4. Endoscopic activity extending proximal to the rectum (≥ 15 cm from anal verge)
5. Demonstrated in the past inadequate response or loss of response or have had unacceptable side effects with approved doses of TNFo antagonists (infliximab, adalimumab, golimumab) and / or vedolizumab
6. May be receiving a therapeutic dose of the following:
   - Oral 5-ASA compounds, provided that dose has been stable for at least the 4 weeks immediately prior to randomisation, and/or
   - Oral corticosteroids (≤ 20 mg per day of prednisone or equivalent), provided that dose has been stable for the 2 weeks immediately prior to randomisation, and/or
   - Oral budesonide (≤ 9 mg per day ), provided that dose has been stable for the 2 weeks immediately prior to randomisation, and/or
   - Azathioprine, 6-MP or methotrexate, provided that dose has been stable for the 8 weeks immediately prior to randomisation.
   - Probiotics (e.g. S. boulardii) provided that dose has been stable for the 4 weeks immediately prior to randomisation.
7. Patients with extensive colitis or pancolitis of >10 years duration or family history of colorectal cancer or personal history of increased colorectal cancer risk must have had a negative colorectal cancer screening within <1 year prior to enrolment (otherwise to be done during screening colonoscopy).
8. Women of childbearing potential (WOCBP) and men able to father a child must be ready to use highly effective methods of birth control per ICH M3 (R2) that result in a low failure rate of less than 1% per year when used consistently and correctly.
9. Signed and dated written informed consent for 1368.5, in accordance with GCP and local legislation prior to admission into the trial

### Exclusion criteria

Patients meeting any of these exclusion criteria must not be enrolled into the trial:
1. Evidence of abdominal abscess at screening
2. Evidence of fulminant colitis or toxic megacolon at screening
3. Ileostomy, colostomy, or known fixed symptomatic stenosis of the intestine
4. Treatment with:
   - any non-biologic medication (e.g. cyclosporine, tacrolimus or mycophenolate mofetil, intavenous corticosteroids, tofacitinib), other than those allowed per inclusion criteria, within 30 days prior to randomisation
   - any biologic treatment with a TNFα antagonist (adalimumab, infliximab, golimumab) or vedolizumab within 8 weeks prior to randomisation
   - rectal 5-ASA, parenteral or rectal corticosteroids (incl. budesonide) within 2 weeks prior to screening
   - any investigational non-biologic drug for UC (including but not limited to JAK inhibitors, S1P modulators) within 30 days prior to randomisation
   - any investigational biologic for UC (including but not limited to ustekinumab and other IL-23 inhibitors) within 12 weeks prior to randomisation (except etrolizumab: within 8 weeks prior to randomisation)
   - any prior exposure to an anti-IL-36R antibody, natalizumab or rituximab
5. Positive stool examinations for C. difficile or other intestinal pathogens < 30 days prior to screening
6. have had previous surgery or are anticipated to require surgical intervention for UC
7. Evidence of colonic moderate/severe mucosal dysplasia or colonic adenomas, unless properly removed
8. Primary sclerosing cholangitis
9. Faecal transplant ≤ 6 months before screening

### TREATMENTS

Description of test product, an anti-IL-36R antibody of the present invention:

**Table 3: Description of the test product**

| Substance: | BI 655130 |
|---|---|
| Pharmaceutical formulation: | Solution for infusion |
| Source: | Boehringer Ingelheim Pharma GmbH & Co. Kt3 |
| Chemical form: | Anti-human IL-36 Receptor mAb |
| Molecular weight: | 146 kDa |
| Unit strength: | BI 655130 150mg/vial (20mg/mL), 7.5mL fill volume |
| Route of administration: | Intravenous infusions |
| Posology: | 300 mg single dose at Week 0; or 450 mg, 750 mg or 1200 mg at Week 0, 4 and 8 |
| Duration of use: | 12 weeks |

### ASSESSMENT OF EFFICACY

The changes in UC activity during the trial will be assessed at visits including endoscopies using the modified Mayo score (disease activity score, not including the PGA (physician global assessment) item, but including the modified ESS (any degree of friability defines a score of at least 2), and the Robarts histopathology index (RHI). In addition, the total Mayo score (including PGA in addition to modified Mayo Score) will be explored as secondary endpoint to facilitate indirect comparisons against currently approved or investigational drugs.

At all visits, the partial MCS (pMCS; all subscores except mESS) will be recorded to assess clinical disease activity.

Mucosal healing will be assessed by endoscopy using a blinded central reader and will be defined as a mESS ≤ 1. Local reading will be recorded for exploratory reasons, and to be used for endpoint assessment if central reading is not available for technical reasons.

### MAYO SCORING SYSTEM FOR THE ASSESSMENT OF ULCERATIVE COLITIS ACTIVITY

The Mayo score (Schroeder et al., N Engl J Med, 1987) is a composite disease activity score consisting of four items or subscores: stool frequency (relative to normal), rectal bleeding, physician's global assessment, and endoscopic appearance. As proposed by FDA draft guidance, the endoscopic subscore is modified so that a value of 1 does not include friability. The overall range of the Mayo score is 0-12 (higher scores being worse) and each subscore has a range of 0-3 (Table 10.1: 1). At visits without sigmoidoscopy, a partial Mayo score without endoscopy subscore will be assessed. The overall range of this partial Mayo score is 0-9.

In addition, based on FDA's recommendation, a modified Mayo score will be assessed, which excludes physician's assessment. The overall range of the modified Mayo score is 0-9.

The scores for stool frequency and rectal bleeding will be calculated as an average based on the last 3 non-missing scores from the 7 days prior to each applicable visit, as collected from the patient diary. If the patient undergoes bowel preparation for colonoscopy on any of the days before a visit, the stool frequency and rectal bleeding subscore for that day(s) should be considered missing.

The endoscopic appearance score will be assessed by both, the investigational site and a central reader, who is independent from the investigator.

**Table 4: Mayo score (adopted from Schroeder et al, 1987)**

| **Components** | **Subscore** | **Severity** | **Score** |
|---|---|---|---|
| **CLINICAL RESPONSE** (Patient's Symptoms) | Stool Frequency^{a} (daily) | Normal number of stools for patient | 0 |
| | | 1 to 2 stools more than normal | 1 |
| | | 3 to 4 stools more than normal | 2 |
| | | ≥5 stools more than normal | 3 |
| | Rectal Bleeding^{b} (daily) | No blood seen | 0 |
| | | Streaks of blood with stool | 1 |
| | | Obvious blood with stool | 2 |
| | | Blood alone passes | 3 |
| | Physician's Global Assessment^{d} | Normal | 0 |
| | | Mild disease | 1 |
| | | Moderate disease | 2 |
| | | Severe disease | 3 |
| **MODIFIED ENDOSCOPIC RESPONSE** (Objective Evidence of Inflammation) | Endoscopic Appearance^{e} | Normal | 0 |
| | | Mild disease | 1 |
| | | Moderate disease | 2 |
| | | Severe disease | 3 |

| | | | |
|---|---|---|---|
| a Each patient serves as his or her own control to establish the degree of abnormality of tire stool frequency. b The daily bleeding score represents the most severe bleeding of the day. c Modified endoscopic appearance: 0 (normal), Mild (erythema, decreased vascular pattern, granularity), Moderate (marked erythema, loss of vascular pattern, any friability, erosions), Severe (spontaneous bleeding, ulceration). d The physician's assessment acknowledged the three other criteria, the patinet's daily record of abdominal discomfort and general sense of well-being, and other observations, such as physical findings and die patient's performance status. | | | |

### Schroeder KW, Tremaine WJ, Ilstrup DM

Coated oral 5-aminosalicylic acid therapy for mildly to moderately active ulcerative colitis: a randomized study.

N Engl J Med 317 (26), 1625 - 1629 (1987)

### HISTOLOGIC ACTIVITY SCORE

The Robarts histopathology index is a histologic activity score (Mosli et al, Gut2015). The total score ranges from 0 (no disease activity) to 33 (severe disease activity).

**Table 5: Robarts Histopathology Index (RHI) by components**

| Component | |
|---|---|
| Intercept | |
| Chronic inflammatory infiltrate | 0=No Increase |
| | 1 =Mild but unequivocal increase |
| | 2=Moderate increase |
| | 3=Marked increase |
| Lamina propria neutrophils | 0=None |
| | 1=Mild but unequivocal increase |
| | 2=Moderate increase |
| | 3=Marked increase |
| Neutrophils in epithelium | 0=None: |
| | 1=<5% crypts involved |
| | 2=<50% crypts involved |
| | 3=>50% crypts involved |
| Erosion or ulceration | 0=No erosion, ulceration, or granulation tissue |
| | 1=Recovering epithelium + adjacent, inflammation |
| | 1=Probably erosinu-focally stripped |
| | 2=Unequivocal erosion. |
| | 3=Ulcer or granulation tissue |

| | |
|---|---|
| Mosli MH, Feagan BG, Zou G, et al. Development and validation of a histological index for UC. Gut 2015. Based on this, the RHI will be calculated as follows: RBI = 1 x chronic inflammatory infiltrate level (4 levels) + 2 x lamina propria neutrophils (4 levels) + 3 x neutrophils in epithelium (4 levels) + 5 x erosion or ulceration (4 levels after combining Geboes 5.1 and 5.2) | |

### PATIENT REPORTED OUTCOMES

### Inflammatory Bowel Disease Questionnaire (IBDQ)

The IBDQ is a 32-item self-report questionnaire for patients with IBD to evaluate the patient reported outcomes across 4 dimensions: bowel symptoms (loose stools, abdominal pain), systemic symptoms (fatigue, altered sleep pattern), social function (work attendance, need to cancel social events), and emotional function (anger, depression, irritability). Scores range from 32 to 224 with higher scores indicating better outcomes.

### EQ-5D-5L

The EQ-5D(-5L) is a standardized instrument developed by the EuroQoL Group for use as a generic, preference-based measure of health outcome. The EQ-5D(-5L) questionnaire captures two basic types of information, an overall health rating using a visual analog scale and a descriptive "profile," or "health state". The health state is converted to a single weighted index score by applying coefficients from a validated value set. The index score is used in both clinical and economic evaluations of health care. These two basic types of information cannot be combined and will be reported separately.

The health state index measures five health dimensions. The health states for each respondent are converted into a single index number using a specified set of country-specific weights. A higher score indicates a more preferred health status with 1.0 representing perfect health and 0 representing death. A missing answer on any one question leads to a missing overall score.

For purposes of the analyses for this study, all patients' EQ-5D(-5L) index scores will be calculated using the UK weights.

The VAS asks respondents to rate their present health status on a 0 - 100 visual analog scale, with 0 labelled as "Worst imaginable health state" and 100 labelled as "Best imaginable health state." The VAS score is determined by observing the point at which the subject's hand drawn line intersects the scale.

### Example 2: Treating patients with IBD

In this example, an anti-IL36R antibody is used to treat patients with active inflammatory bowel disease. Initially, each of these patients has, for example, a total MCS (Mayo Clinical Score) of 6 to 12. Alternatively or in addition, each of these patients has, for example, a total MCS (Mayo Clinical Score) of 6 to 12 and mESS (modified Endoscopic Subscore) ≥ 2. A therapeutically effective amount of an anti-IL-36R antibody is administered to each of these patients.

The therapeutically effective amount of the anti-IL36R antibody antibody is administered to each patient as one or more induction dose and/or optionally one or more maintenance dose. The induction dose includes 150 mg, 225 mg, 300 mg, 450 mg, 600 mg, 750, 900 mg or 1,200 mg of said anti-IL-36R antibody. The maintenance dose includes 150 mg, 225 mg or 300 mg of said anti-IL-36R antibody. 1, 2 or 3 or more induction doses are administered to each patient, the last of which is followed by 1, 2, or 3 or more maintenance doses. The induction doses are administered at 4 weeks intervals. The first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered. The induction dose is administered intravenously and the maintenance dose is administered intravenously and/or subcutaneously.

Following the administration of the anti-IL-36R antibody, safety and efficacy assessments reveal the following: At least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment. Alternatively or in addition, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve mucosal healing (defined as mESS≤1) at week 12 of the treatment. Alternatively or in addition, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve combined mucosal healing and histologic remission (defined as mESS≤1 and Robarts Histology Index≤6) at week 12 of the treatment.

Following the administration of the anti-IL-36R antibody, safety and efficacy assessments reveal the following: At least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve clinical remission (CDAI score <150) and endoscopic remission (CDEIS ≤ 4). Alternatively or in addition, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve initial isolated ileitis endoscopic remission as defined by CDEIS ≤ 2. In one embodiment, the PRO response of the patient is assessed. For example, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve either a PRO-2 score of <8 or a reduction from baseline of at least 8 points (PRO-2: Patient reported outcome-2).

Following the administration of the anti-IL-36R antibody, safety and efficacy assessments reveal the following: At least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve improved Clinical Remission (i.e., mMCS SFS=0 or 1, if drop ≥1 from BL; and RBS=0; and mESS≤1) by week 12. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve improved Mucosal Healing (i.e., mESS ≤ 1) by week 12. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve improved Clinical Response (i.e., total MCS reduction ≥3 pts. and ≥30% from BL; AND RBS drop from baseline by ≥1pt., or absolute RBS ≤1 pt) by week 12. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve improved IBDQ score by week 12. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients achieve improved combined Mucosal healing and histologic remission (i.e., mESS ≤ 1 and Robarts Histology Index ≤ 6) by week 12.

Following the administration of the anti-IL-36R antibody, safety and efficacy assessments reveal the following: At least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients maintain an improved Clinical Remission (i.e., mMCS SFS=0 or 1, if drop ≥1 from BL; and RBS=0; and mESS≤1) after the administration of the one or more maintenance doses. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients maintain an improved Mucosal Healing (i.e., mESS ≤ 1) after the administration of the one or more maintenance doses. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients maintain an improved Clinical Response (i.e., total MCS reduction ≥3 pts. and ≥30% from BL; AND RBS drop from baseline by ≥1pt., or absolute RBS ≤1 pt) after the administration of the one or more maintenance doses. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients maintain an improved IBDQ score after the administration of the one or more maintenance doses. Alternatively or in addition, at least , at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the patients maintain an improved combined Mucosal healing and histologic remission (i.e., mESS ≤ 1 and Robarts Histology Index ≤ 6) after the administration of the one or more maintenance doses. The improved effects are compared to placebo. The improved effects are maintained at higher percentage with an anti-IL-36R antibody of the present invention than with placebo. At least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the mammals or patients maintain improved effects with the anti-IL-36R antibody than with placebo.

In an embodiment relating to any of the above aspects, the percentage of mammals or patients receiving an anti-IL-36R antibody of the present invention shows improved symptoms after 12 weeks of treatment compared with the percentage of mammals or patients on placebo. In a related embodiment, the improved symptoms comparise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6). In a related embodiment, the improved symptoms are maintained up to 40, 44, 48 or 52 weeks after the administration of the one or more maintenance doses. In a related embodiment, the improved symptoms are maintained at higher percentage with the anti-IL-36R antibody than with placebo. In a related embodiment, the improved effects are maintained at higher percentage with the anti-IL-36R antibody than with placebo. In a related embodiment, at least 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%,68%, 69%, 70%, 71%, 72%, 73%, 74%, 75%, 76%, 77%, 78%, 79%, 80%, 81%, 82%, 83%, 84%, 85%, 86%, 87%, 88%, 89%, or 90% of the mammals or patients show improved symptoms after 12 weeks of treatment compared with the percentage of mammals or patients on placebo.

Following the administration of the anti-IL-36R antibody, safety and efficacy assessments reveal the following: a higher percentage of mammals or patients receiving an anti-IL-36R antibody of the present invention show improved symptoms after 12 weeks of treatment compared with a lesser percentage of mammals or patients on placebo. The improved symptoms comprise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).

Following the administration of the anti-IL-36R antibody, safety and efficacy assessments reveal the following: the improved effects (including the remission or improved symptoms) last for 4, 5, 6, 7, 8, 9, 10, 11, 12, 13, 14, 15, 16, 17, 18, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks following the administration of the last maintenance dose of the anti-IL36R antibody.

### Example 3: Treating patients with moderate-to-severely active ulcerative colitis who have failed previous biologics therapy

In this example, an anti-IL36R antibody of the present invention is used to treat adult patients with moderate to severe active inflammatory bowel disease. Each of these patients has a total MCS (Mayo Clinical Score) of 6 to 12 and has failed a previous biologics (e.g., infliximab, golimumab, adalimumab or vedolizumab) and/or small-molecule (e.g., tofacitinib) therapy. Alternatively or in addition, each of these patients has a total MCS (Mayo Clinical Score) of 6 to 12 and mESS (modified Endoscopic Subscore) ≥ 2 and has failed a previous therapy. A therapeutically effective amount of an anti-IL-36R antibody is administered to each of these patients.

The therapeutically effective amount of the anti-IL36R antibody is administered to each patient as one or more induction dose and/or optionally one or more maintenance dose. The induction dose includes 150 mg, 225 mg, 300 mg, 450 mg, 600 mg, 750, 900 mg or 1,200 mg of said anti-IL-36R antibody. The maintenance dose includes 150 mg, 225 mg or 300 mg of said anti-IL-36R antibody. 1, 2 or 3 or more induction doses are administered to each patient, the last of which is followed by 1, 2, or 3 or more maintenance doses. The induction doses are administered at 4 weeks intervals. The first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered. The induction dose is administered intravenously and the maintenance dose is administered intravenously and/or subcutaneously.

Following the administration of the anti-IL-36R antibody, safety and efficacy assessments (as described in Example 1) reveal the following: At least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment. Alternatively or in addition, at least 10%, 20%, 30%, 40%, 50%, 60%, 70% or 80% of the patients achieve mucosal healing (defined as mESS≤1) at week 12 of the treatment. Alternatively or in addition, at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

### Example 4: Treating Patients with fistulizing Crohn's Disease

In this example, an anti-IL36R antibody of the present invention is used to treat adult patients with fistulizing Crohn's Disease (fistulizing CD). Each patient of Screening Cohort and Study Cohort must meet all of the following inclusion criteria to be included into the trial: 1. 18-75 years at date of signing informed consent 2. Male or female patients. Women of childbearing potential (WOCBP)1 must be ready and able to use highly effective methods of birth control per ICH M3 (R2) that result in a low failure rate of less than 1% per year when used consistently and correctly. Restrictions regarding contraception for female patients are not applicable for Screening Cohort. 3. Diagnosis of clinical Crohn's Disease ≥ 4 months prior to screening by clinical and endoscopic evidence and corroborated by a histopathology report 4. Has > 1 draining perianal fistulas (> 4 weeks duration before enrolment as a complication of CD, confirmed by MRI at screening) and a clinical indication to insert a seton drainage. For Screening Cohort a historical MRI is sufficient. 5. Additional enterocutaneous or abdominal fistulas are permitted (except rectovaginal fistulas) 6. Absent, mild or moderate clinical activity with CDAI < 250. CDAI is not applicable for Screening Cohort. 7. Demonstrated in the past inadequate response or loss of response or have had unacceptable side effects with approved doses of at least one of the following compounds: Immunesuppressive agents (e.g. thiopurines, methotrexate), TNFα antagonists (e.g. infliximab, adalimumab, certolizumab pegol; or respective biosimilars), vedolizumab, ustekinumab, azathioprine and / or antibiotics 8. Patients with family history of colorectal cancer or personal history of increased colorectal cancer risk must have had a negative ileocolorectal cancer screening within <1 year prior to screening per local guidance (otherwise to be done during screening ileocolonoscopy). 9. Signed and dated written informed consent in accordance with ICH-GCP and local legislation prior to admission to the trial. A therapeutically effective amount of an anti-IL-36R antibody of the present invention is administered to each of these patients.

The therapeutically effective amount of the anti-IL36R antibody is administered to each patient as one or more induction dose and/or optionally one or more maintenance dose. The induction dose includes 150 mg, 225 mg, 300 mg, 450 mg, 600 mg, 750, 900 mg or 1,200 mg of said anti-IL-36R antibody. The maintenance dose includes 150 mg, 225 mg or 300 mg of said anti-IL-36R antibody. 1, 2 or 3 or more induction doses are administered to each patient, the last of which is followed by 1, 2, or 3 or more maintenance doses. The induction doses are administered at 4 weeks intervals. The first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered. The induction dose is administered intravenously and the maintenance dose is administered intravenously and/or subcutaneously.

Following the administration of the anti-IL-36R antibody, safety and efficacy assessments (as described in Example 1) reveal the following: the total number of deregulated genes at week 4 is increased or decreased. Alternatively or in addition, at least 10%, 20%, 30%, 40%, 50%, 60%, 70% or 80% of the patients achieve perineal fistula remission at week 12 of the treatment. Alternatively or in addition, at least 10%, 20%, 30%, 40%, 50%, 60%, 70% or 80% of the patients achieve combined perianal fistula remission at week 12 of the treatment.

Following the administration of the anti-IL-36R antibody, safety and efficacy assessments (as described in Example 1) reveal the following: At least 10%, 20%, 30%, 40%, 50%, 60%, 70% or 80% of the patients achieve clinical remission (defined as mMCS SFS (modified Mayo Clinical Score Stool Frequency Score)=0 or 1, If drop ≥1 from BL (Base Line); and/or RBS (Rectal Bleeding Score)=0; and/or mESS≤1) at week 12 of the treatment. Alternatively or in addition, at least 10%, 20%, 30%, 40%, 50%, 60%, 70% or 80% of the patients achieve mucosal healing (defined as mESS≤1) at week 12 of the treatment. Alternatively or in addition, at least 10%, 20%, 30%, 40%, 50%, 60%, 70% or 80% of the patients achieve combined mucosal healing and histologic remission (defined as mESS≤1 and Robarts Histology Index≤6) at week 12 of the treatment.

While certain aspects and embodiments of the invention have been described, these have been presented by way of example only, and are not intended to limit the scope of the invention. Indeed, the novel methods and systems described herein may be embodied in a variety of other forms without departing from the spirit thereof. The accompanying claims and their equivalents are intended to cover such forms or modifications as would fall within the scope and spirit of the invention.

All patents and/or publications including journal articles cited in this disclosure are expressly incorporated herein by reference.

## Claims

1. A method of treating inflammatory bowel disease in a mammal, said method comprising administering to the mammal a therapeutically effective amount of an anti-IL-36R antibody.

2. A method of treating mild to severe inflammatory bowel disease in a mammal, said method comprising administering to the mammal a therapeutically effective amount of an anti-IL-36R antibody.

3. A method of treating ulcerative colitis in a mammal, said method comprising administering to the mammal a therapeutically effective amount of an anti-IL-36R antibody.

4. A method of treating Crohn's disease in a mammal, said method comprising administering to the mammal a therapeutically effective amount of an anti-IL-36R antibody.

5. A method of treating fistulizing Crohn's disease in a mammal, said method comprising administering to the mammal a therapeutically effective amount of an anti-IL-36R antibody.

6. A method of treating moderate to severe active inflammatory bowel disease in patients who have failed a previous therapy, comprising administering to said each patient a therapeutically effective amount of an anti-IL-36R antibody.

7. A method of treating moderate to severe active inflammatory bowel disease in adult patients, wherein each of said patients has a total MCS (Mayo Clinical Score) of 6 to 12 and has failed a previous therapy, comprising administering to said each patient a therapeutically effective amount of an anti-IL-36R antibody.

8. A method for reducing or inhibiting symptoms of moderate to severe active inflammatory bowel disease in adult patients, wherein each of said patients has a total MCS (Mayo Clinical Score) of 6 to 12 and has failed a previous therapy, comprising administering to said each patient a therapeutically effective amount of an anti-IL-36R antibody.

9. The method according to any of claims 5-8, wherein each of said patients has a total MCS (Mayo Clinical Score) of 6 to 12 and mESS (modified Endoscopic Subscore) ≥ 2 and has failed a previous therapy.

10. The method according to any of the preceding claims, wherein the inflammatory bowel disease is ulcerative colitis or Crohn's disease.

11. The method according to any of claims 5-8, wherein the previous therapy comprises a biologics therapy or a small-molecule therapy or both.

12. The method according to claim 11, wherein the biologics therapy comprises a treatment with an anti-TNF antibody.

13. The method according to claim 11, wherein the biologics therapy comprises a treatment with one or more of infliximab, golimumab, adalimumab or vedolizumab.

14. The method according to claim 11, wherein the small-molecule therapy comprises a treatment with tofacitinib.

15. The method according to any of the preceding claims, wherein the anti-IL-36R is administered by a route of administration comprising intravenous, subcutaneous, intraperitoneal or intranasal.

16. The method according to any of the preceding claims, wherein at least 10%, 20%, 30%, 40%, 50%, 60%, 70% or 80% of the mammals or patients achieve clinical remission (defined as mMCS SFS(modified Mayo Clinical Score Stool Frequency Score)=0 or 1, If drop ≥1 from BL (Base Line); and/or RBS (Rectal Bleeding Score)=0; and/or mESS≤1) at week 12 of the treatment.

17. The method according to any of the preceding claims, wherein at least 10%, 20%, 30%, 40%, 50%, 60%, 70% or 80% of the mammals or patients achieve mucosal healing (defined as mESS≤1) at week 12 of the treatment.

18. The method according to any of the preceding claims, wherein at least 10%, 20%, 30%, 40%, 50%, 60%, 70% or 80% of the mammals or patients achieve combined mucosal healing and histologic remission (defined as mESS≤1 and Robarts Histology Index≤6) at week 12 of the treatment.

19. The method according to any of the preceding claims, wherein the anti-IL-36R antibody comprises: a) a light chain variable region comprising the amino acid sequence of SEQ ID NO: 26 (L-CDR1); the amino acid sequence of SEQ ID NO: 35, 102, 103, 104, 105 106 or 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

20. The method according to any of the preceding claims, wherein the anti-IL-36R antibody comprises:
I. a) a light chain variable region comprising the amino acid sequence of SEQ ID NO: 26 (L-CDR1); the amino acid sequence of SEQ ID NO: 102 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
II. a) a light chain variable region comprising the amino acid sequence of SEQ ID NO: 26 (L-CDR1); the amino acid sequence of SEQ ID NO: 103 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
III. a) a light chain variable region comprising the amino acid sequence of SEQ ID NO: 26 (L-CDR1); the amino acid sequence of SEQ ID NO: 104 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
IV. a) a light chain variable region comprising the amino acid sequence of SEQ ID NO: 26 (L-CDR1); the amino acid sequence of SEQ ID NO: 105 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
V. a) a light chain variable region comprising the amino acid sequence of SEQ ID NO: 26 (L-CDR1); the amino acid sequence of SEQ ID NO: 106 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).
VI. a) a light chain variable region comprising the amino acid sequence of SEQ ID NO: 26 (L-CDR1); the amino acid sequence of SEQ ID NO: 140 (L-CDR2); the amino acid sequence of SEQ ID NO: 44 (L-CDR3); and b) a heavy chain variable region comprising the amino acid sequence of SEQ ID NO: 53 (H-CDR1); the amino acid sequence of SEQ ID NO: 62, 108, 109, 110 or 111 (H-CDR2); the amino acid sequence of SEQ ID NO: 72 (H-CDR3).

21. The method according to any of the preceding claims, wherein the anti-IL-36R antibody comprises:
(i) a light chain variable region comprising the amino acid sequence of SEQ ID NO: 77; and a heavy chain variable region comprising the amino acid sequence of SEQ ID NO: 87;or
(ii) a light chain variable region comprising the amino acid sequence of SEQ ID NO: 77; and a heavy chain variable region comprising the amino acid sequence of SEQ ID NO: 88; or
(iii) a light chain variable region comprising the amino acid sequence of SEQ ID NO: 77; and a heavy chain variable region comprising the amino acid sequence of SEQ ID NO: 89; or
(iv) a light chain variable region comprising the amino acid sequence of SEQ ID NO: 80; and a heavy chain variable region comprising the amino acid sequence of SEQ ID NO: 87;or
(v) a light chain variable region comprising the amino acid sequence of SEQ ID NO: 80; and a heavy chain variable region comprising the amino acid sequence of SEQ ID NO: 88; or
(vi) a light chain variable region comprising the amino acid sequence of SEQ ID NO: 80; and a heavy chain variable region comprising the amino acid sequence of SEQ ID NO: 89; or
(vii) a light chain variable region comprising the amino acid sequence of SEQ ID NO: 85; and a heavy chain variable region comprising the amino acid sequence of SEQ ID NO: 100; or
(viii) a light chain variable region comprising the amino acid sequence of SEQ ID NO: 85; and a heavy chain variable region comprising the amino acid sequence of SEQ ID NO:101; or
(ix) a light chain variable region comprising the amino acid sequence of SEQ ID NO: 86; and a heavy chain variable region comprising the amino acid sequence of SEQ ID NO: 100; or
(x) a light chain variable region comprising the amino acid sequence of SEQ ID NO: 86; and a heavy chain variable region comprising the amino acid sequence of SEQ ID NO:101.

22. The method according to any of the preceding claims, wherein the anti-IL-36R antibody comprises:
i. a light chain comprising the amino acid sequence of SEQ ID NO: 115; and a heavy chain comprising the amino acid sequence of SEQ ID NO: 125; or
ii. a light chain comprising the amino acid sequence of SEQ ID NO: 115; and a heavy chain comprising the amino acid sequence of SEQ ID NO: 126; or
iii. a light chain comprising the amino acid sequence of SEQ ID NO: 115; and a heavy chain comprising the amino acid sequence of SEQ ID NO: 127; or
iv. a light chain comprising the amino acid sequence of SEQ ID NO: 118; and a heavy chain comprising the amino acid sequence of SEQ ID NO: 125; or
v. a light chain comprising the amino acid sequence of SEQ ID NO: 118; and a heavy chain comprising the amino acid sequence of SEQ ID NO: 126; or
vi. a light chain comprising the amino acid sequence of SEQ ID NO: 118; and a heavy chain comprising the amino acid sequence of SEQ ID NO: 127; or
vii. a light chain comprising the amino acid sequence of SEQ ID NO: 123; and a heavy chain comprising the amino acid sequence of SEQ ID NO: 138; or
viii. a light chain comprising the amino acid sequence of SEQ ID NO: 123; and a heavy chain comprising the amino acid sequence of SEQ ID NO: 139; or
ix. a light chain comprising the amino acid sequence of SEQ ID NO: 124; and a heavy chain comprising the amino acid sequence of SEQ ID NO: 138.

23. The method according to any of the preceding claims, wherein the anti-IL-36R antibody is administered as one or more induction dose and/or optionally one or more maintenance dose.

24. The method according to claim 23, wherein the induction dose comprises 150 mg, 225 mg, 300 mg, 450 mg, 600 mg, 750, 900 mg or 1,200 mg of said anti-IL-36R antibody.

25. The method according to any of claims 23-24, wherein 1, 2 or 3 induction doses are administered.

26. The method according to any of claims 23-25, wherein 2 or 3 induction doses are administered at 4 weeks intervals.

27. The method according to claim 23, wherein the maintenance dose comprises 150 mg, 225 mg or 300 mg of said anti-IL-36R antibody.

28. The method according to any of claims 23-28, wherein 1, 2 or 3 maintenance doses are administered to the patient; wherein a first maintenance dose is administered after the last induction dose.

29. The method according to claim 28, wherein the first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered.

30. The method of claim 29, wherein the induction dose is administered intravenously and the maintenance dose is administered intravenously or subcutaneously.

31. A method of treating inflammatory bowel disease in a mammal, said method comprising administering to the mammal a therapeutically effective amount of an anti-IL-36R antibody, wherein the anti-IL-36R antibody is administered in one or more induction dose and/or optionally one or more maintenance dose.

32. The method of claim 31, wherein the induction dose comprises 150 mg, 225 mg, 300 mg, 450 mg, 600 mg, 750, 900 mg or 1,200 mg of said anti-IL-36R antibody.

33. The method of claim 31, wherein 1, 2 or 3 induction doses are administered.

34. The method of claim 33, wherein 2 or 3 induction doses are administered at 4 weeks intervals.

35. The method of claim 31, wherein the maintenance dose comprises 150 mg, 225 mg or 300 mg of said anti-IL-36R antibody.

36. The method of claim 31, wherein 1, 2 or 3 maintenance doses are administered to the patient; wherein a first maintenance dose is administered after the last induction dose.

37. The method of claim 36, wherein the first maintenance dose is administered 2 to 8 weeks, 4 to 6 weeks, 2 weeks, 4 weeks, 6 weeks or 8 weeks, after the last induction dose is administered and the second maintenance dose is administered 4, 6, 8 or 12 weeks after said first maintenance dose is administered.

38. The method of claim 31, wherein the induction dose is administered intravenously and the maintenance dose is administered intravenously or subcutaneously.

39. The method according to any of the preceding claims, wherein the mammal or the patient maintains clinical remission after the administration of the one or more maintenance doses.

40. The method according to any of the preceding claims, wherein the IL-36R antibody is an anti-IL-36R antibody disclosed herein.

41. The method according to any of the preceding claims, wherein the mammal or the patient maintains an improved Clinical Remission (i.e., mMCS SFS=0 or 1, if drop ≥1 from BL; and RBS=0; and mESS≤1) after the administration of the one or more maintenance doses; or maintains an improved Mucosal Healing (i.e., mESS ≤ 1) after the administration of the one or more maintenance doses; or maintains maintains an improved Clinical Response (i.e., total MCS reduction ≥3 pts. and ≥30% from BL; AND RBS drop from baseline by ≥1pt., or absolute RBS ≤1 pt) after the administration of the one or more maintenance doses; or maintains an improved IBDQ score after the administration of the one or more maintenance doses; or maintains an improved combined Mucosal healing and histologic remission (i.e., mESS ≤ 1 and Robarts Histology Index ≤ 6) after the administration of the one or more maintenance doses.

42. The method of claim 41, wherein the improved effects are maintained at higher percentage with the anti-IL-36R antibody than with placebo.

43. The method of claim 41, wherein the improved effects are maintained for 4 to 12 weeks following the administration of the last maintenance dose.

44. The method according to any of the preceding claims, wherein a higher percentage of mammals or patients receiving the anti-IL-36R antibody show improved symptoms after 12 weeks of treatment compared with a lesser percentage of mammals or patients on placebo.

45. The method of claim 44, wherein the improved symptoms comprise improved Clinical Remission (i.e., mMCS SFS=0 or 1, if drop ≥1 from BL; and RBS=0; and mESS≤1), improved Mucosal Healing (i.e., mESS ≤ 1), improved Clinical Response (i.e., total MCS reduction ≥3 pts. and ≥30% from BL; AND RBS drop from baseline by ≥1pt., or absolute RBS ≤1 pt), improved IBDQ score, or improved combined Mucosal healing and histologic remission (i.e., mESS ≤ 1 and Robarts Histology Index ≤ 6).
